# EUROPEAN PATENT SPECIFICATION

(11) **EP 3 585 517 B1**
(45) Date of publication and mention of the grant of the patent: **03.07.2024**
(21) Application number: 17934356.1
(22) Date of filing: 28.12.2017
(51) Int. Cl.: B01F 101/23, B01L 3/00

(54) **CARTRIDGE FOR USE IN IN-VITRO DIAGNOSTICS**
KARTUSCHE ZUR VERWENDUNG IN DER VITRO DIAGNOSTIK
CARTOUCHE DEVANT ÊTRE UTILISÉE DANS DES DIAGNOSTICS IN VITRO

(30) Priority: 29.12.2016 IL 24985616; 29.12.2016 IL 24985716
(43) Date of publication of application: 01.01.2020
(73) Proprietor: Ador Diagnostics S.r.l., 00012 Rome (IT)
(72) Inventor: HODKO, Dalibor, Poway, CA 92064 (US); SWANSON, Paul D., Santee, California 92071 (US); HURGIN, Vladimir, Gan Yavne 7085147 (IL); SCHNELL, Amit, 3606811 Kiryat Tivon (IL)
(74) Representative: Evens, Paul Jonathan
(86) International application number: PCT/IL2017/051398
(87) International publication number: WO 2018/122852

(56) References cited:
- US-A- 3 713 780
- US-A1- 2006 002 827
- US-A1- 2006 194 207
- US-A1- 2009 053 689
- US-A1- 2010 291 588
- US-A1- 2011 318 728
- US-A1- 2012 064 614
- US-A1- 2012 156 113
- US-A1- 2013 331 298
- US-A1- 2016 186 240

## Description

### FIELD OF THE INVENTION

The present invention relates to in-vitro diagnostics generally, and in particular to a cartridge for use in in-vitro diagnostics.

### BACKGROUND OF THE INVENTION

Various apparatus and methods for in-vitro diagnostics are known in the art.

US 2010/0291588 relates to systems and methods including self-contained cartridges with detection systems and fluid delivery systems, and discloses a cartridge for use in in-vitro diagnostics, with the cartridge according to the pre-amble of appended independent claim 1. US2012/064614 is also prior art.

### SUMMARY OF THE INVENTION

The present invention seeks to provide a cartridge for in-vitro diagnostics. In accordance with the present invention, there is provided a cartridge for use in in-vitro diagnostics, as defined in appended independent claim 1. Embodiments of the present invention are defined in appended claims dependent on independent claim 1. Methods of using the cartridge in in-vitro diagnostics are described to help understand the present invention and do not form part of the present invention.

### BRIEF DESCRIPTION OF A PREFERRED EMBODIMENT

The present invention will be understood and appreciated more fully from the following detailed description in which:

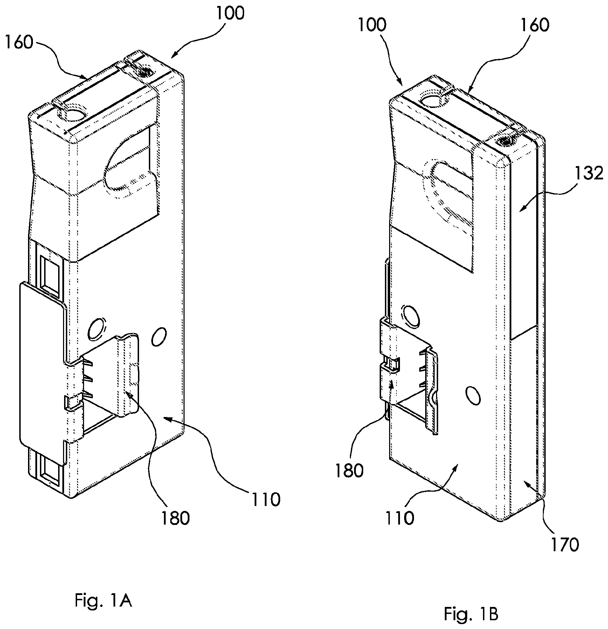

Figs. 1A and 1B are simplified pictorial illustrations of a cartridge for use in in-vitro diagnostics, constructed and operative in accordance with a preferred embodiment of the present invention;

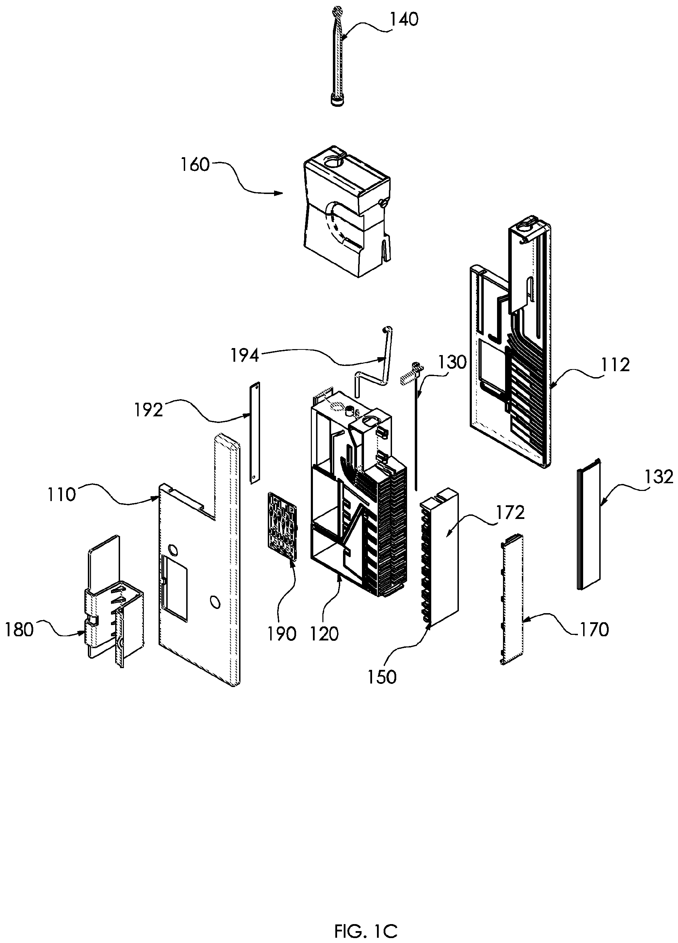

Fig. 1C is a simplified exploded view illustration of the cartridge of Figs. 1A & 1B;

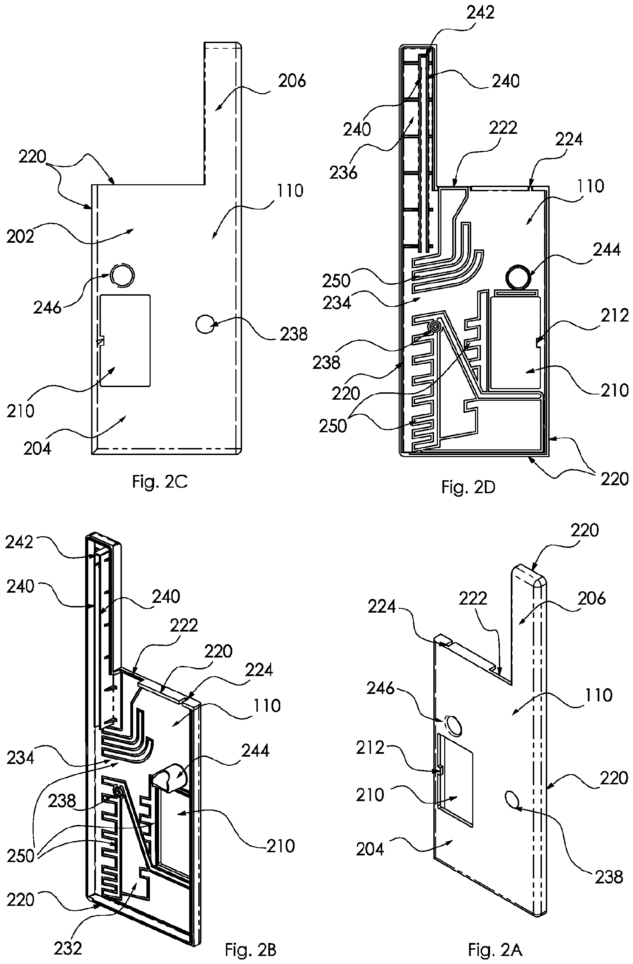

Figs. 2A and 2B are simplified pictorial illustrations of respective exterior and interior surfaces of a first outer housing portion of the cartridge of Figs. 1A - 1C;
Figs. 2C and 2D are simplified plan view illustrations of respective exterior and interior surfaces of the first outer housing portion of the cartridge of Figs. 1A- 1C;

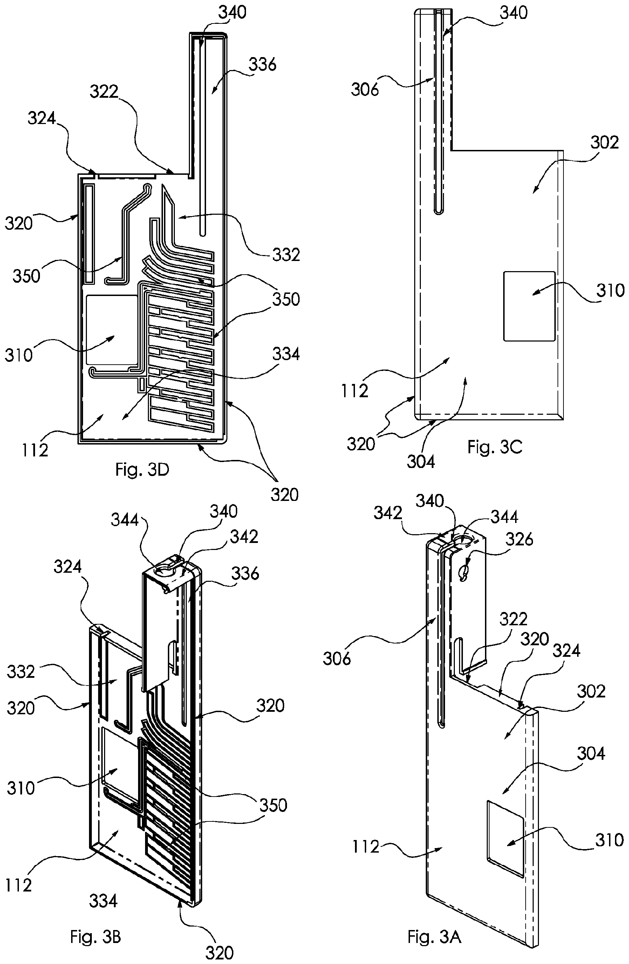

Figs. 3A and 3B are simplified pictorial illustrations of respective exterior and interior surfaces of a second outer housing portion of the cartridge of Figs. 1A- 1C;
Figs. 3C and 3D are simplified plan view illustrations of respective exterior and interior surfaces of the second outer housing portion of the cartridge of Figs. 1A- 1C;

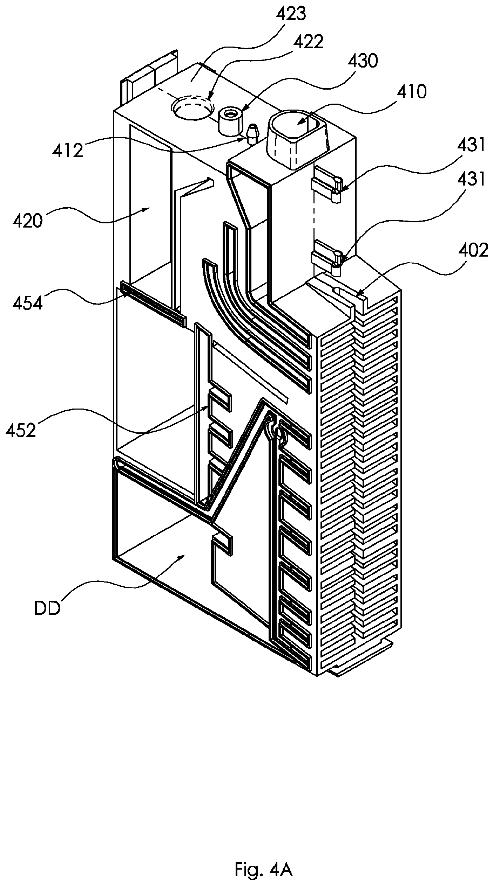

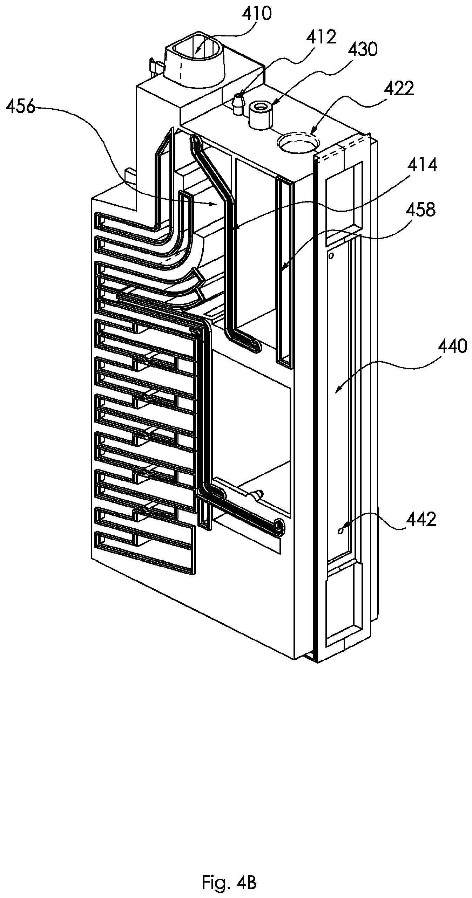

Figs. 4A and 4B are simplified pictorial illustrations of respective first and second sides of a central portion of the cartridge of Figs. 1A - 1C;

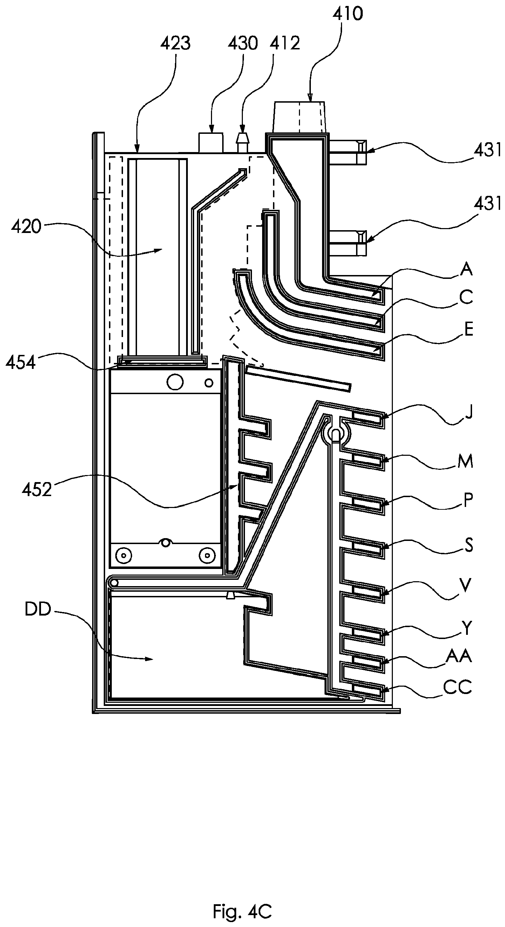

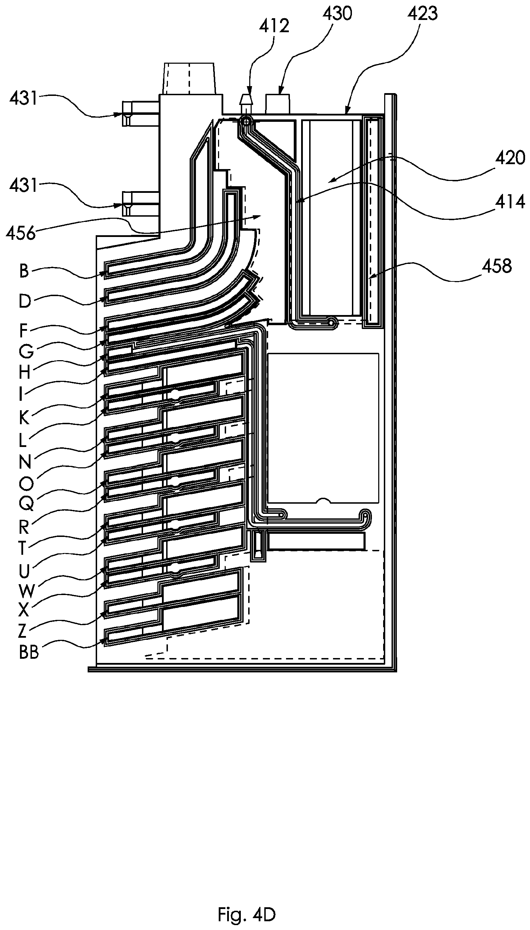

Figs. 4C and 4D are simplified plan view illustrations of respective first and second sides of the central portion of the cartridge of Figs. 1A - 1C;

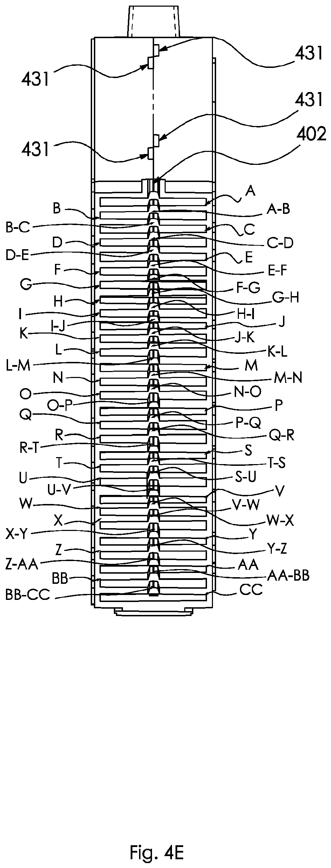

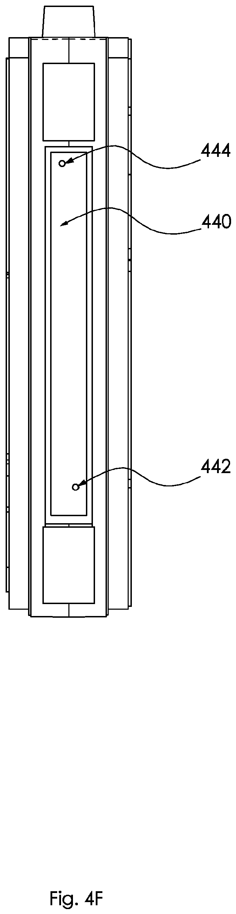

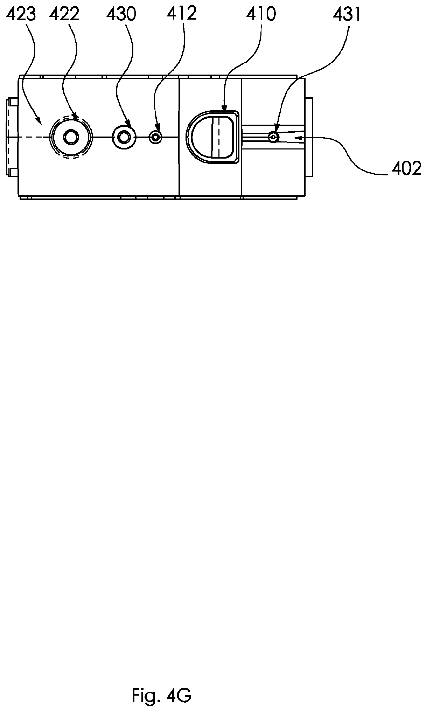

Figs. 4E, 4F and 4G are simplified plan view illustrations of respective first and second side edges and a top edge of the central portion of the cartridge of Figs. 1A- 1C;

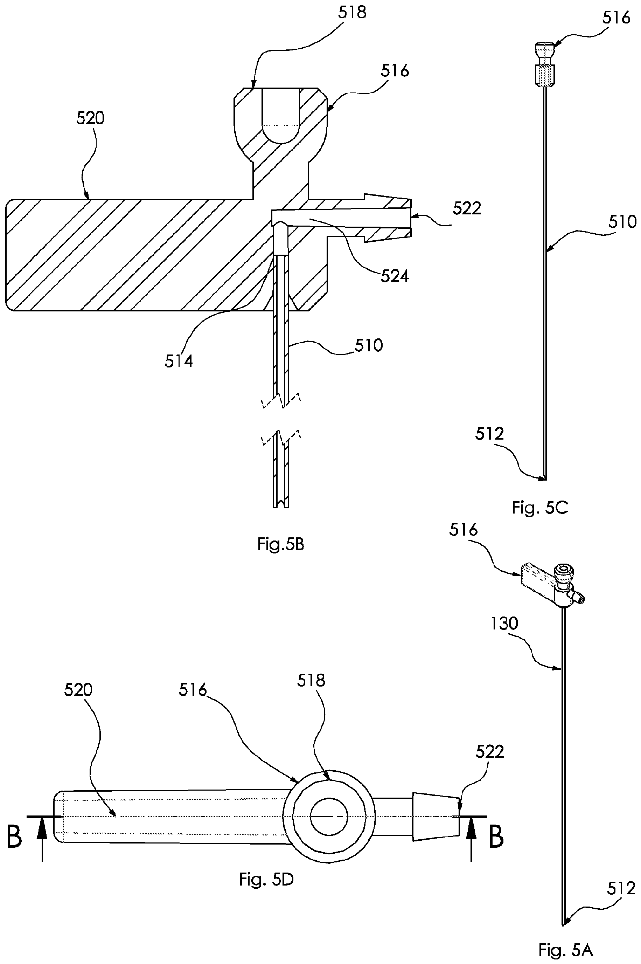

Figs. 5A, 5B, 5C and 5D are simplified respective pictorial, partial side sectional view, end view and top view illustrations of a needle assembly forming part of the cartridge of Figs. 1A & 1B, Fig. 5B being taken along lines B - B in Fig. 5D;

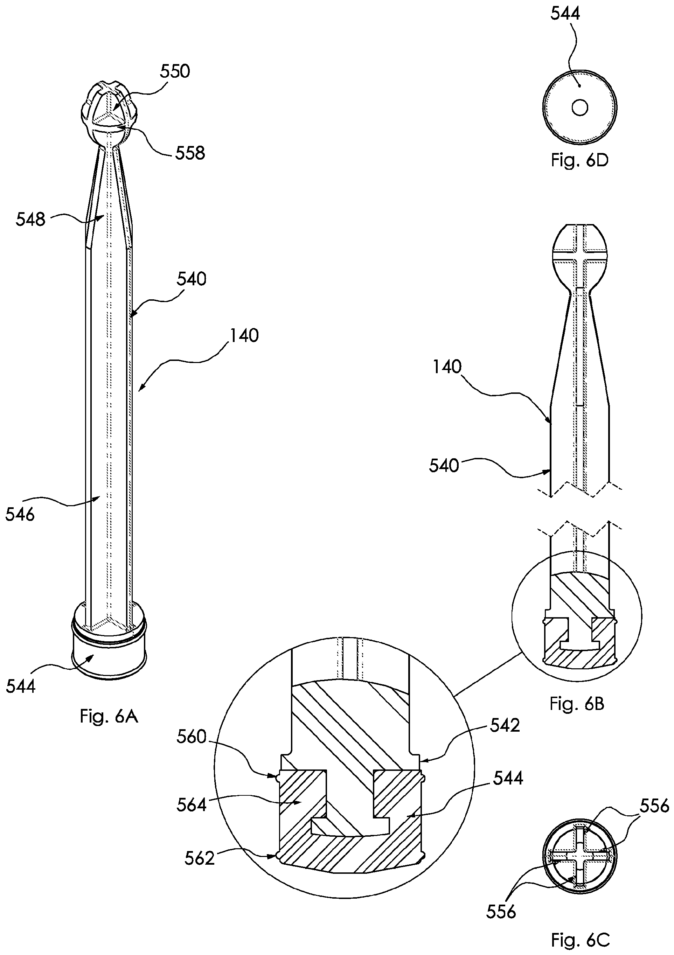

Figs. 6A, 6B, 6C and 6D are simplified respective pictorial view, partially planar, partially sectional side view, top view and bottom view illustrations of a piston assembly forming part of the cartridge of Figs. 1A & 1B;

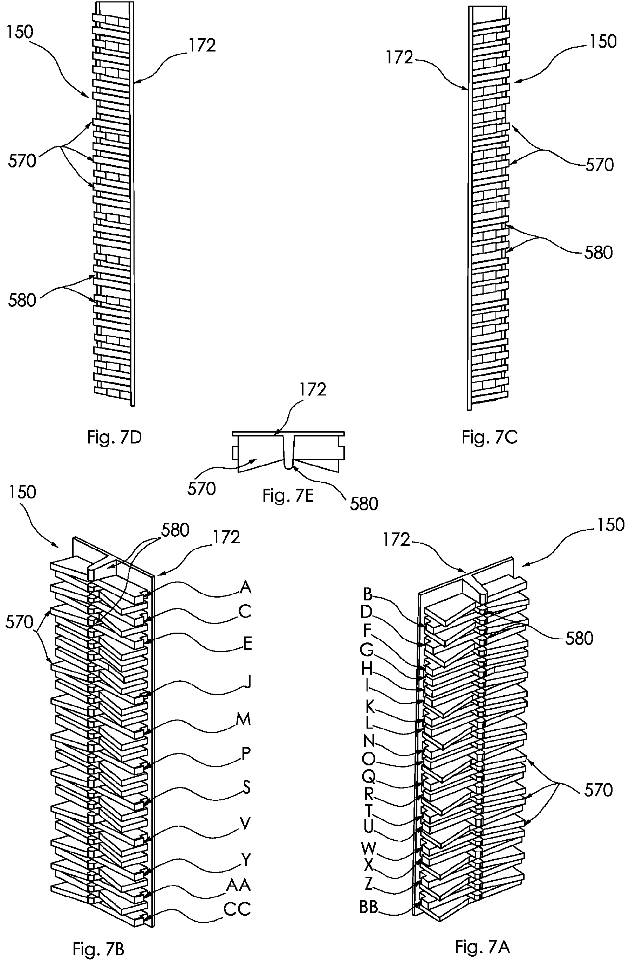

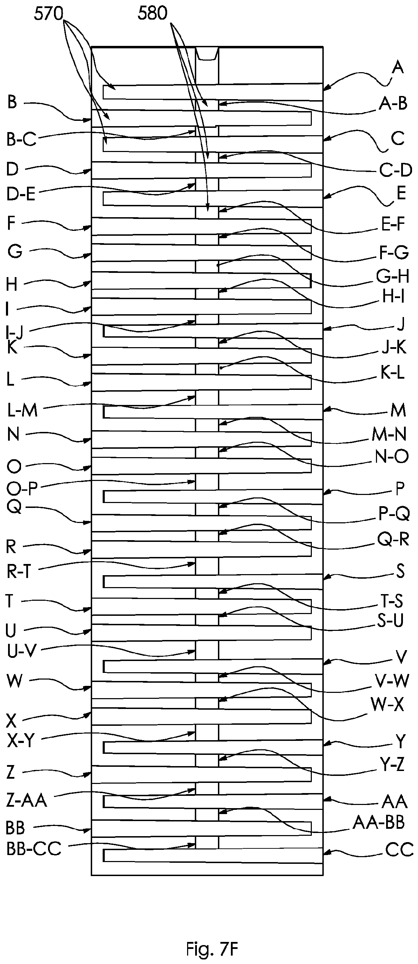

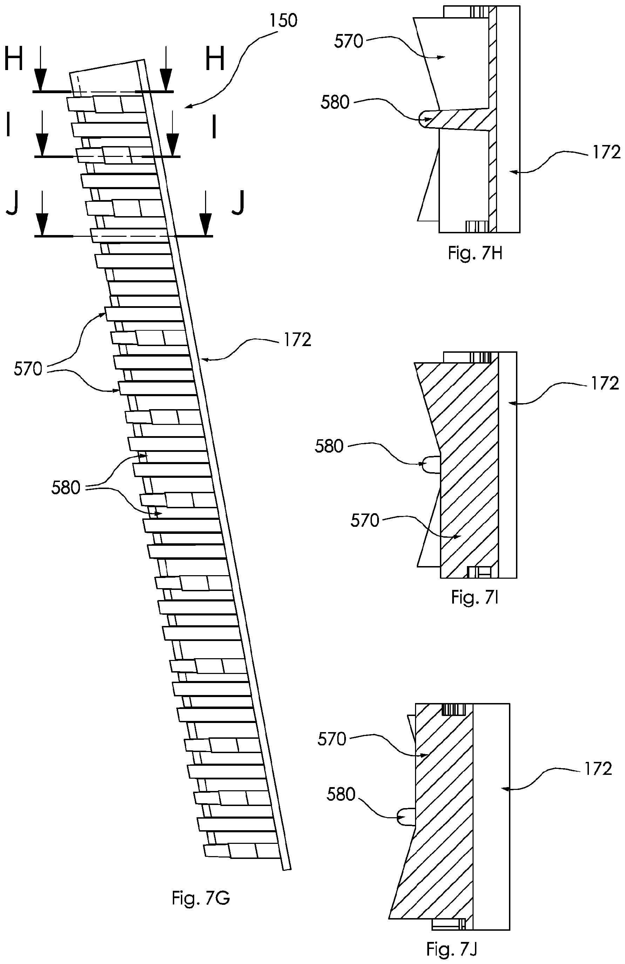

Figs. 7A, 7B, 7C, 7D, 7E, 7F, 7G, 7H, 7I and 7J are, respectively, simplified first and second pictorial views, first and second edge views, a top view, an interior planar view, an angled edge view and three sectional illustrations of a septum portion forming part of the cartridge of Figs. 1A - 1C, Figs. 7H, 7I and 7J being taken along respective section lines H - H, I - I and J - J in Fig. 7G;

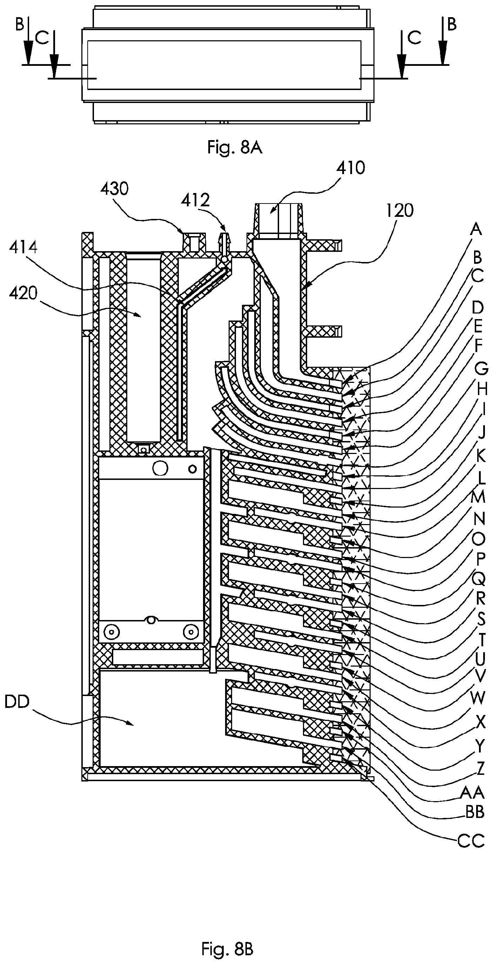

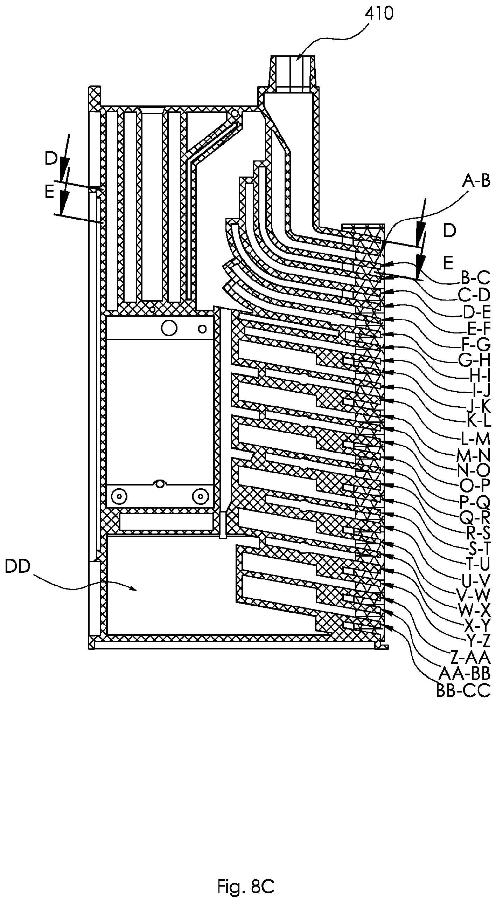

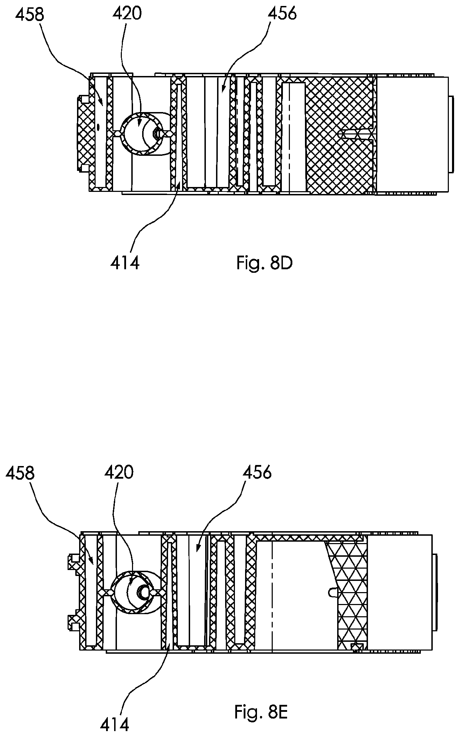

Figs. 8A, 8B, 8C, 8D and 8E are, respectively, a simplified bottom planar view and simplified sectional illustrations of the central element of Figs. 4A - 4G overmolded with the septum portion of Figs. 7A - 7J, Figs. 8B, 8C, 8D and 8E being taken along respective section lines B - B and C - C in Fig. 8A and section lines D - D and E - E in Fig. 8C;

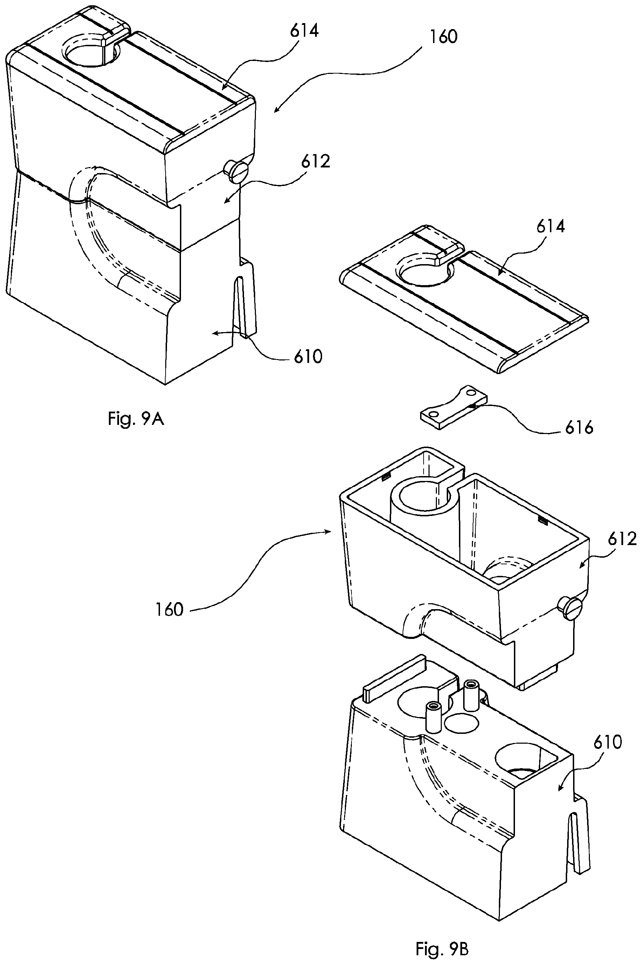

Figs. 9A and 9B are simplified assembled and exploded view illustrations of a sample insertion subassembly forming part of the cartridge of Figs. 1A - 1C;

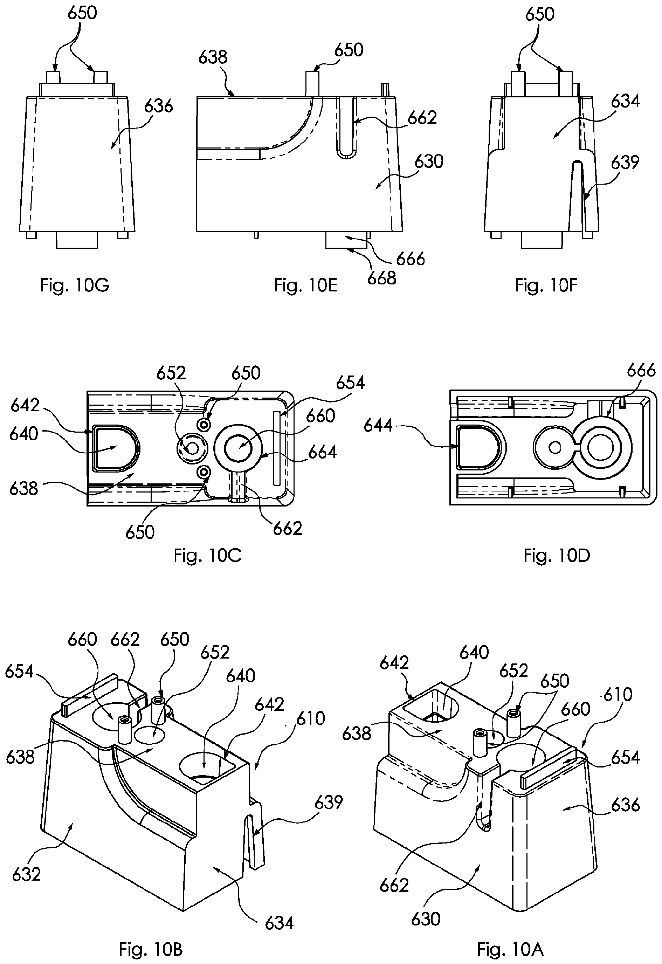

Figs. 10A, 10B, 10C, 10D, 10E, 10F and 10G are simplified respective first and second pictorial, top and bottom plan view, front plan view and first and second side plan view illustrations of a first element of the sample insertion subassembly of Figs. 9A & 9B;

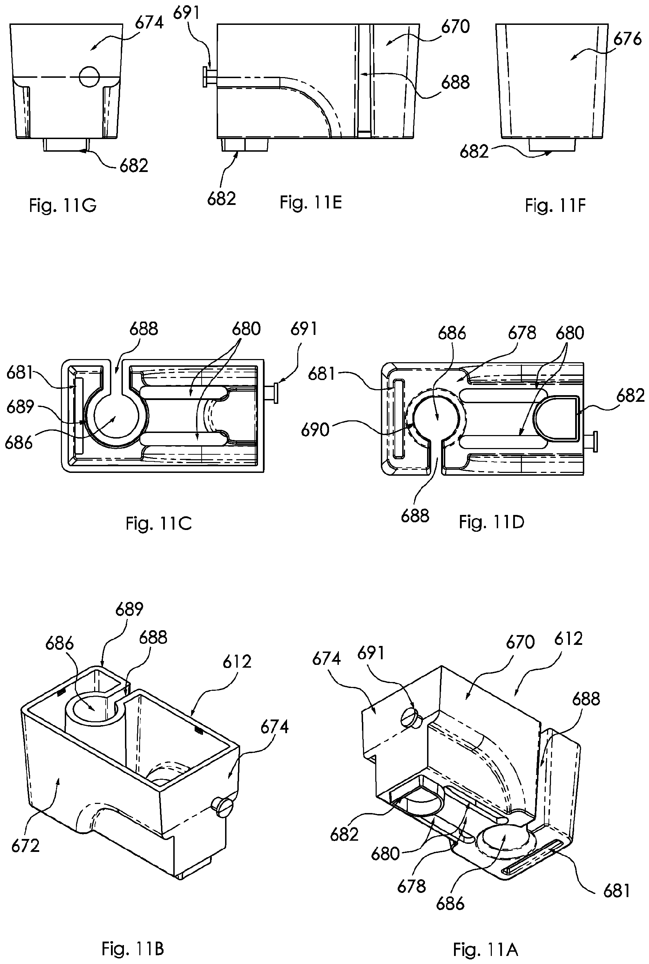

Figs. 11A, 11B, 11C, 11D, 11E, 11F and 11G are simplified respective first and second pictorial, top and bottom plan view, front plan view and first and second side plan view illustrations of an intermediate portion of the sample insertion subassembly of Figs. 9A & 9B;

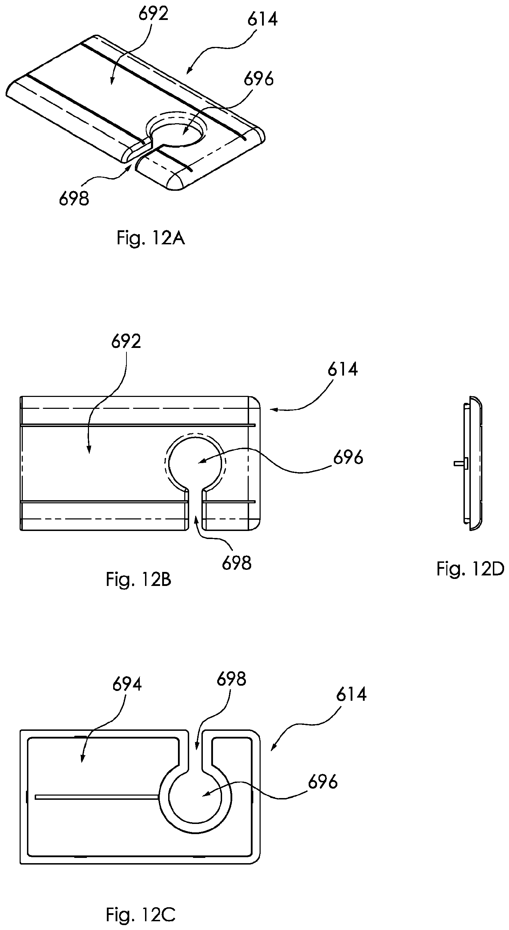

Figs. 12A, 12B, 12C and 12D are simplified respective pictorial and planar top, bottom and edge views of a cover portion of the sample insertion subassembly of Figs. 9A & 9B;

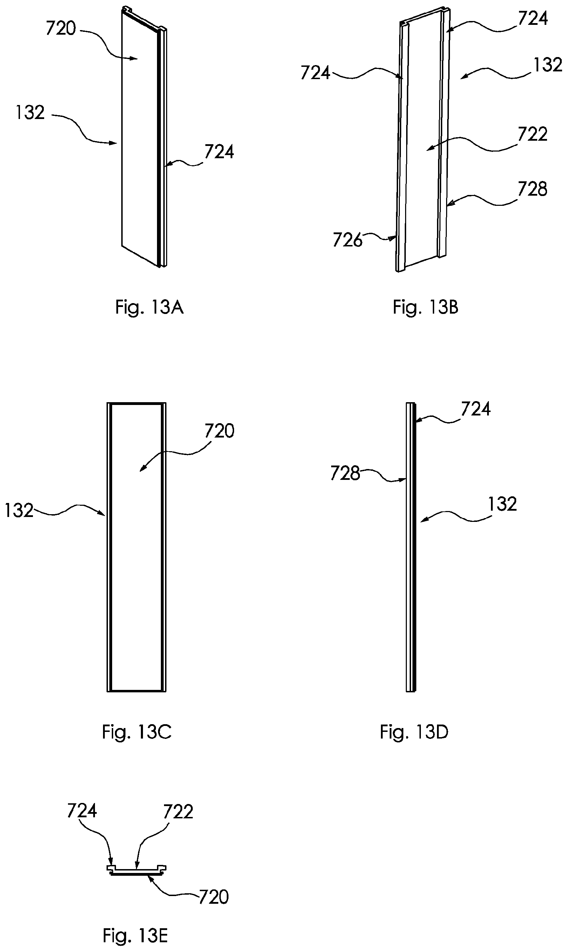

Figs. 13A, 13B, 13C, 13D and 13E are simplified respective first and second pictorial and planar side, edge and top views of a cover element for the needle assembly sample insertion subassembly of Figs. 5A - 5D;

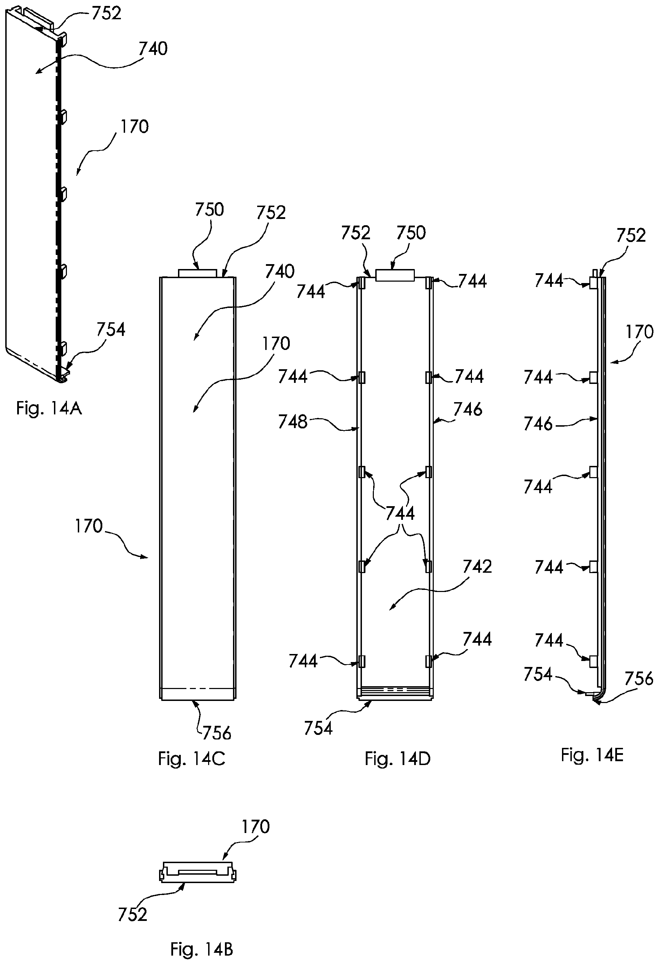

Figs. 14A, 14B, 14C, 14D and 14E are simplified respective pictorial and planar top, outer side, inner side and edge views of a cover element for a fluid solution insertion area of the cartridge of Figs. 1A - 1C;

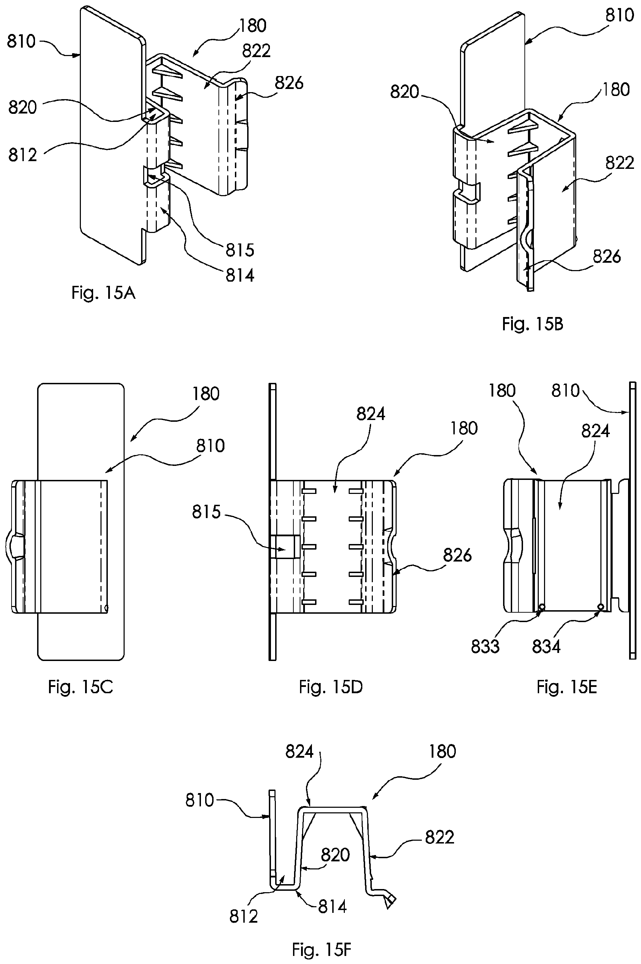

Figs. 15A, 15B, 15C, 15D, 15E and 15F are simplified respective first and second pictorial and first, second, third and fourth planar views of a safety lock element forming part of the cartridge of Figs. 1A - 1C;

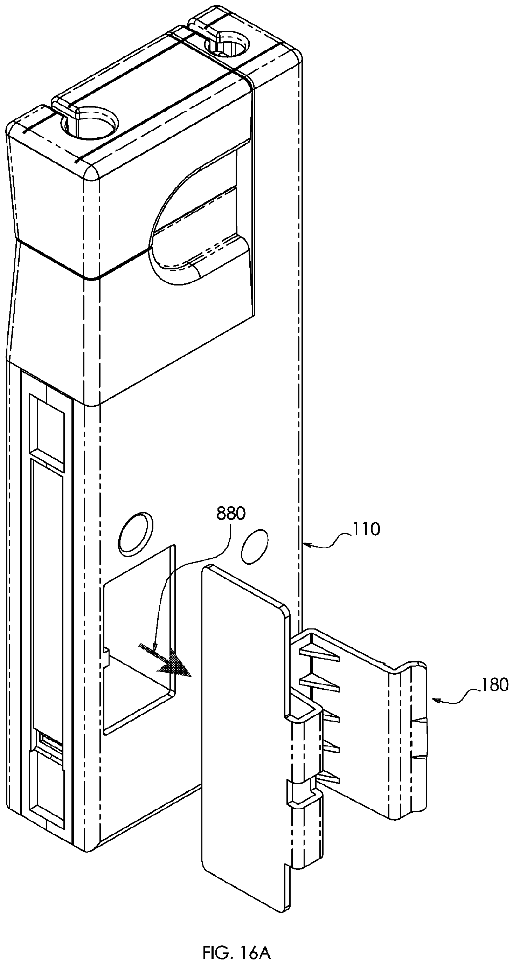

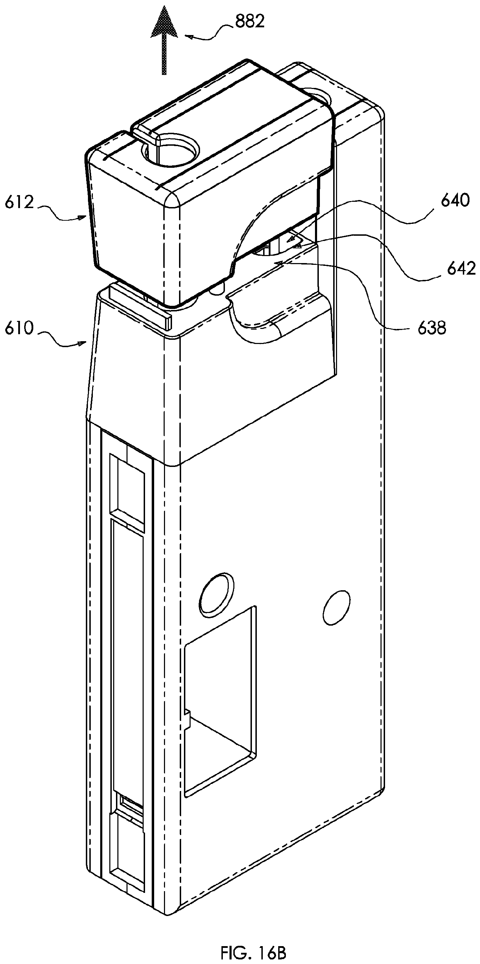

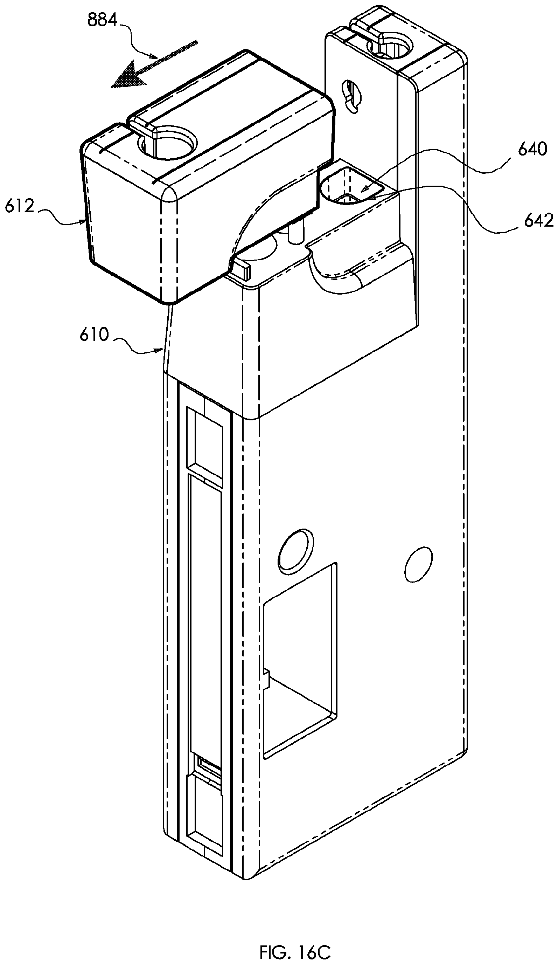

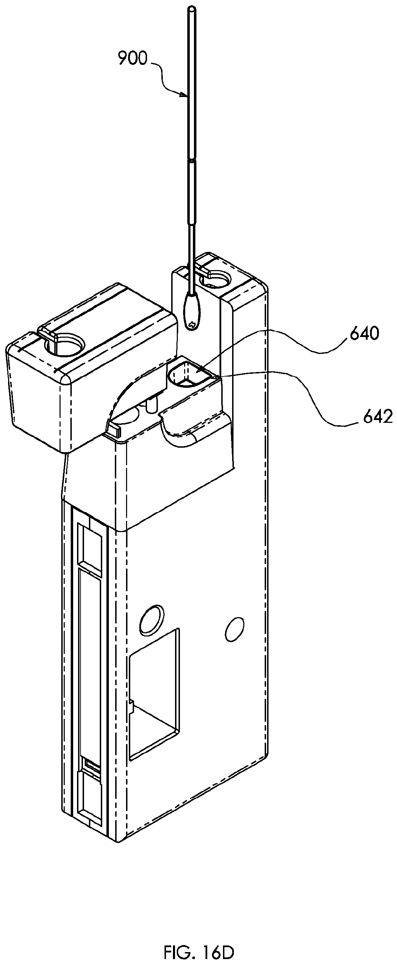

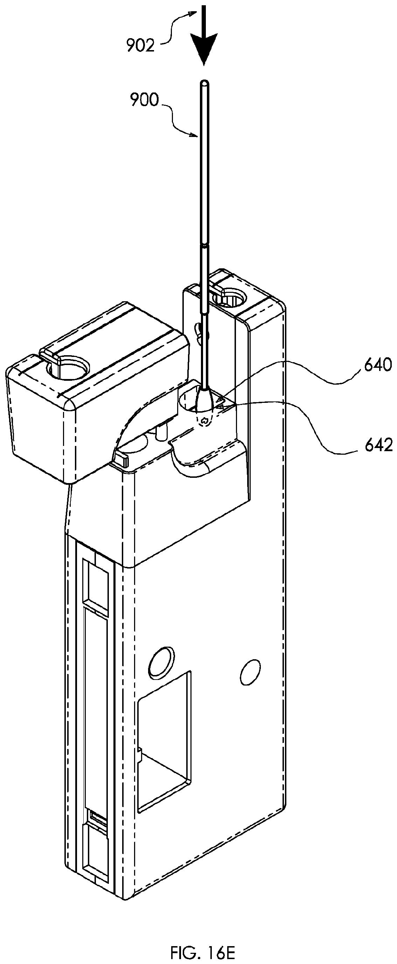

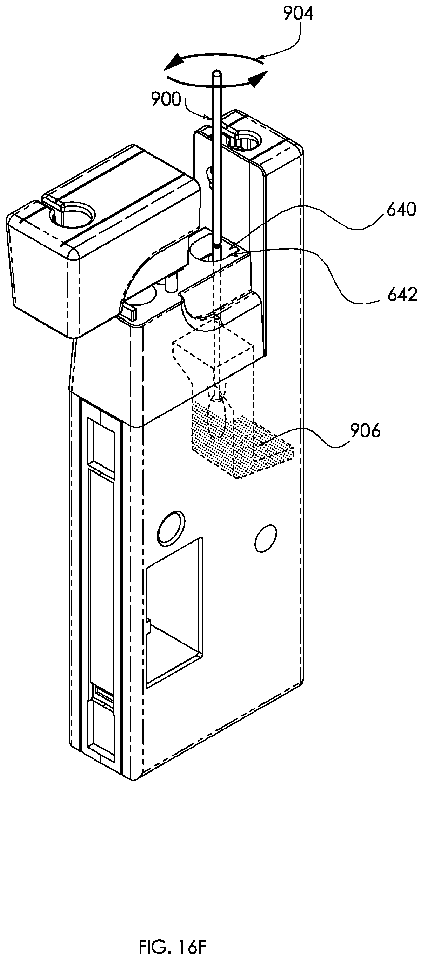

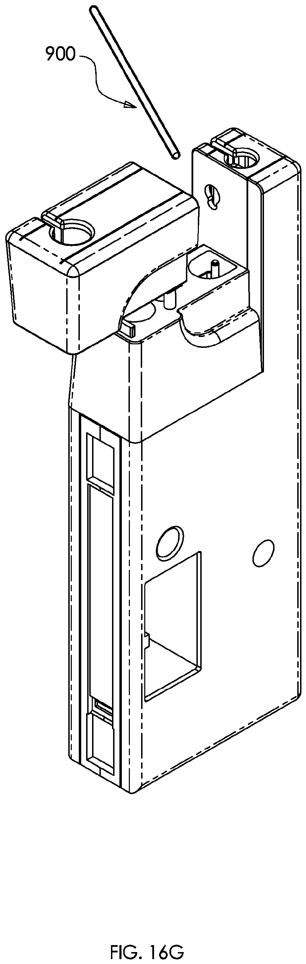

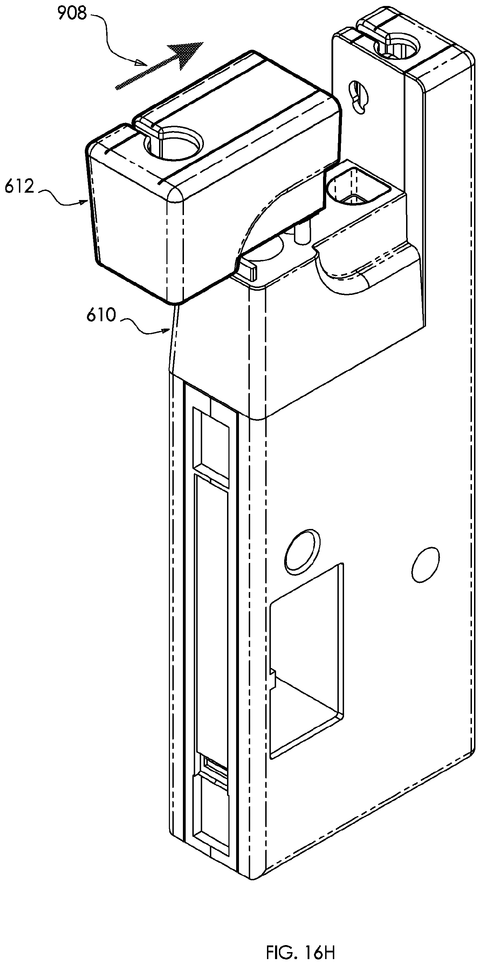

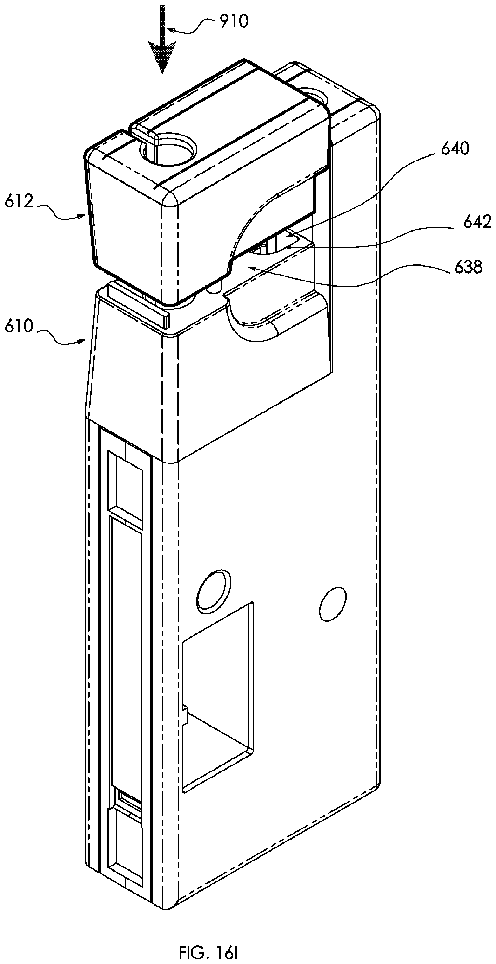

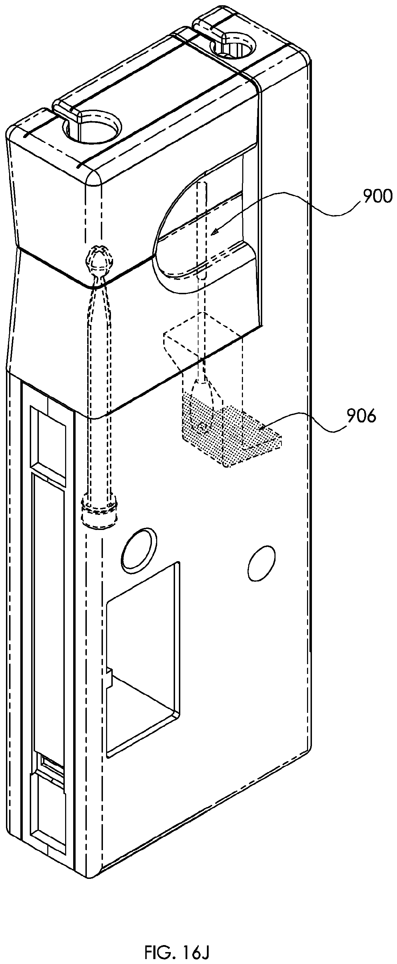

Figs. 16A, 16B, 16C, 16D, 16E, 16F, 16G, 16H, 16I and 16J are simplified illustrations of typical initial steps in the operation of the cartridge of Figs. 1A- 15F;

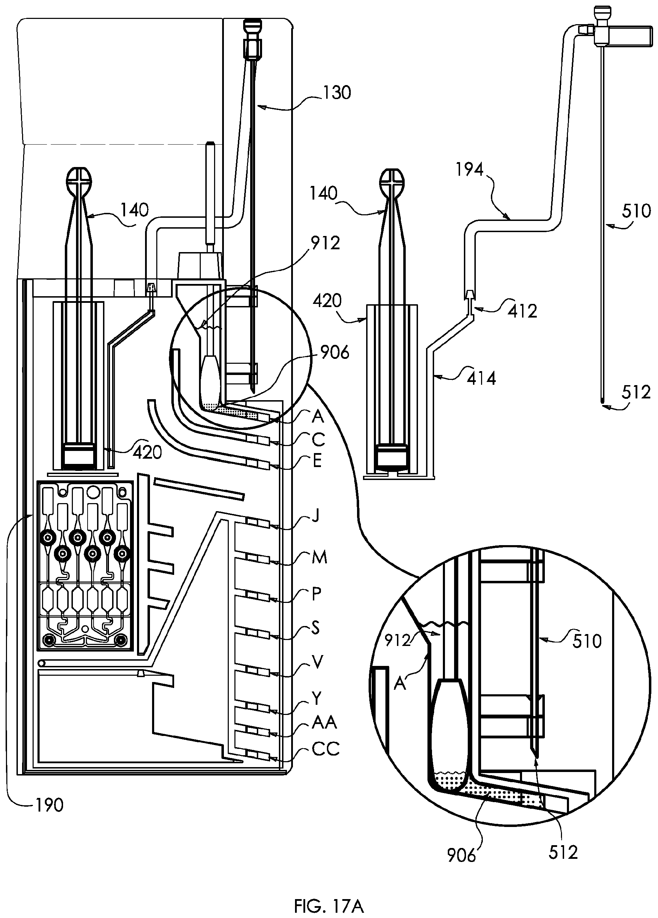

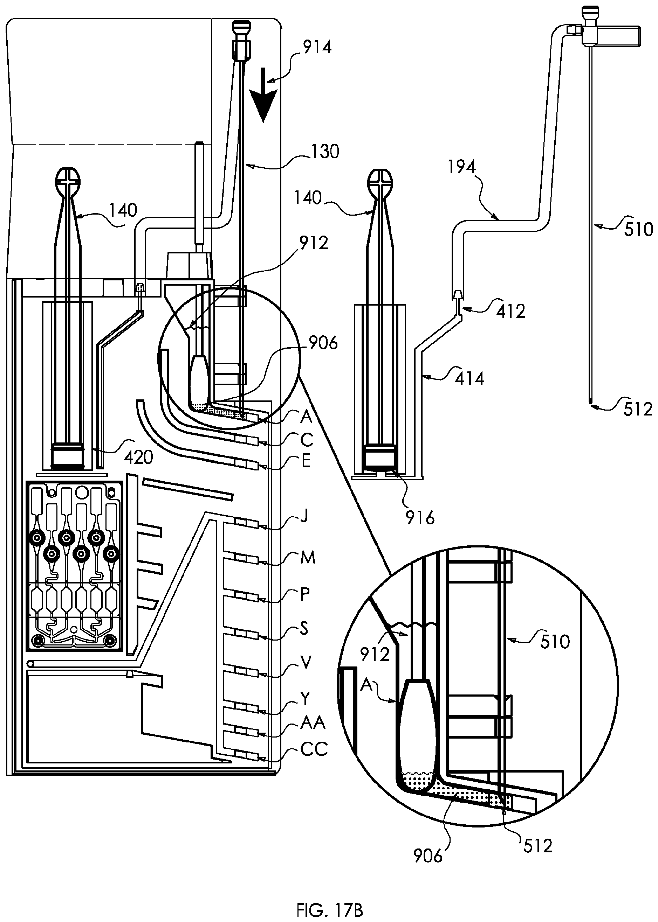

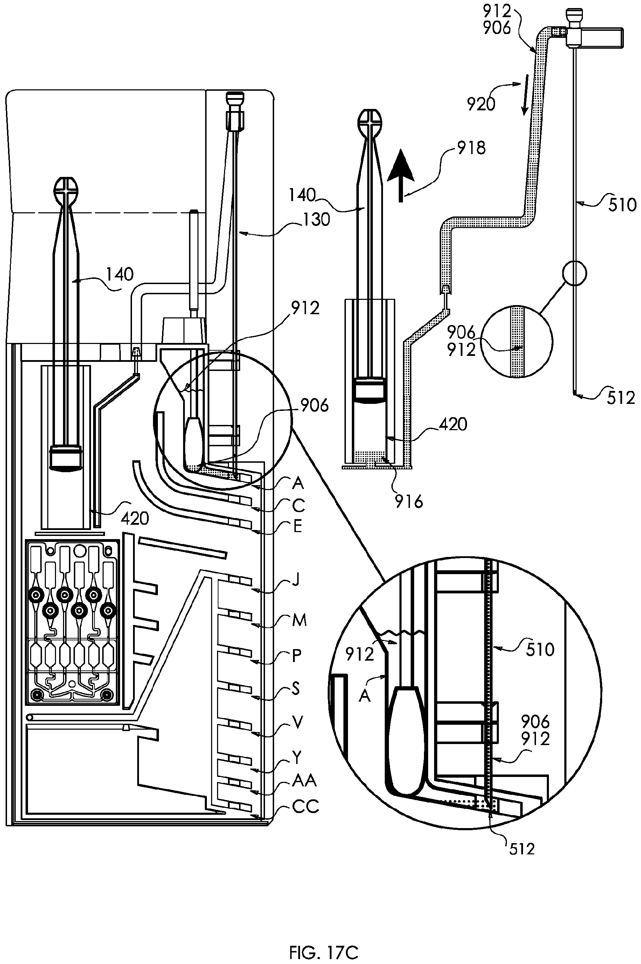

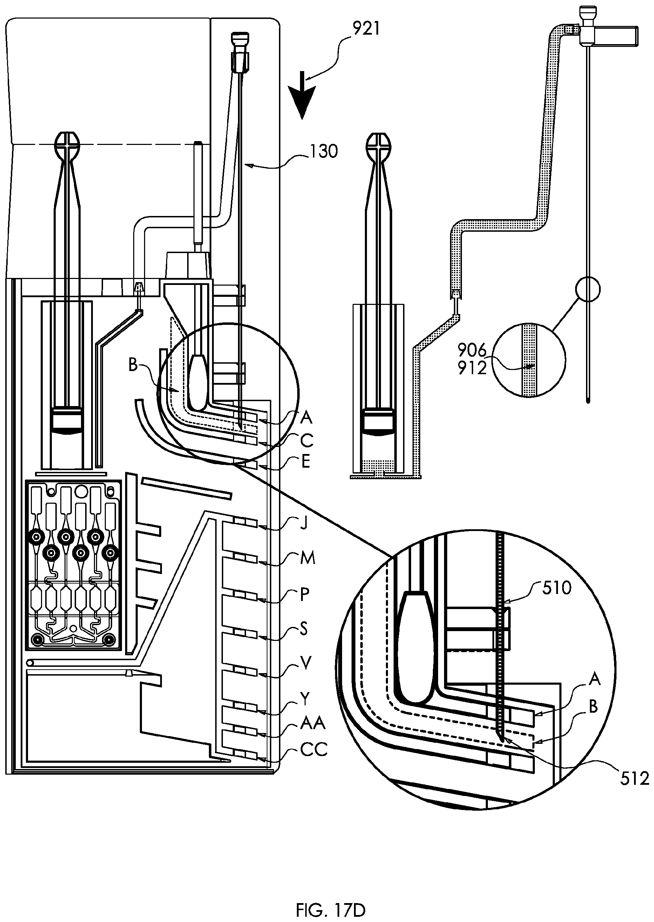

Figs. 17A, 17B, 17C and 17D are simplified illustrations of typical further steps in the operation of the cartridge of Figs. 1A - 15F, wherein Fig. 17A shows an operational state identical to that of Fig. 16J, Figs. 17A - 17D showing the cartridge of Figs. 1A - 15F with the first outer housing portion thereof removed and operative engagement with operational volumes A and B;

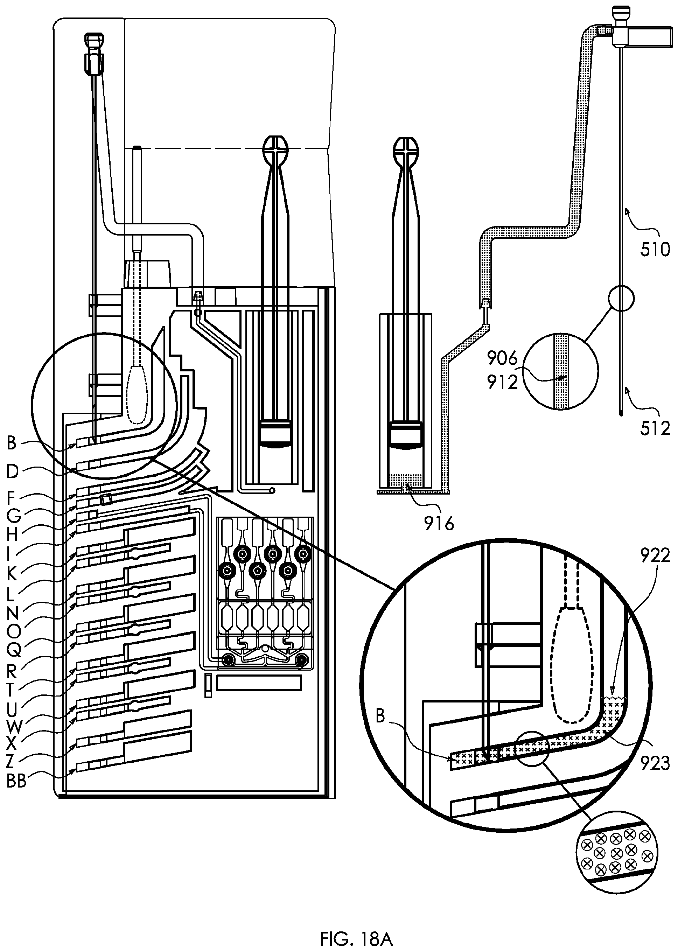

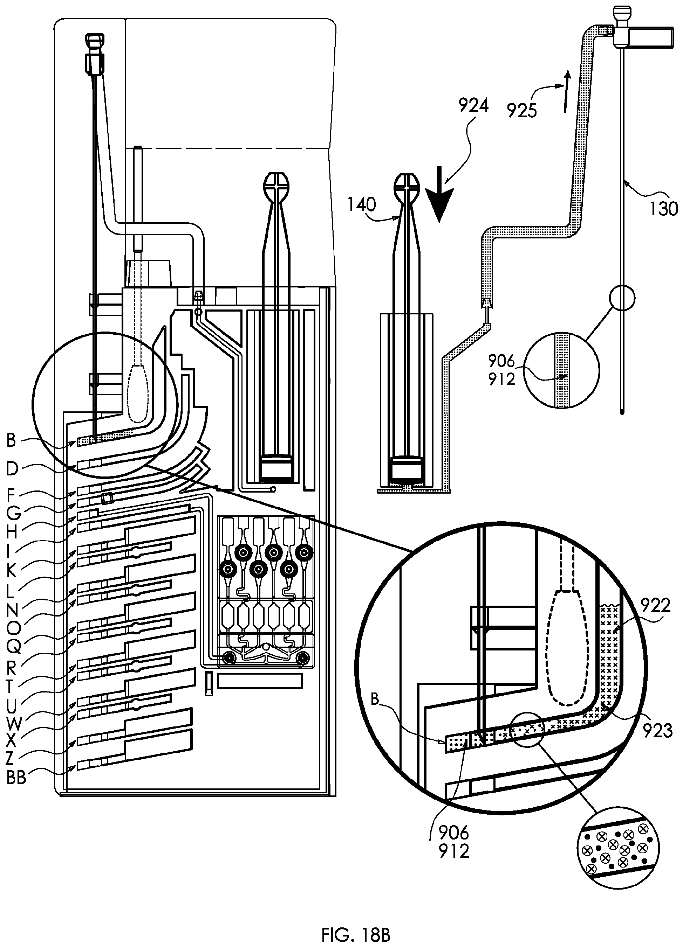

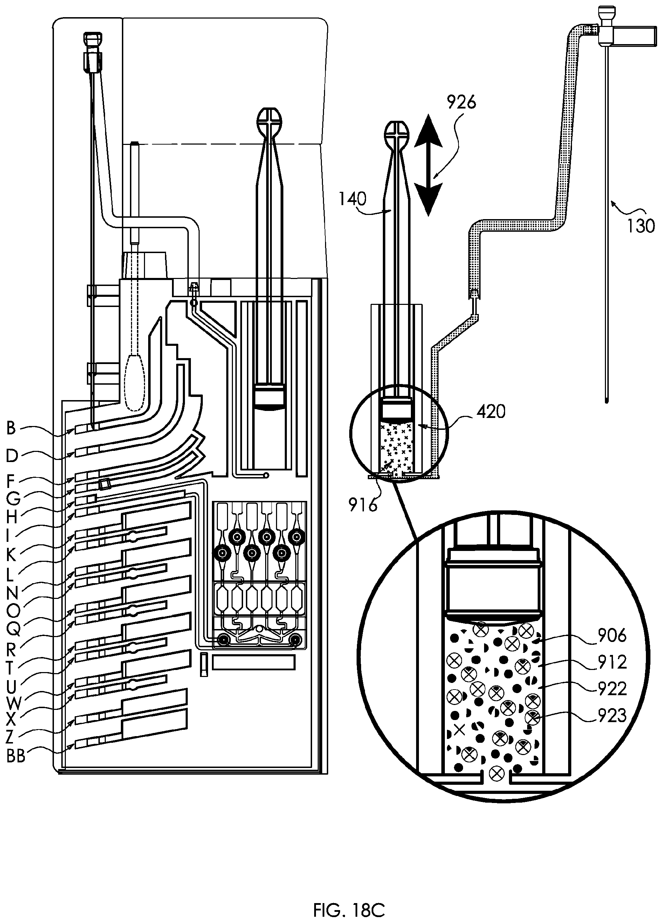

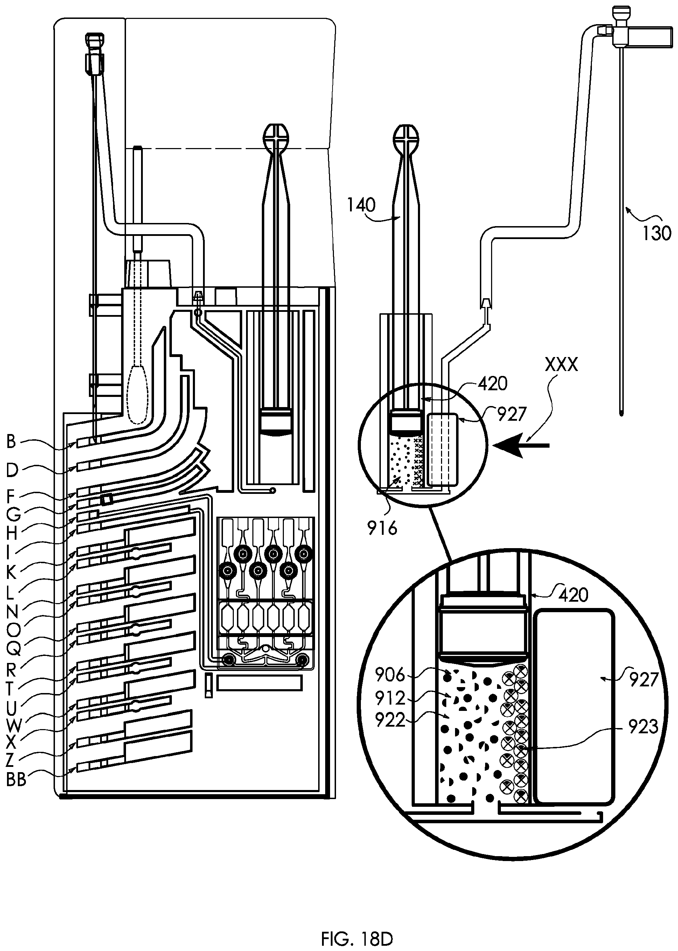

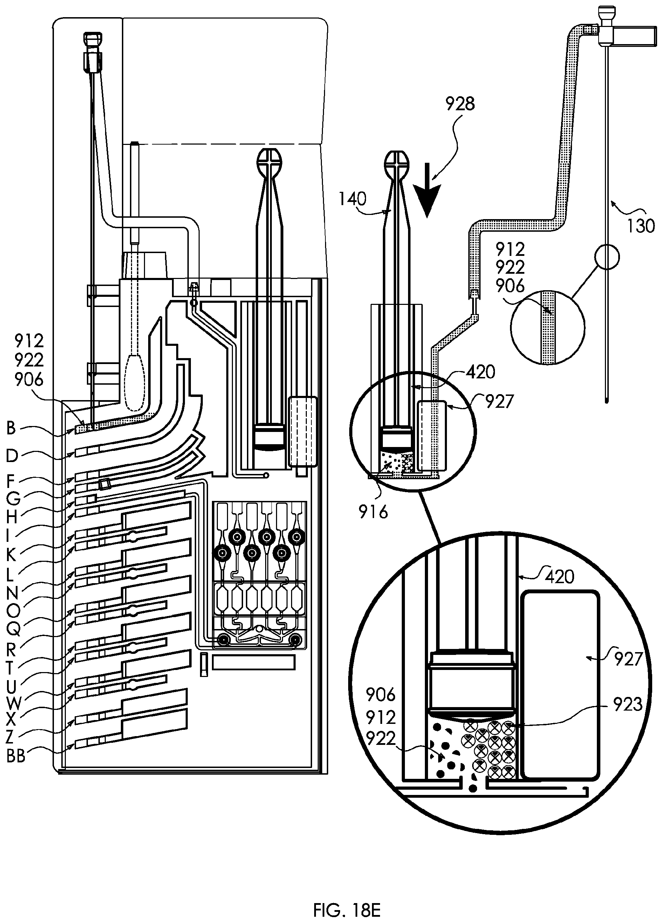

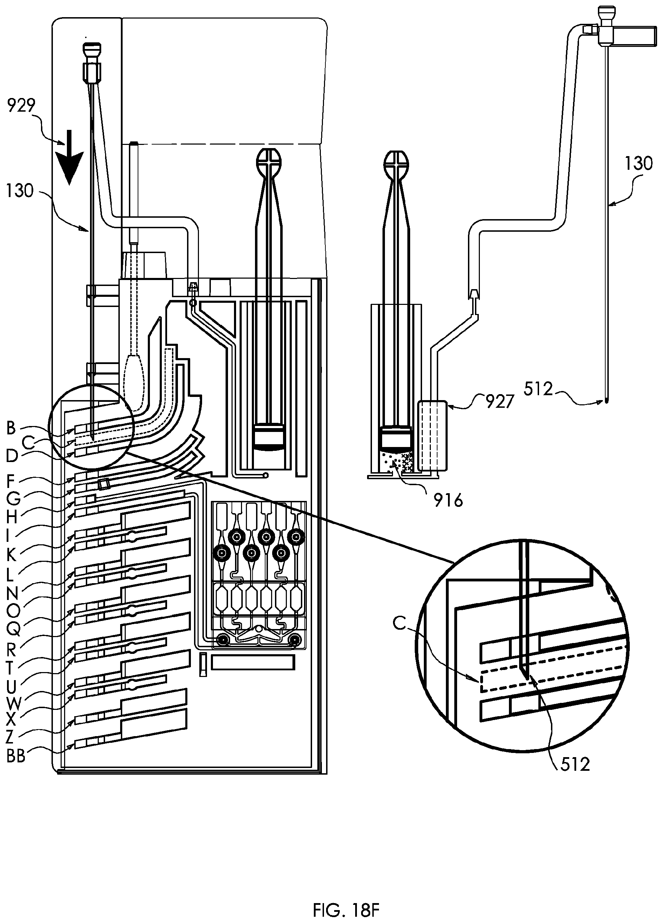

Figs. 18A, 18B, 18C, 18D, 18E and 18F are simplified illustrations of typical still further steps in the operation of the cartridge of Figs. 1A - 15F, wherein Fig. 18A shows an operational state identical to that of Fig. 17D, Figs. 18A - 18F showing the cartridge of Figs. 1A - 15F in a viewing direction opposite to that of Figs. 17A - 17D and with the second outer housing portion thereof removed and showing operative engagement with operational volumes B and C;

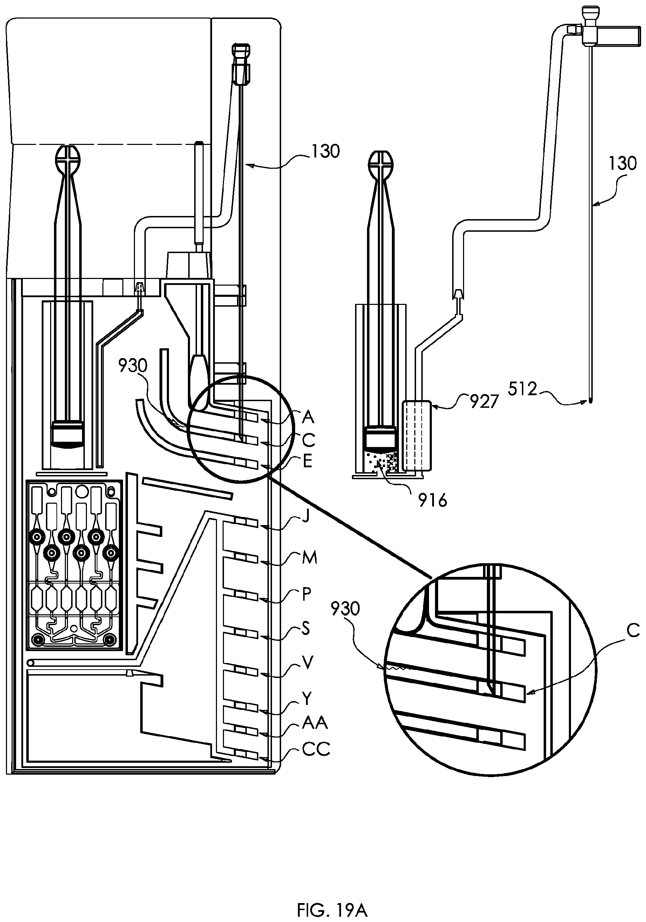

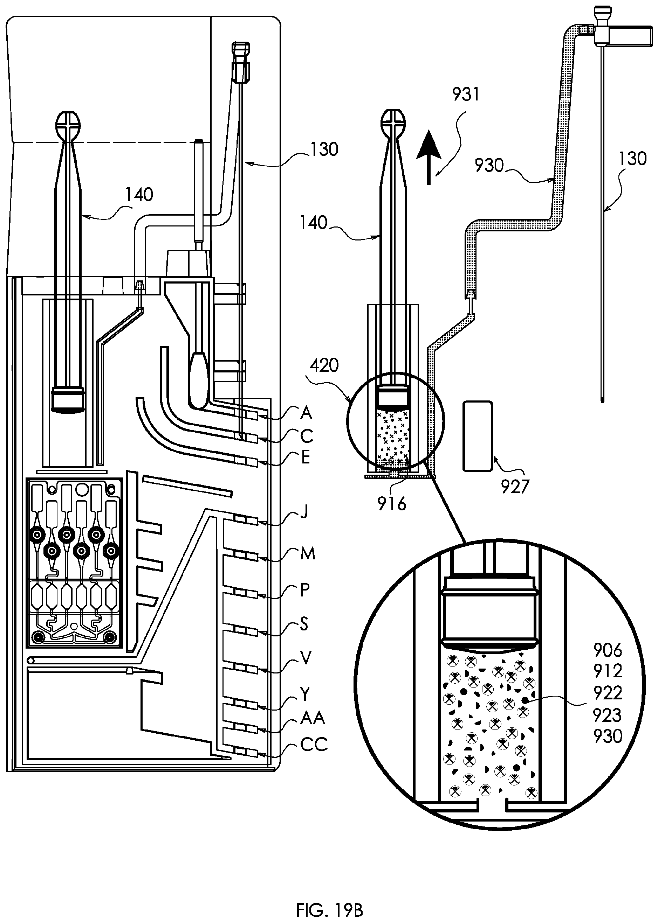

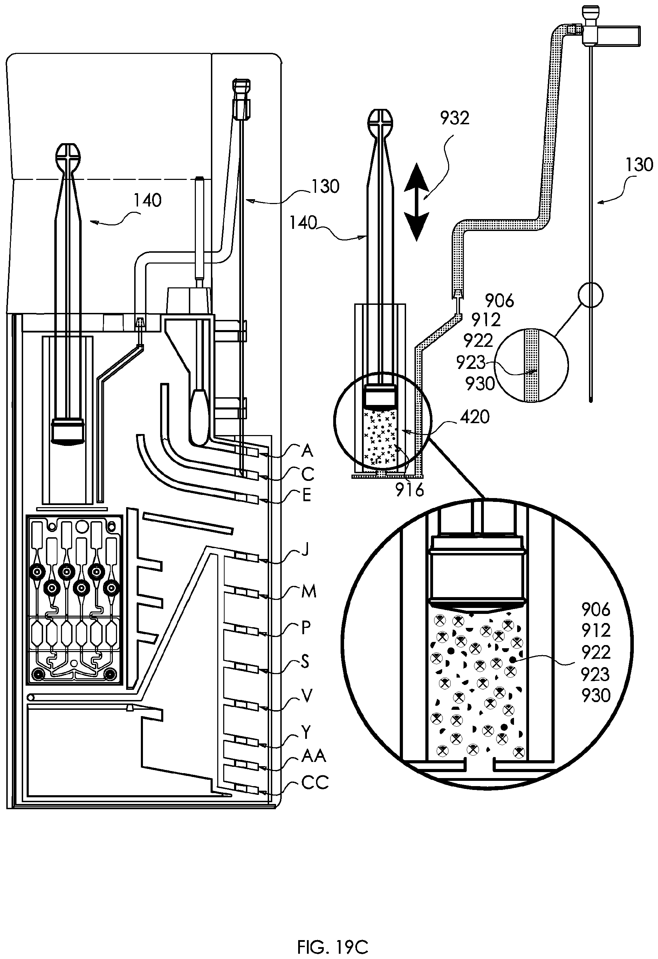

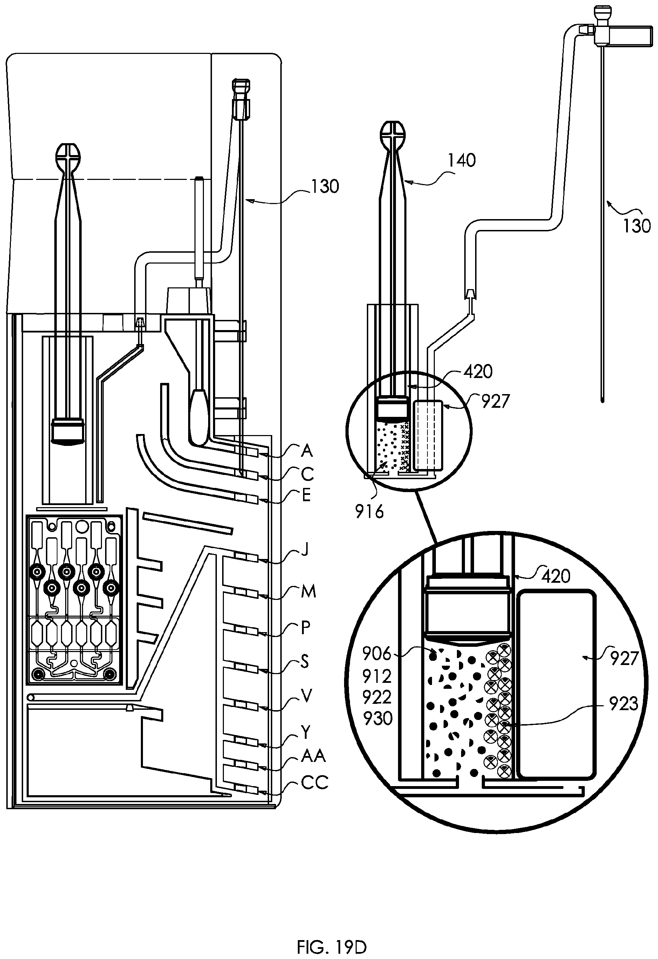

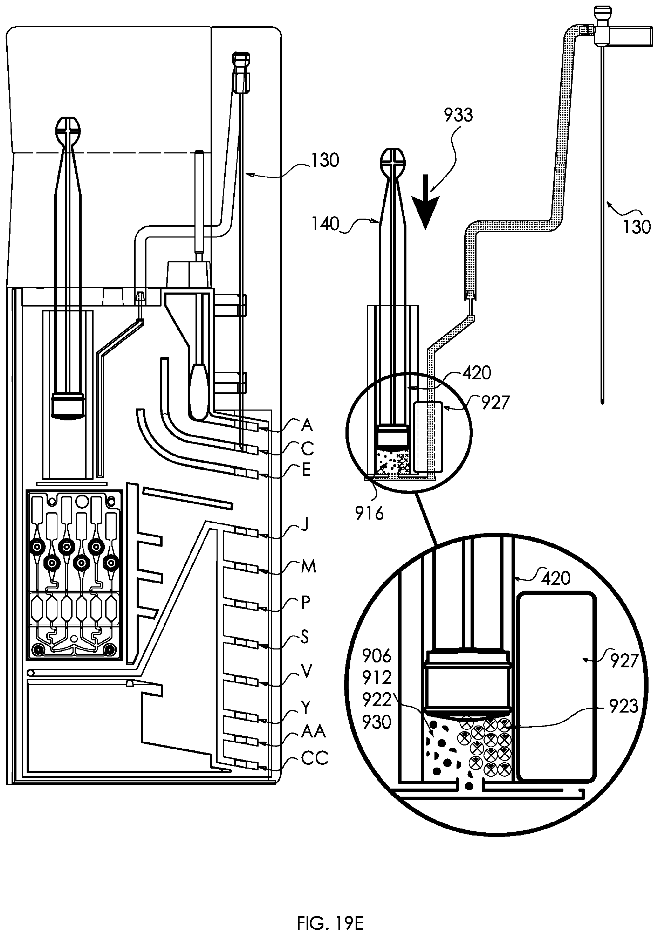

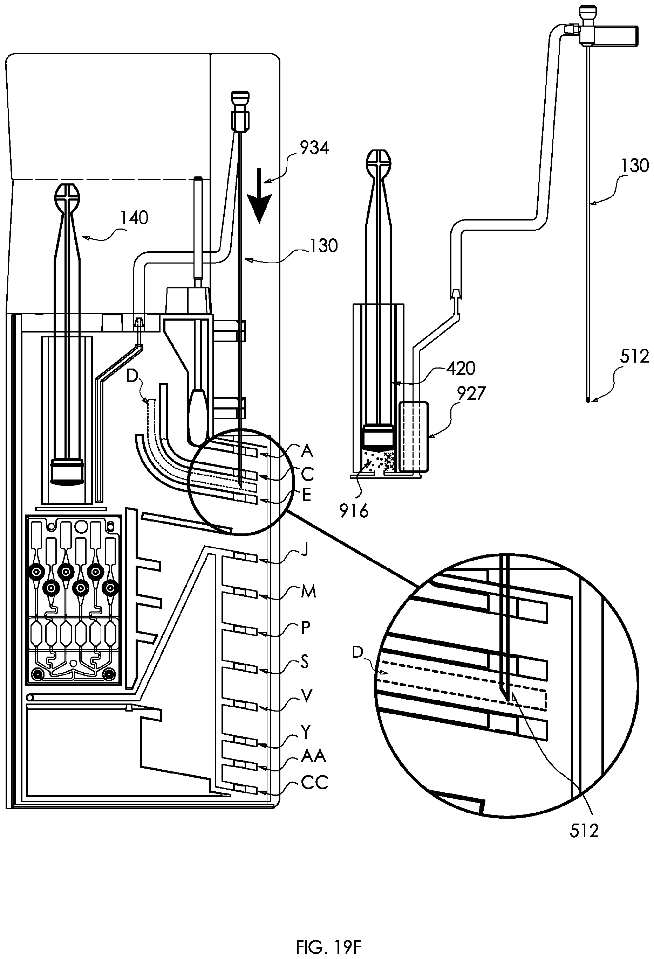

Figs. 19A, 19B, 19C, 19D, 19E and 19F are simplified illustrations of typical yet further steps in the operation of the cartridge of Figs. 1A - 15F, wherein Fig. 19A shows an operational state identical to that of Fig. 18F, Figs. 19A - 19F showing the cartridge of Figs. 1A - 15F in a viewing direction opposite to that of Figs. 18A -18F and with the first outer housing portion thereof removed and showing operative engagement with operational volumes C and D;

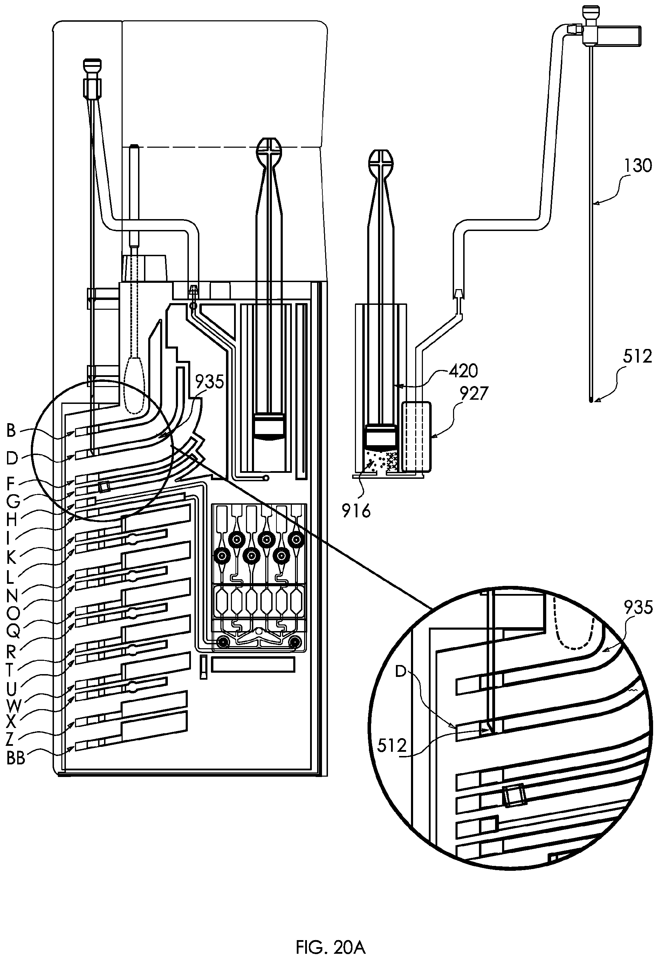

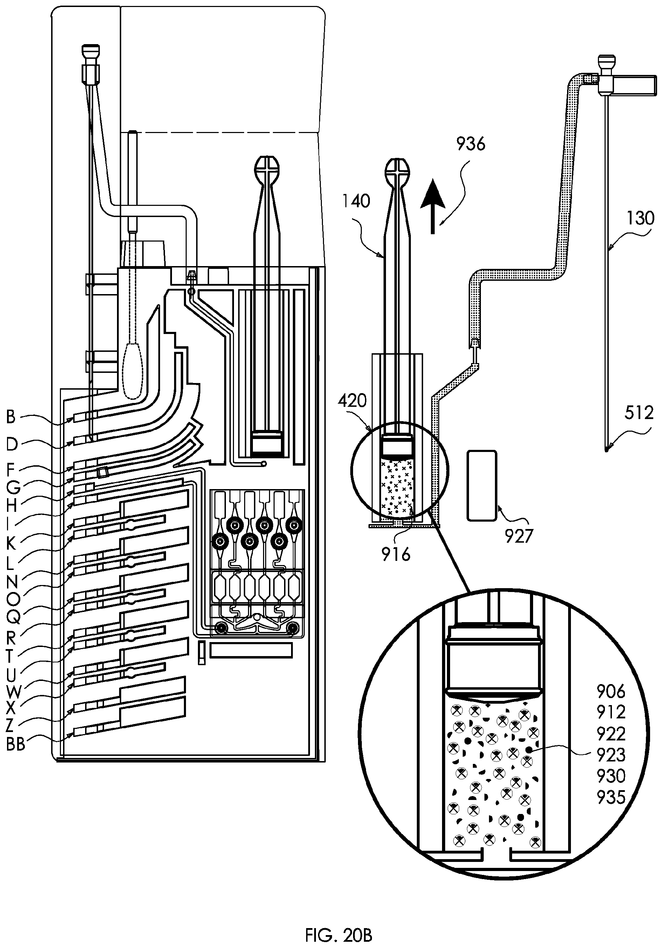

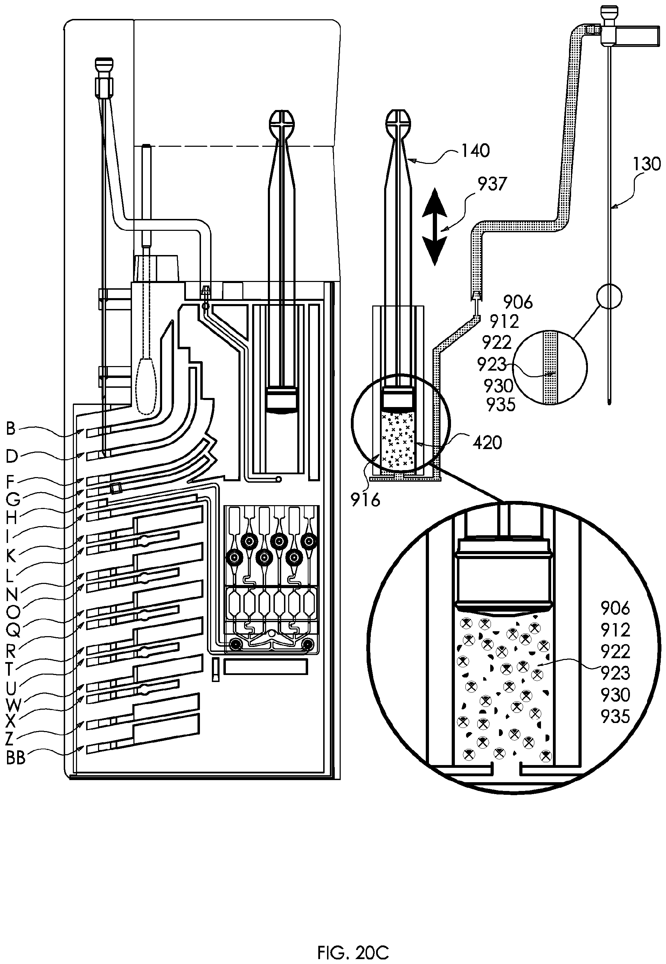

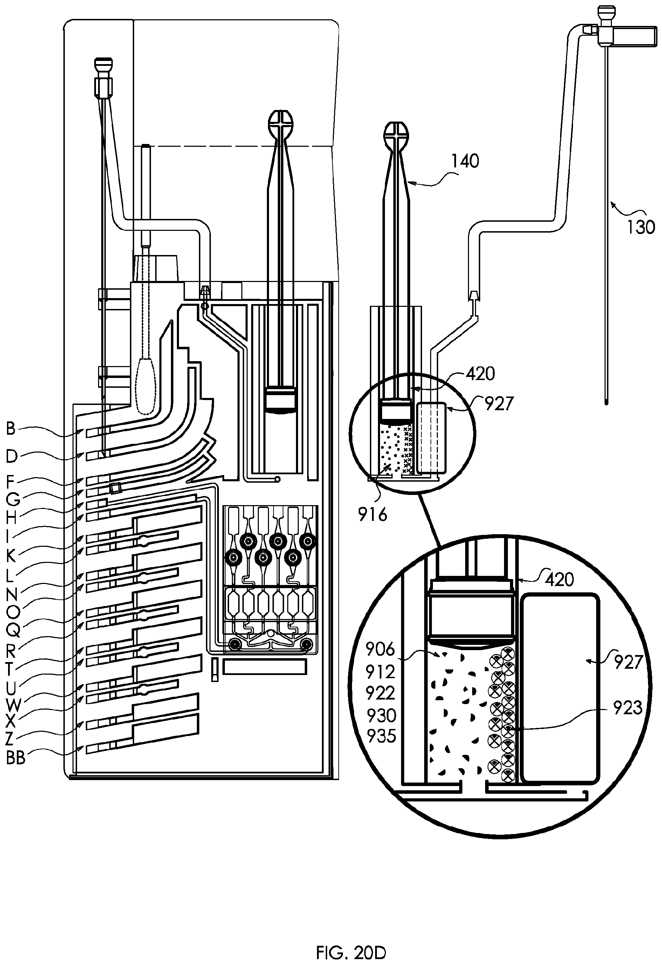

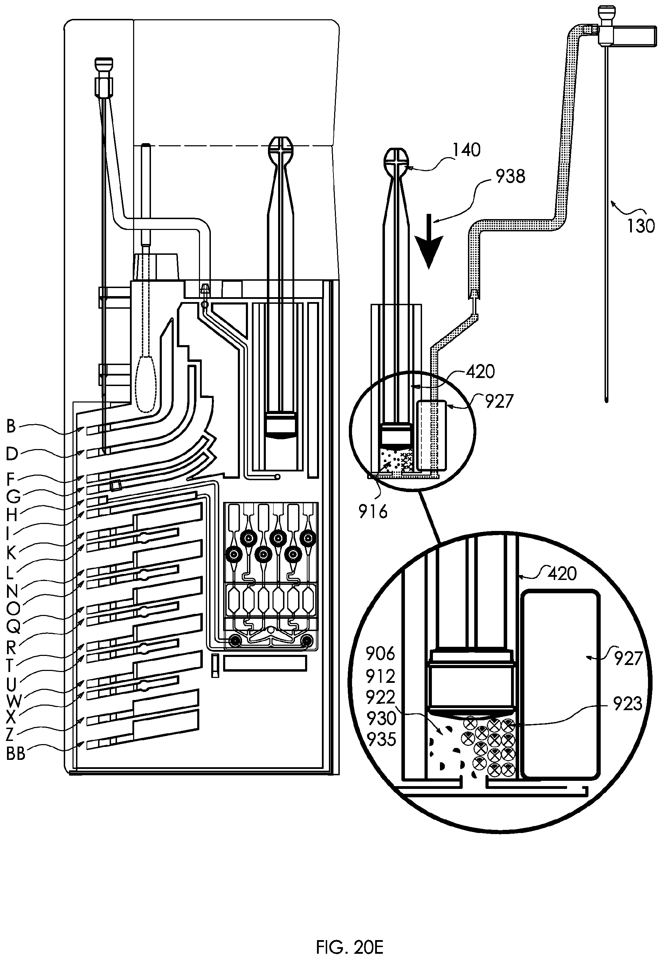

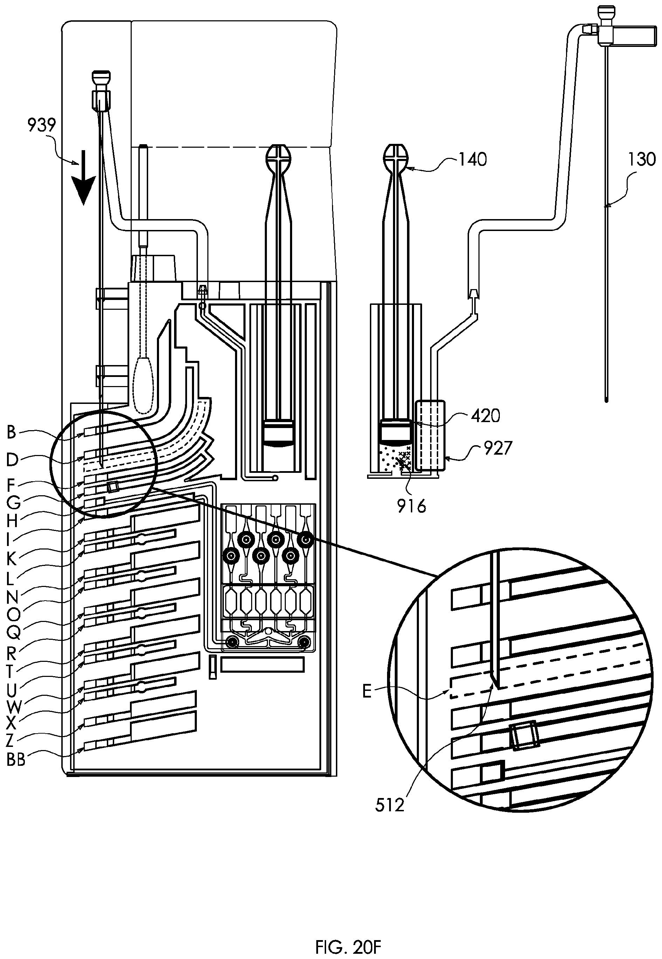

Figs. 20A, 20B, 20C, 20D, 20E and 20F are simplified illustrations of typical additional steps in the operation of the cartridge of Figs. 1A - 15F, wherein Fig. 20A shows an operational state identical to that of Fig. 19F, Figs. 20A - 20F showing the cartridge of Figs. 1A - 15F in a viewing direction opposite to that of Figs. 19A - 19F and with the second outer housing portion thereof removed and showing operative engagement with operational volumes D and E;

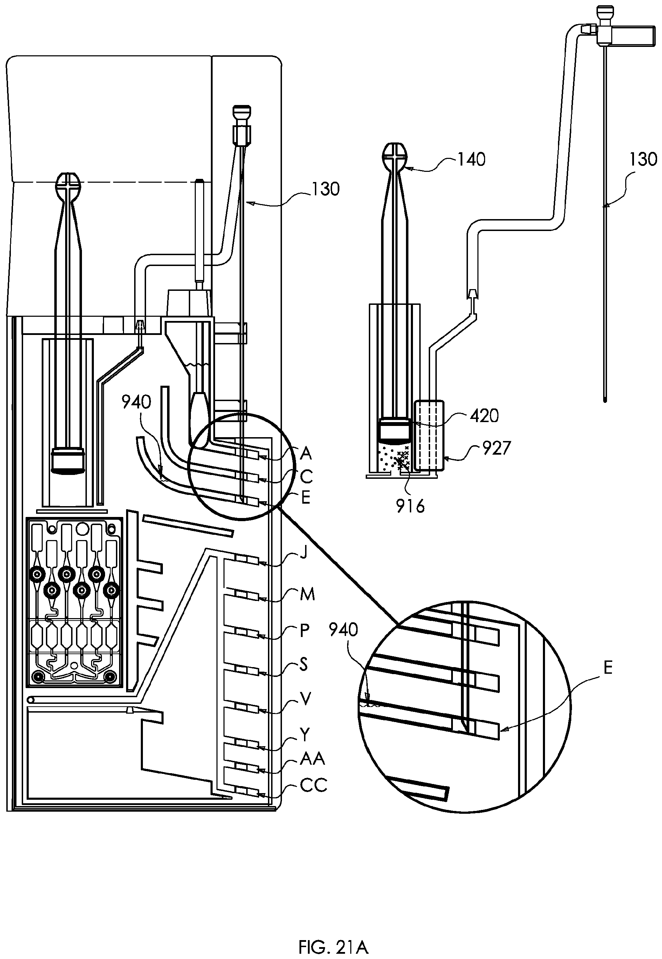

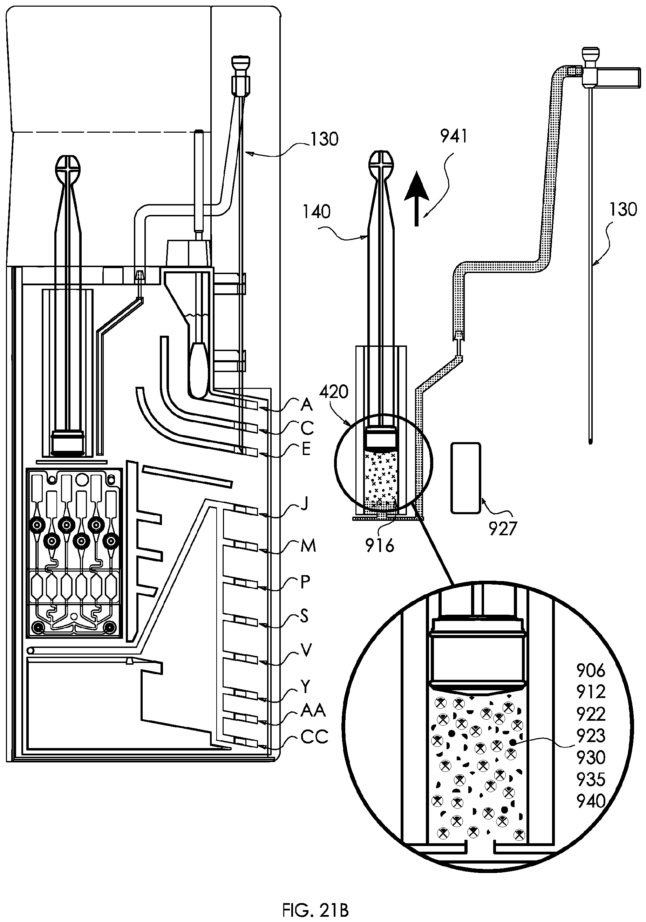

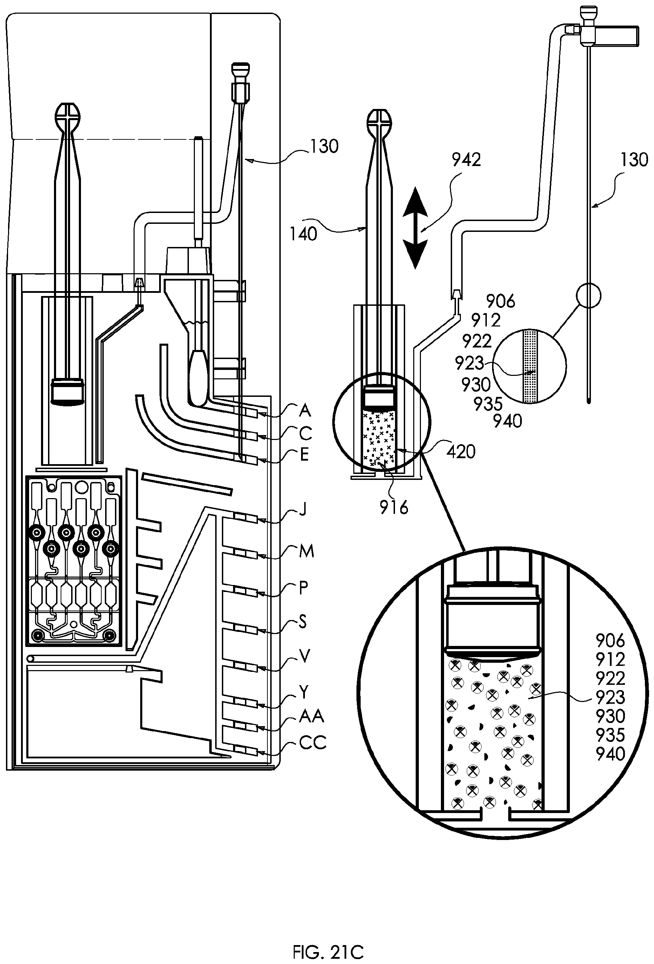

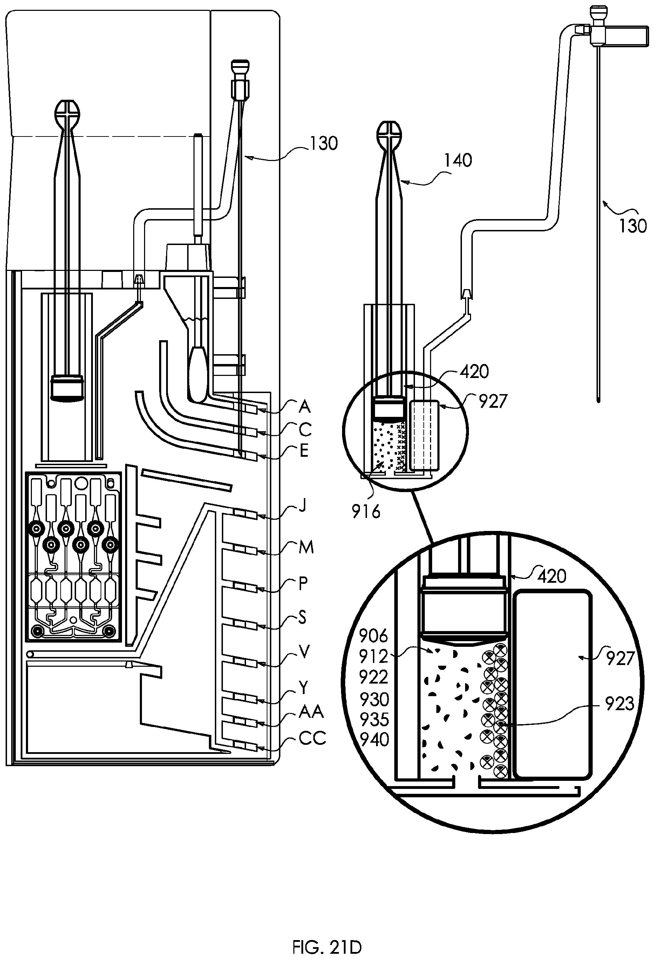

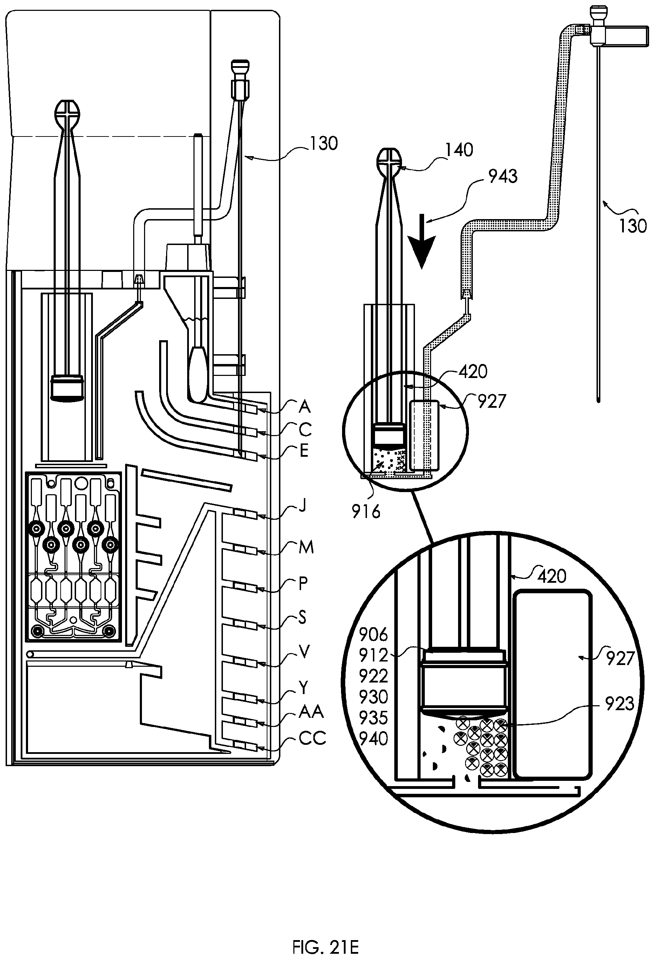

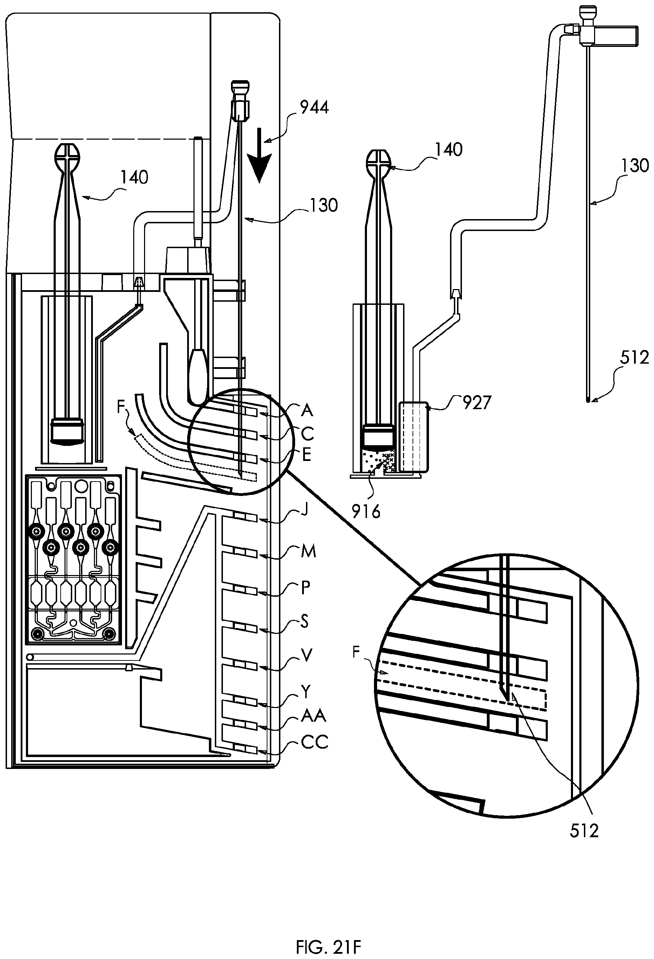

Figs. 21A, 21B, 21C, 21D, 21E and 21F are simplified illustrations of typical additional steps in the operation of the cartridge of Figs. 1A - 15F, wherein Fig. 21A shows an operational state identical to that of Fig. 20F, Figs. 21A - 21F showing the cartridge of Figs. 1A - 15F in a viewing direction opposite to that of Figs. 20A - 20F and with the first outer housing portion thereof removed and showing operative engagement with operational volumes E and F;

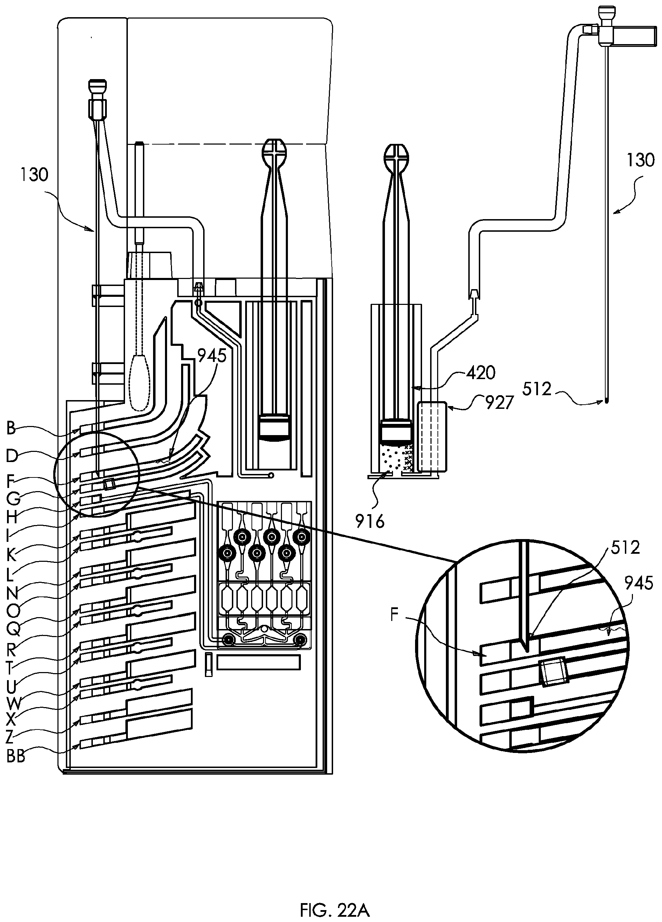

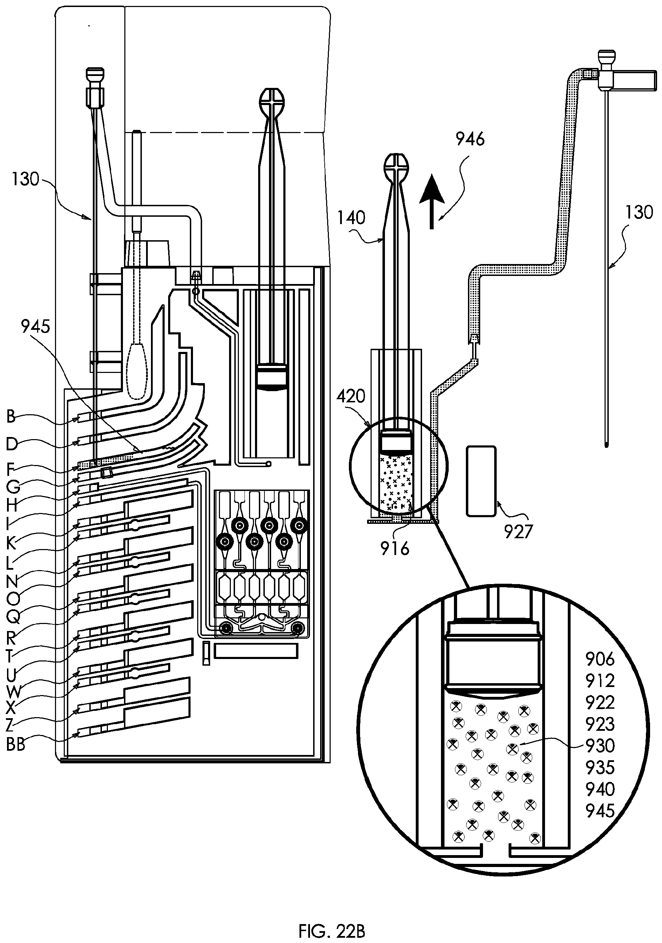

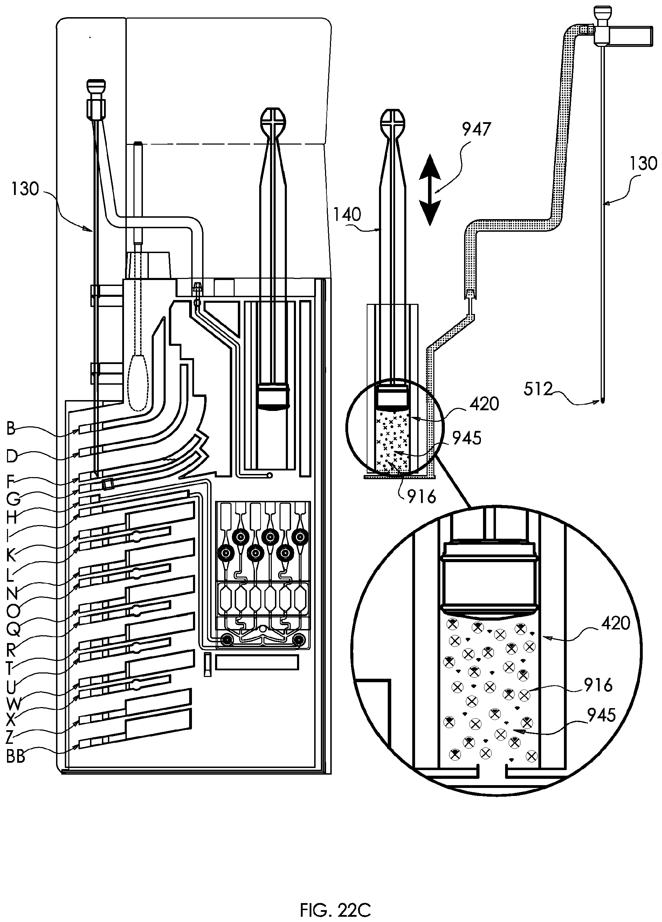

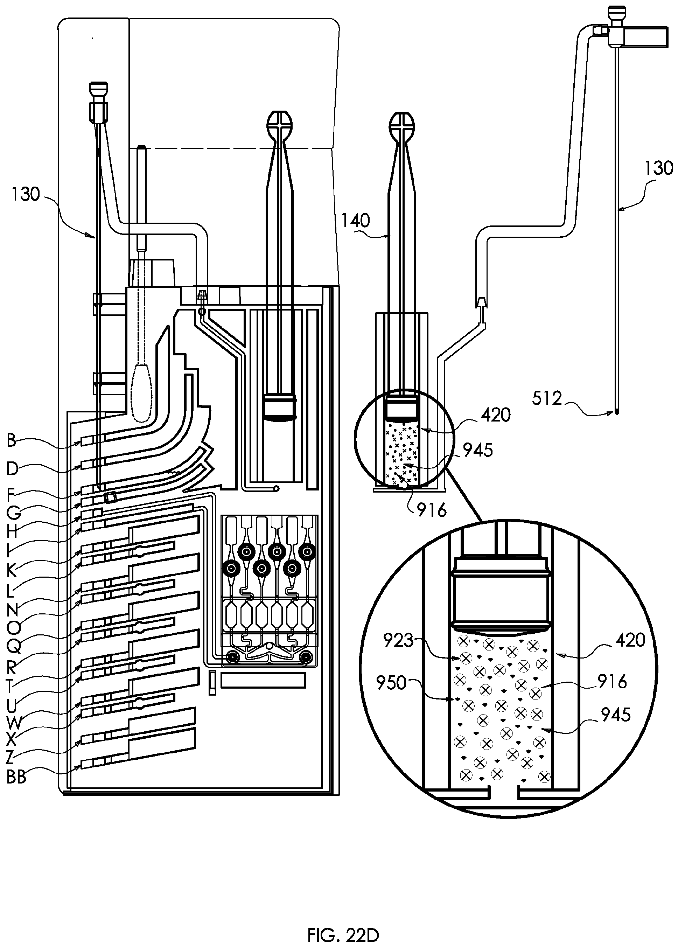

Figs. 22A, 22B, 22C and 22D are simplified illustrations of typical further steps in the operation of the cartridge of Figs. 1A - 15F, wherein Fig. 22A shows an operational state generally identical to that of Fig. 21F, Figs. 22A - 22D showing the cartridge of Figs. 1A - 15F with the second outer housing portion thereof removed and showing operative engagement with operational volumes F and G;

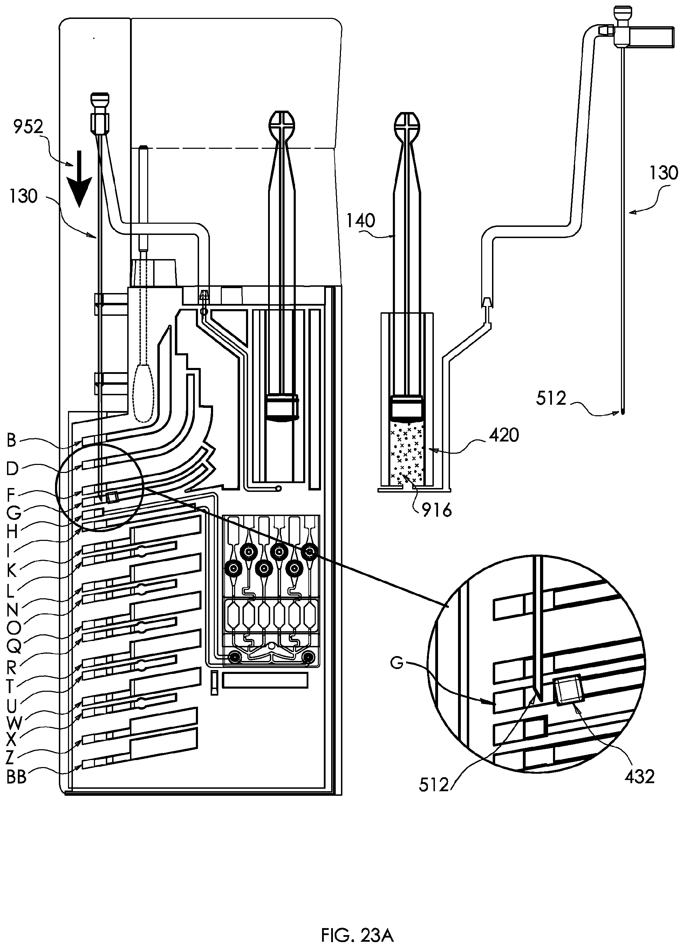

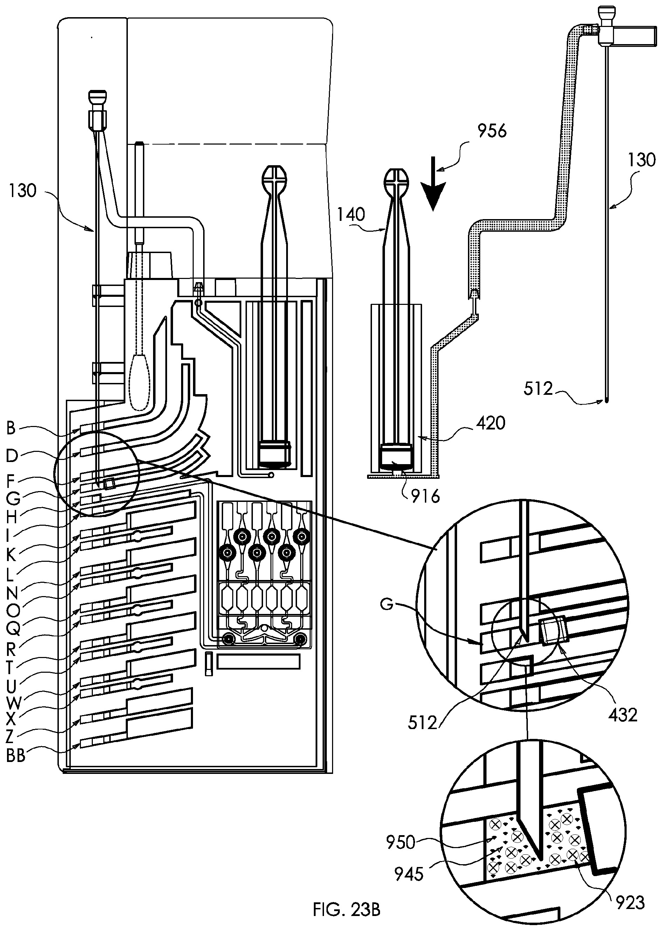

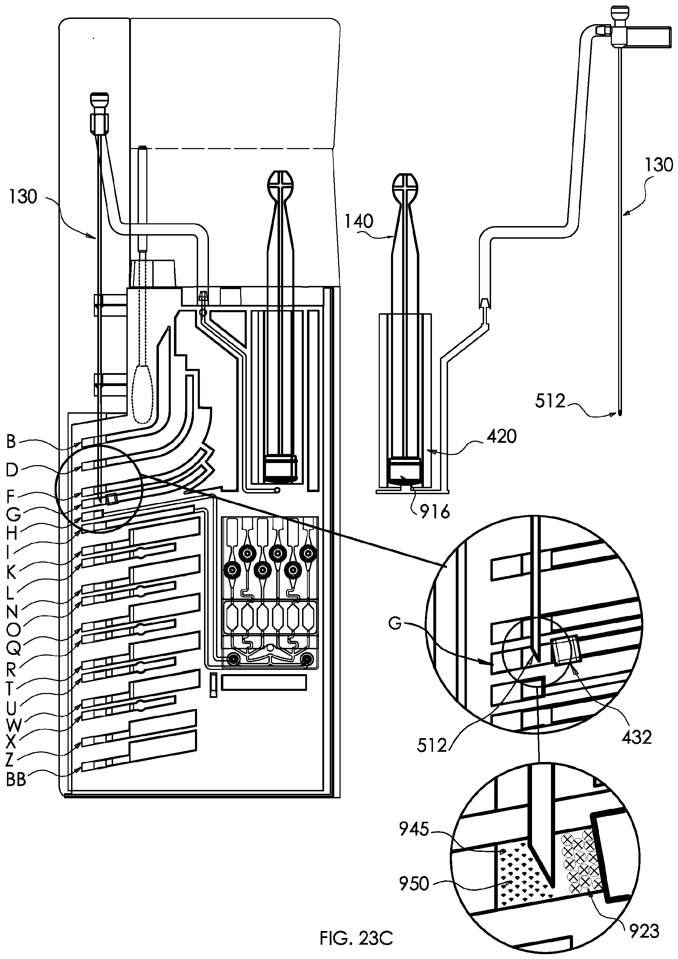

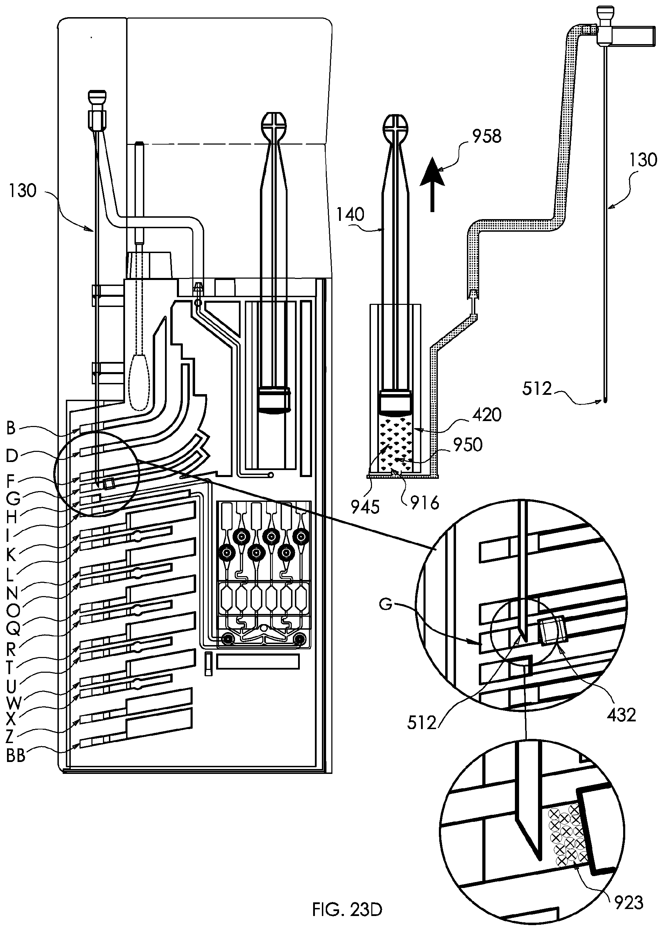

Figs. 23A, 23B, 23C and 23D are simplified illustrations of typical further steps in the operation of the cartridge of Figs. 1A - 15F, Figs. 23A - 23D showing the cartridge of Figs. 1A - 15F with the second outer housing portion thereof removed and showing operative engagement with operational volumes G and H;

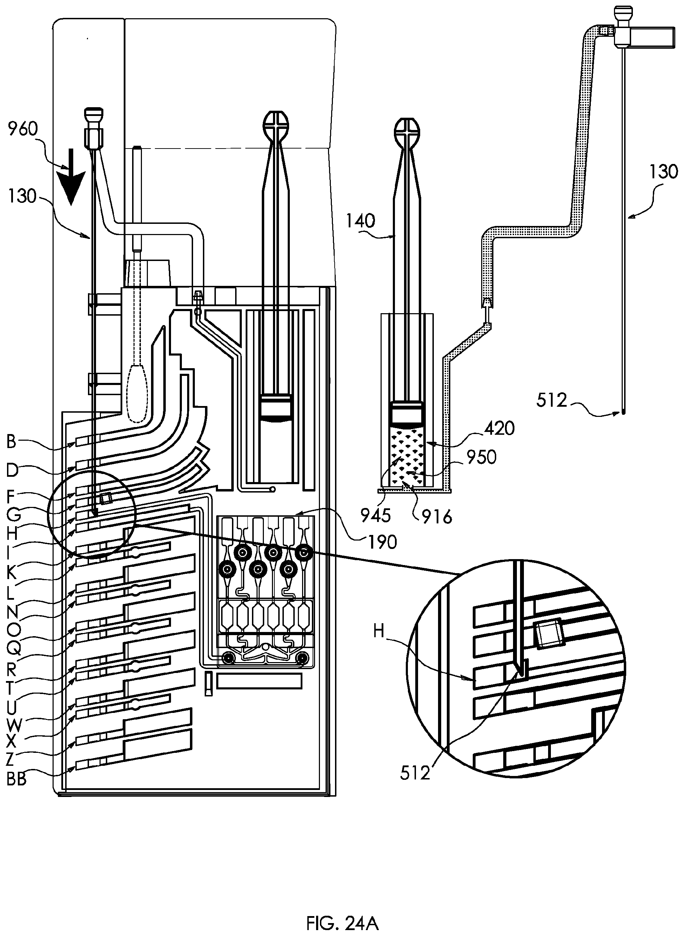

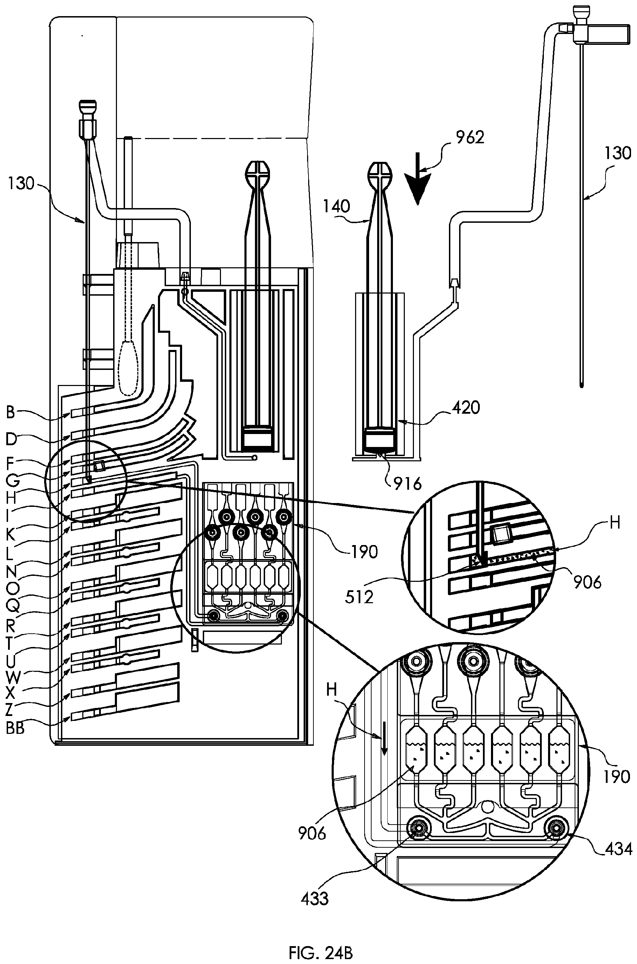

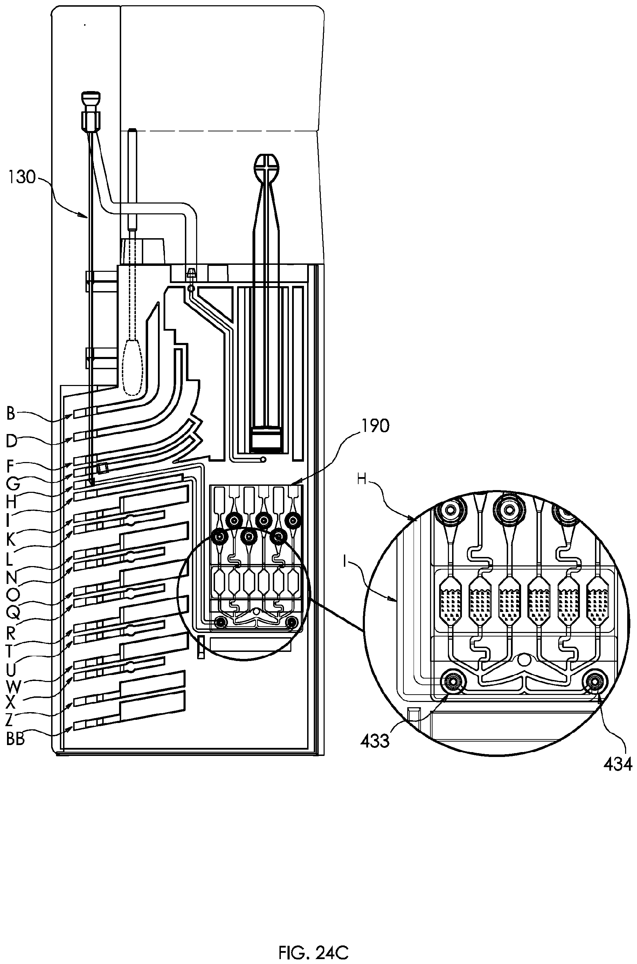

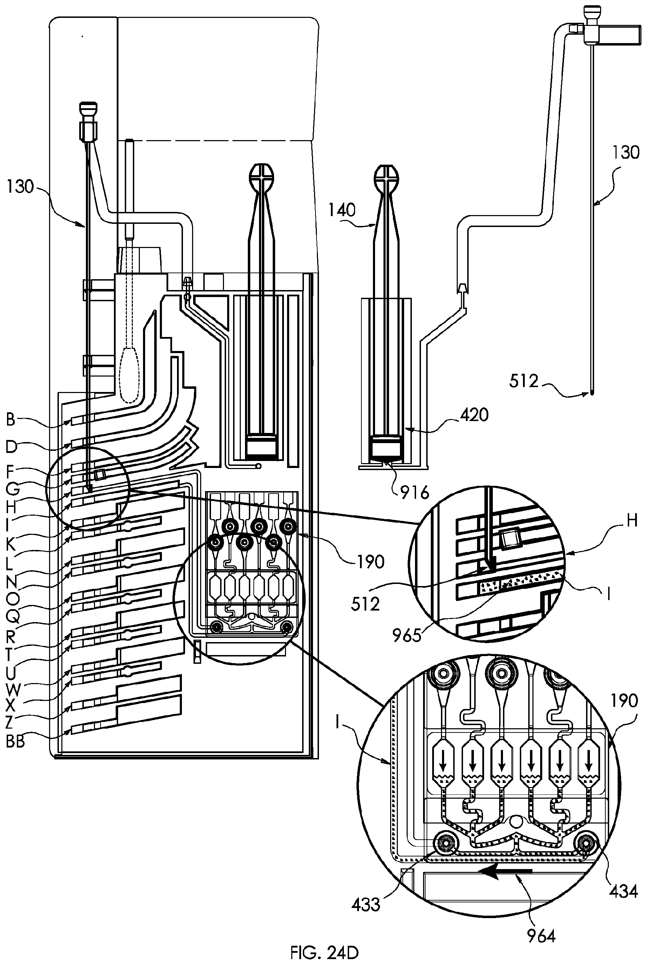

Figs. 24A, 24B, 24C and 24D are simplified illustrations of typical further steps in the operation of the cartridge of Figs. 1A - 15F, Figs. 24A - 24C showing the cartridge of Figs. 1A - 15F with the second outer housing portion thereof removed and showing operative engagement with operational volumes H and I;

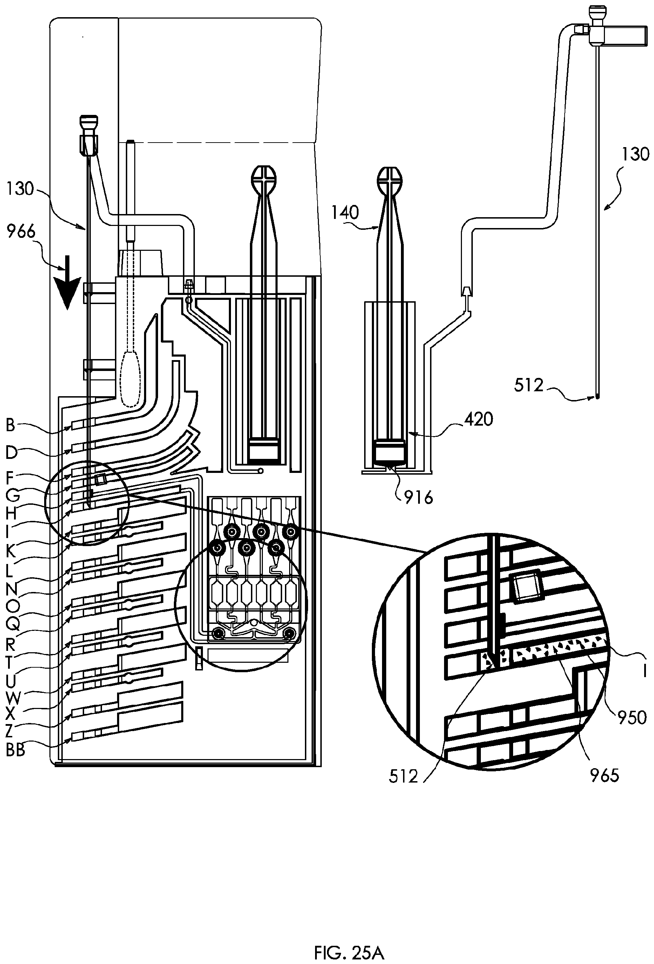

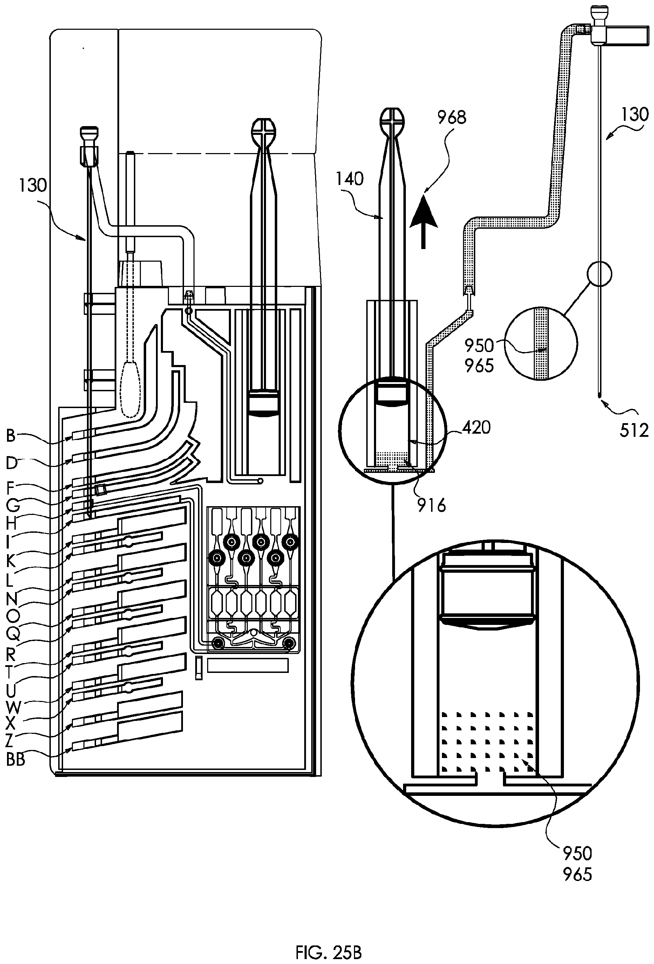

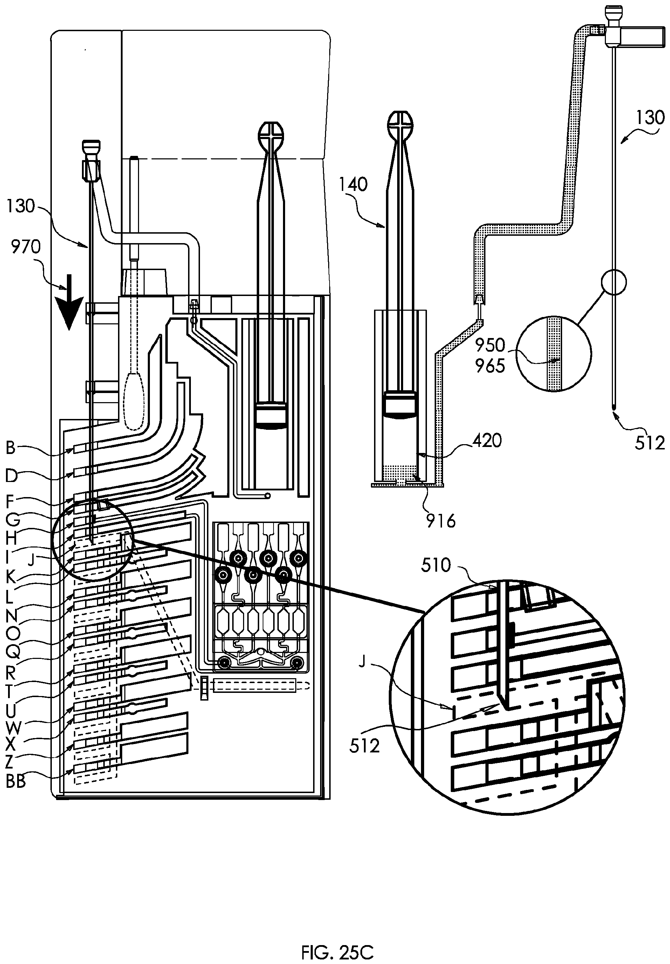

Figs. 25A, 25B and 25C are simplified illustrations of typical additional steps in the operation of the cartridge of Figs. 1A - 15F, with the second outer housing portion thereof removed and showing operative engagement with operational volumes I and J;

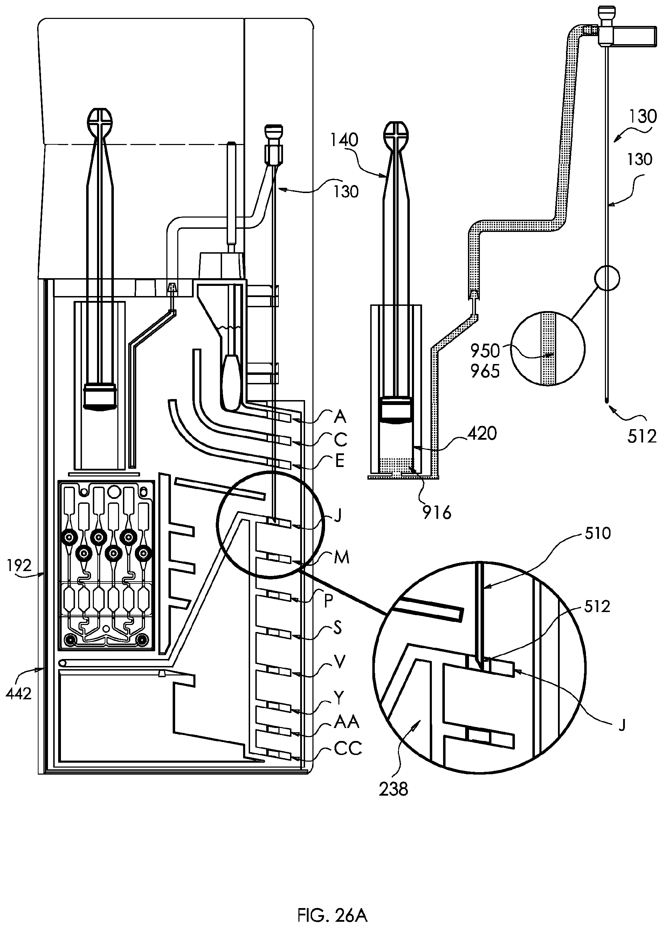

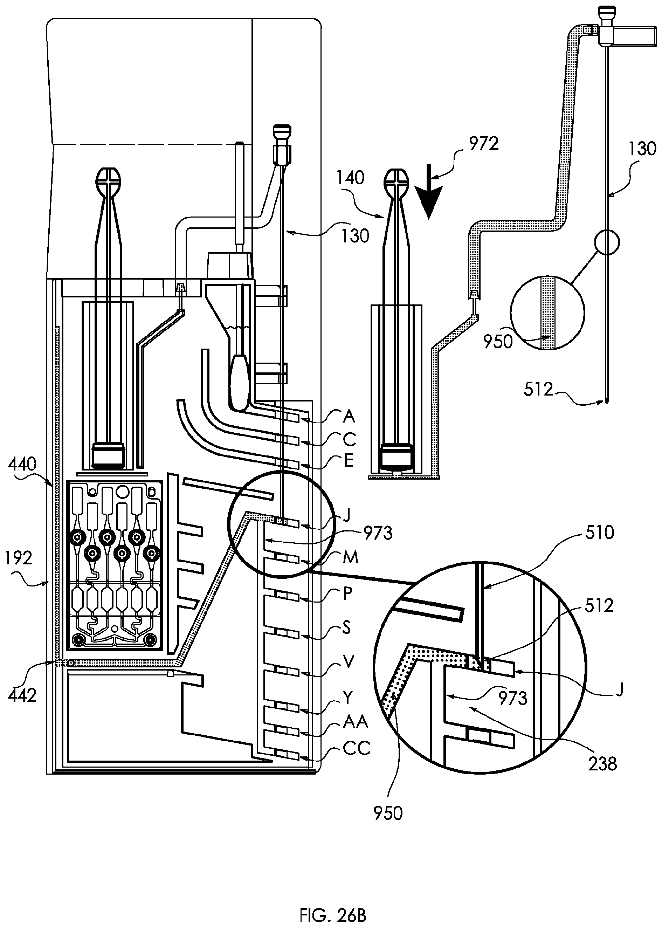

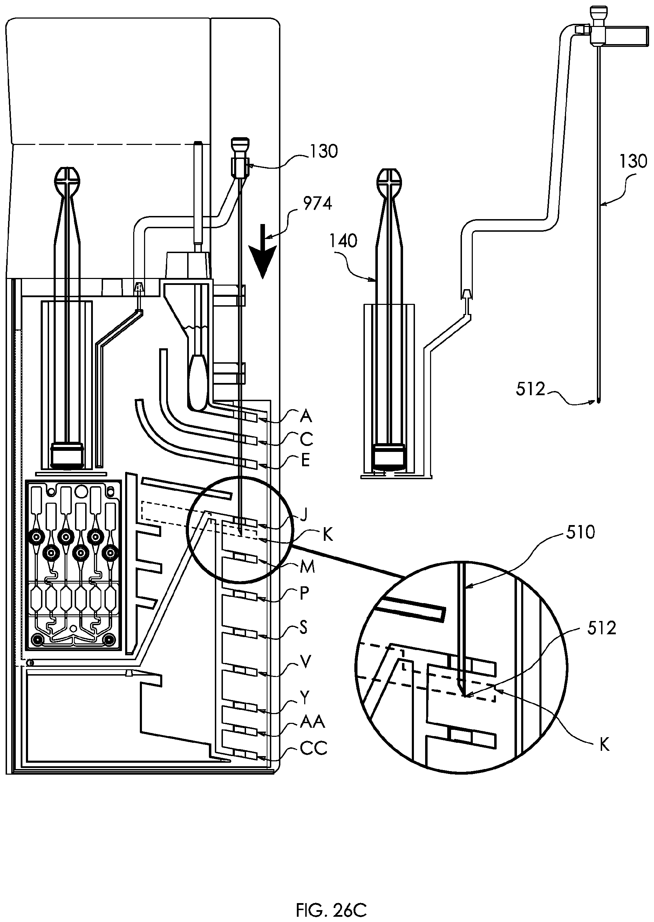

Figs. 26A, 26B and 26C are simplified illustrations of typical additional steps in the operation of the cartridge of Figs. 1A - 15F, wherein Fig. 26A shows an operational state identical to that of Fig. 25C, Figs. 26A - 26C showing the cartridge of Figs. 1A - 15F in a viewing direction opposite to that of Figs. 25A - 25C and with the first outer housing portion thereof removed and showing operative engagement with operational volumes J and K;

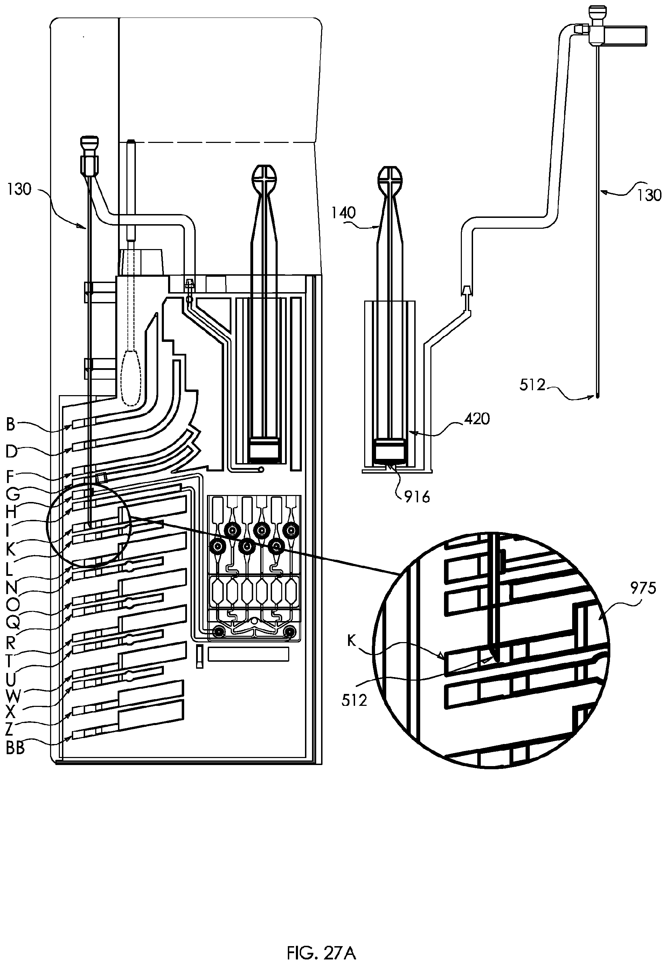

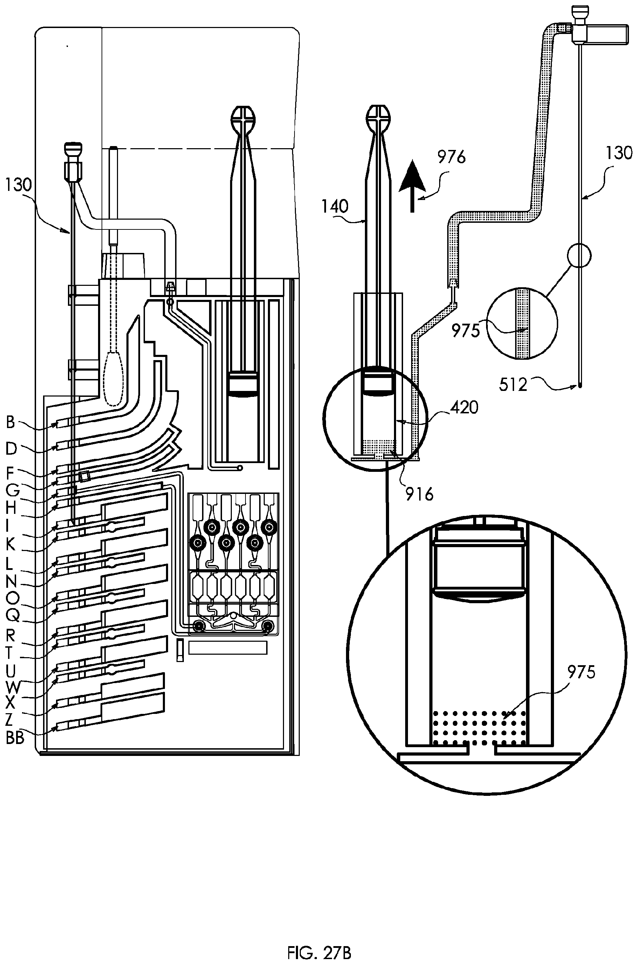

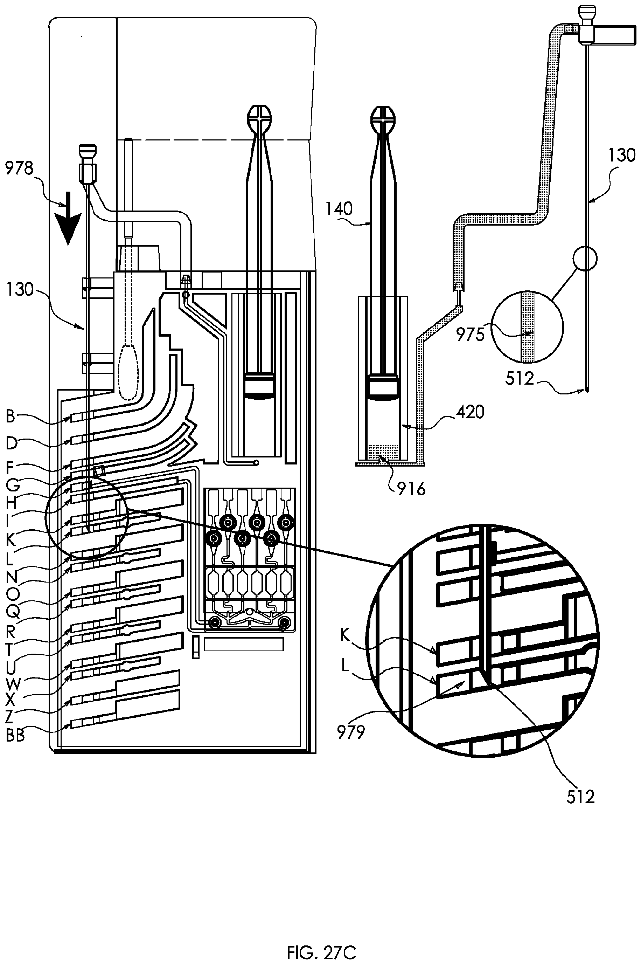

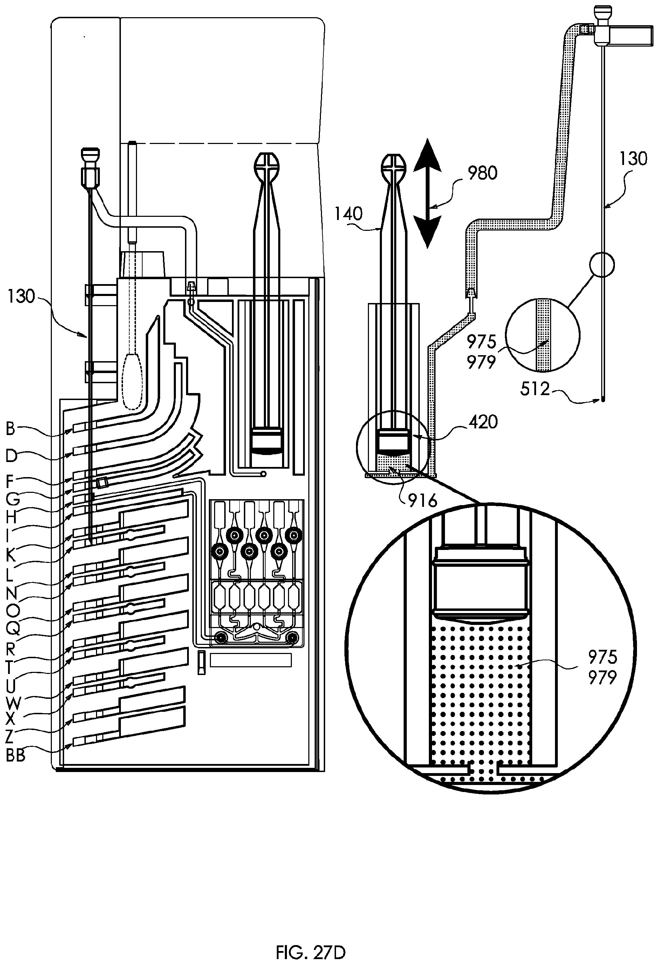

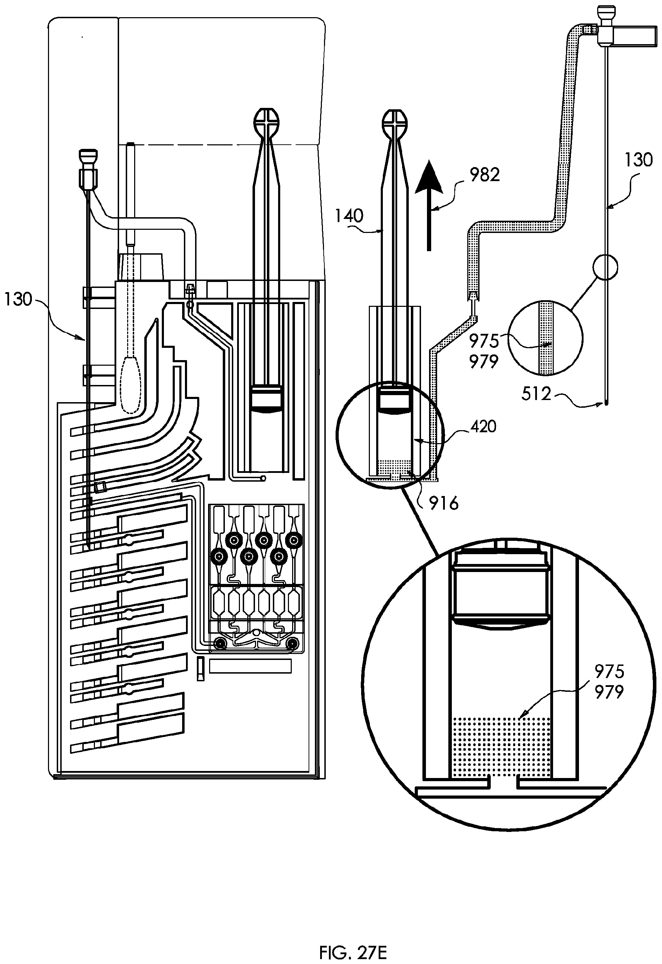

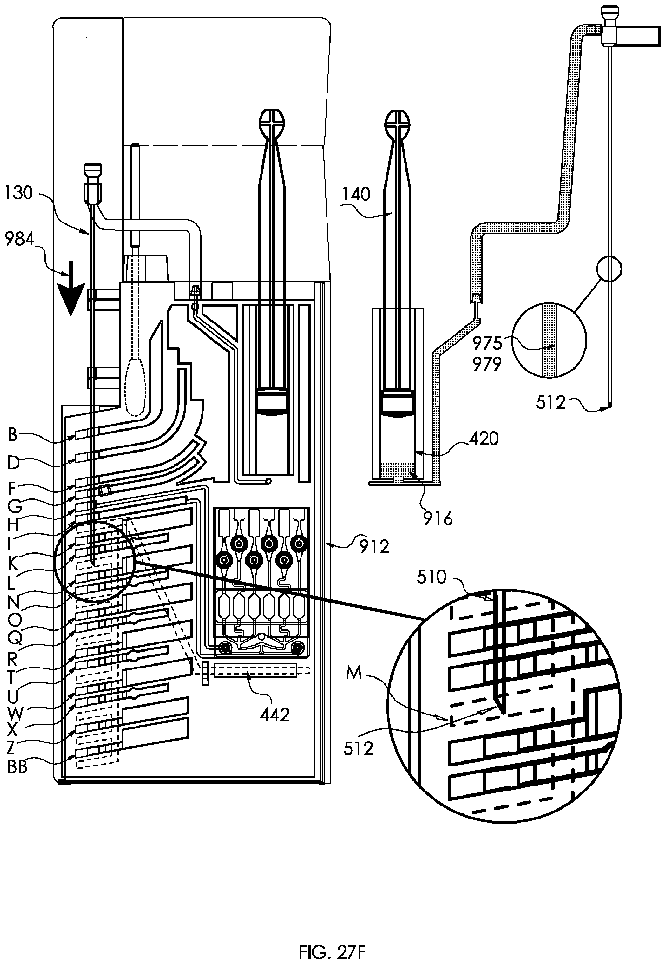

Figs. 27A, 27B, 27C, 27D, 27E and 27F are simplified illustrations of typical additional steps in the operation of the cartridge of Figs. 1A - 15F, wherein Fig. 27A shows an operational state identical to that of Fig. 26C, Figs. 27A - 27F showing the cartridge of Figs. 1A - 15F with the second outer housing portion thereof removed and showing operative engagement with operational volumes K, L and M;

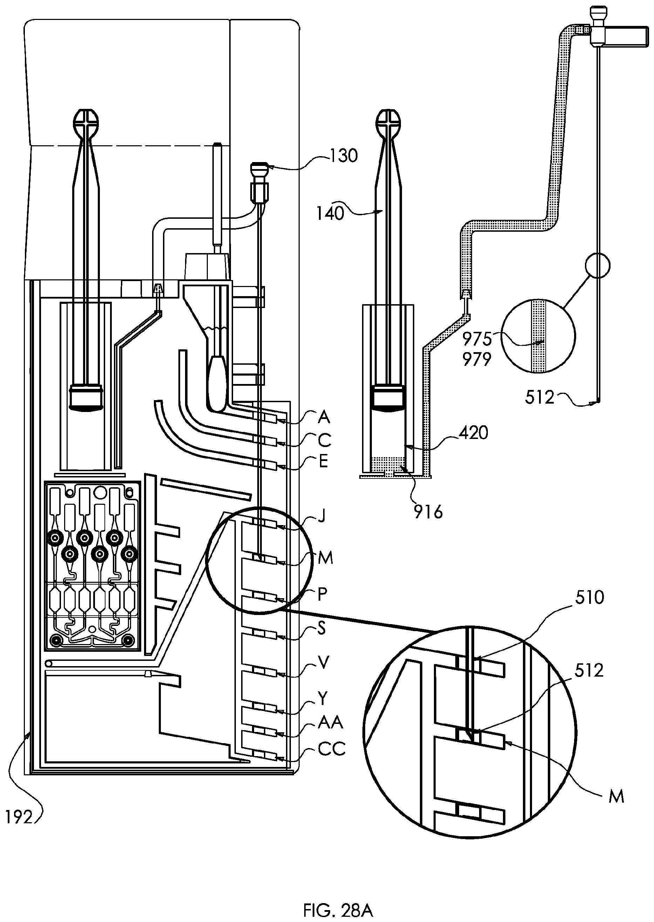

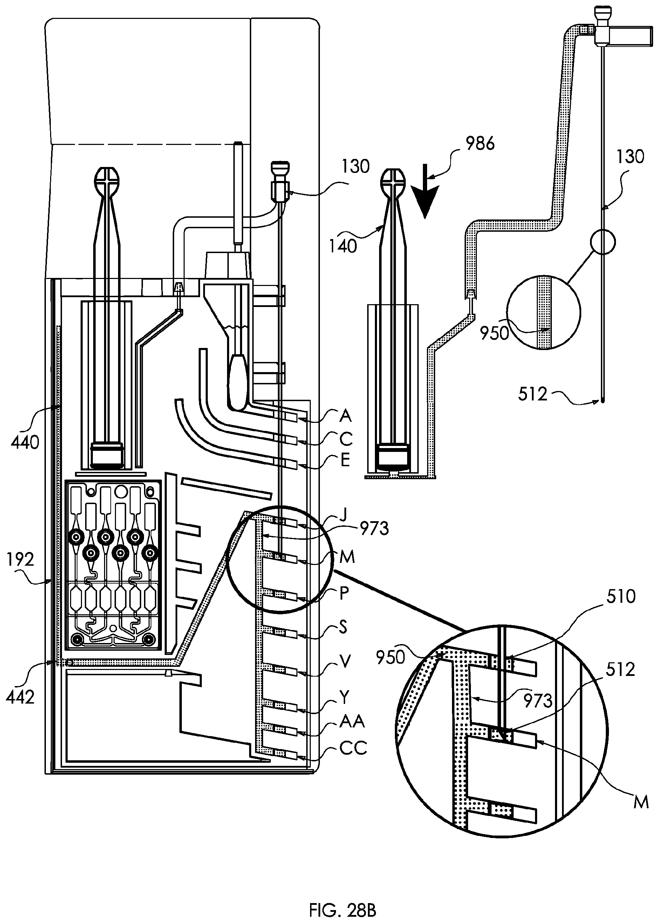

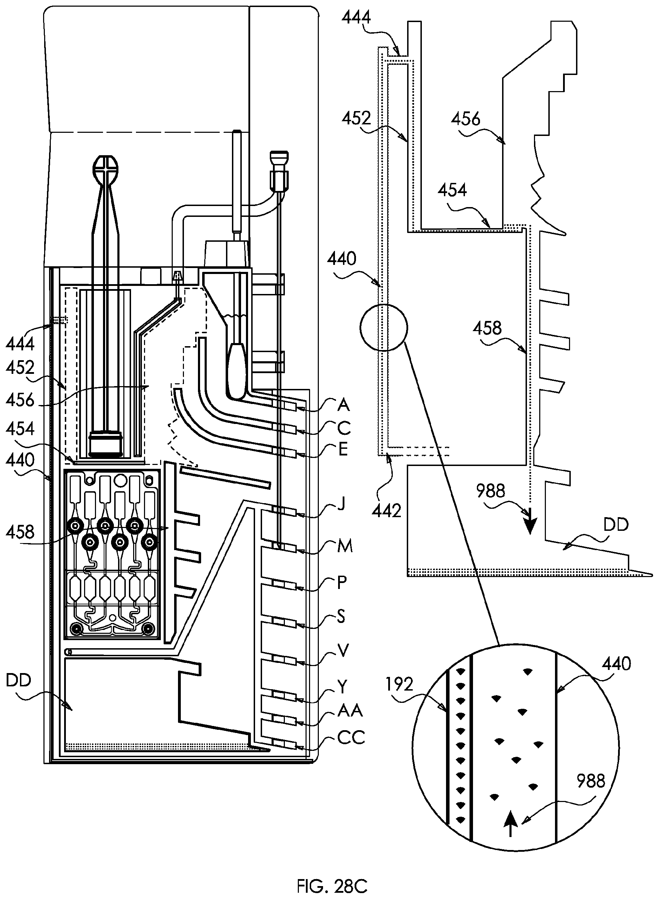

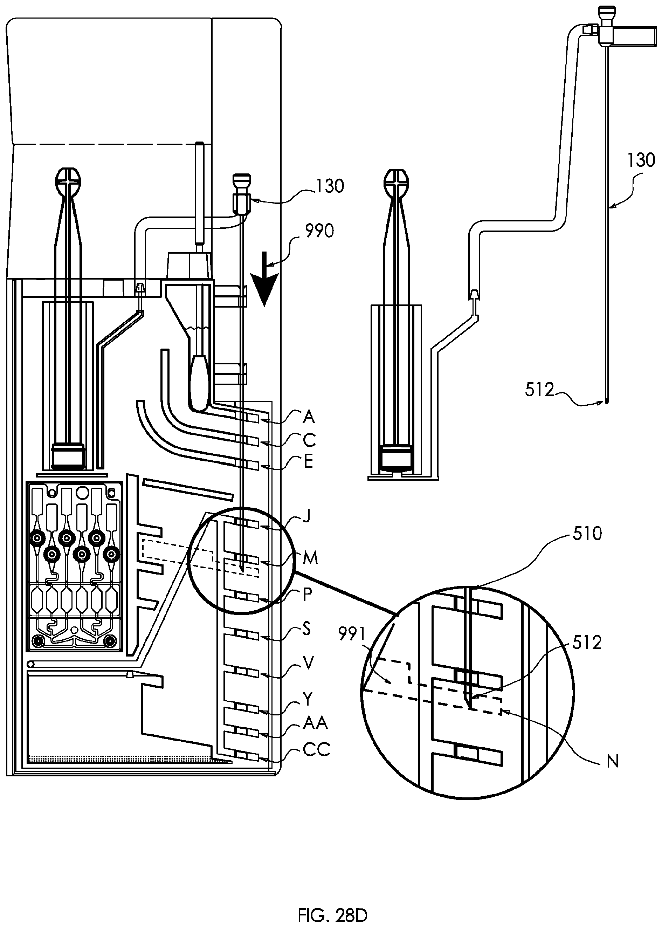

Figs. 28A, 28B, 28C and 28D are simplified illustrations of typical additional steps in the operation of the cartridge of Figs. 1A - 15F, wherein Fig. 28A shows an operational state identical to that of Fig. 27F, Figs. 28A - 28D showing the cartridge of Figs. 1A - 15F in a viewing direction opposite to that of Figs. 27A - 27F and with the first outer housing portion thereof removed and showing operative engagement with operational volumes M and N;

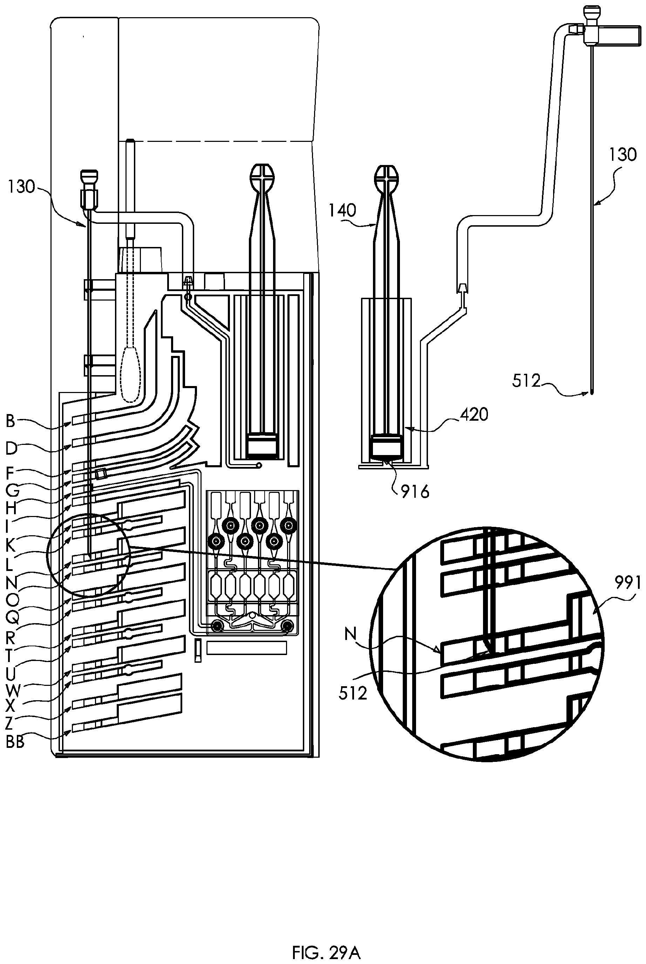

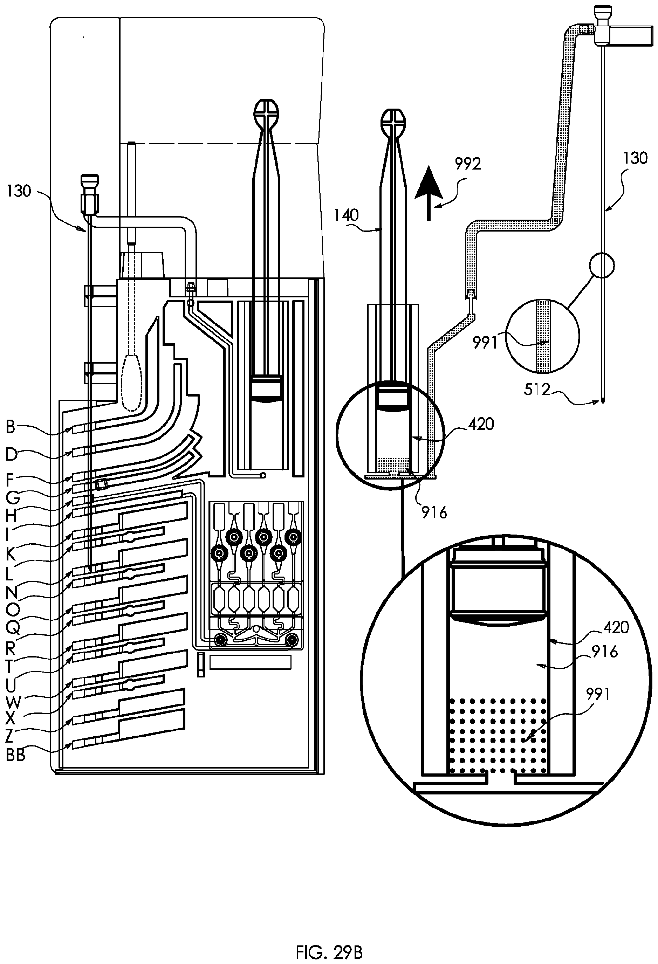

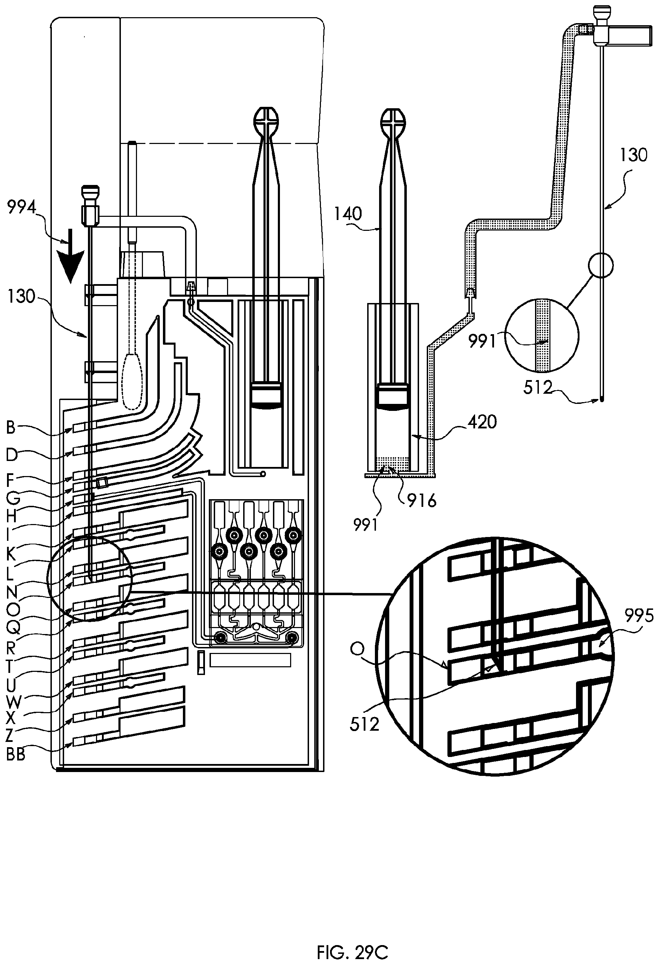

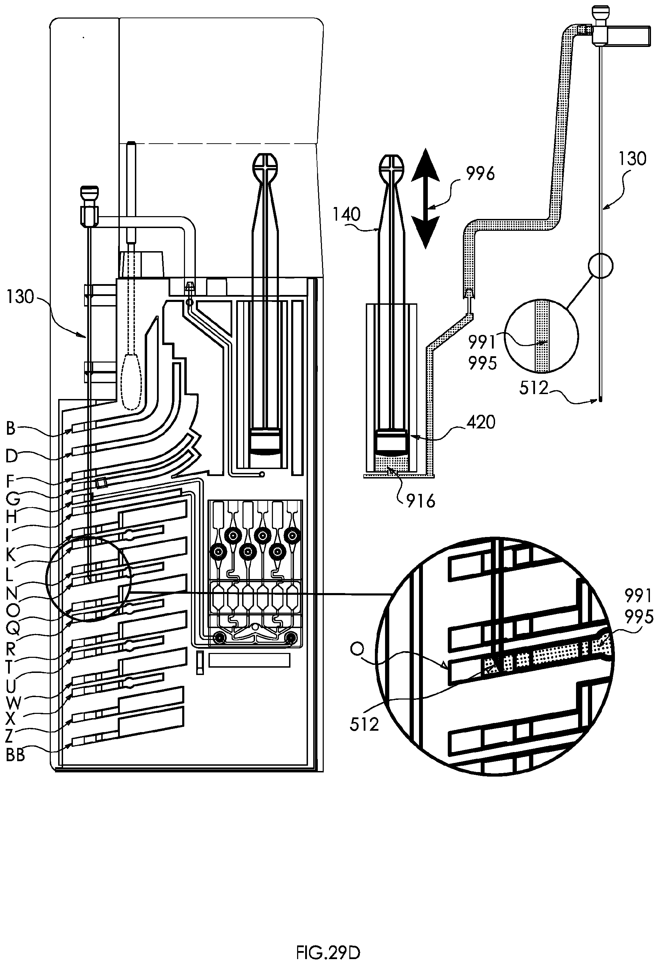

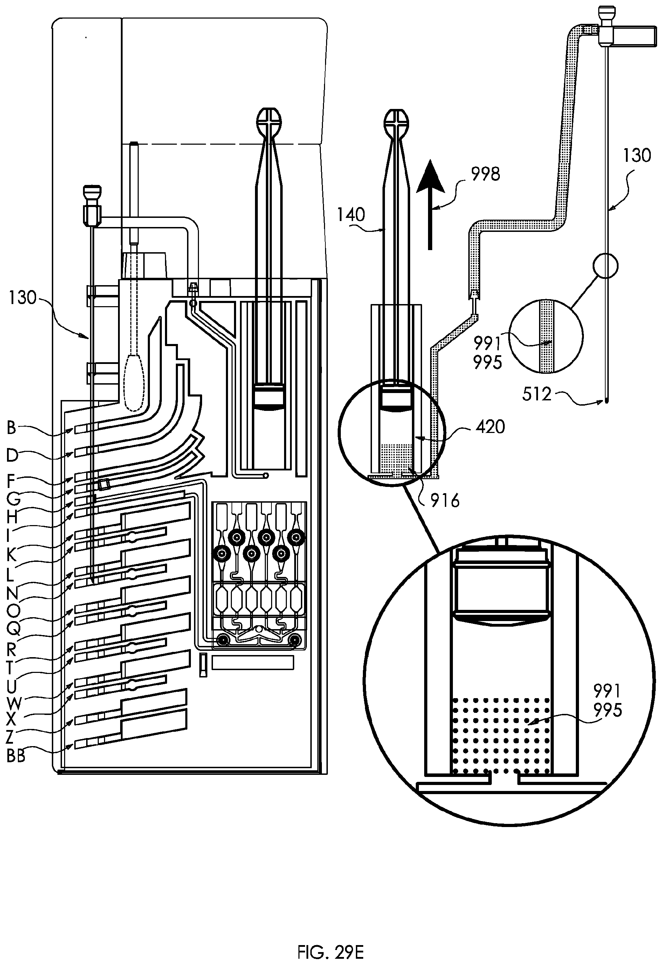

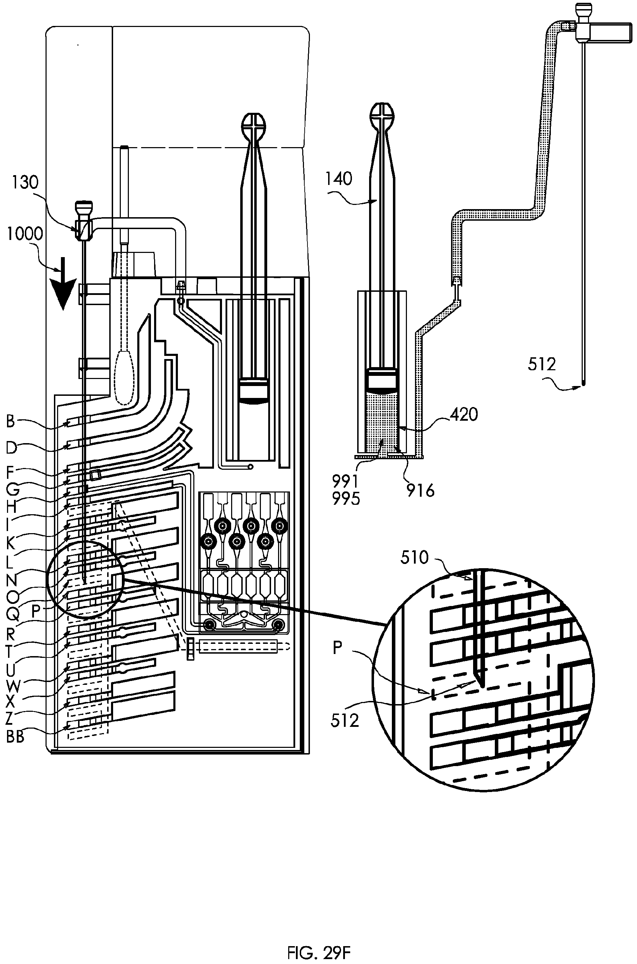

Figs. 29A, 29B, 29C, 29D, 29E and 29F are simplified illustrations of typical additional steps in the operation of the cartridge of Figs. 1A - 15F, wherein Fig. 29A shows an operational state identical to that of Fig. 28D, Figs. 29A - 29F showing the cartridge of Figs. 1A - 15F in a viewing direction opposite to that of Figs. 28A - 28D and with the second outer housing portion thereof removed and showing operative engagement with operational volumes N, O and P;

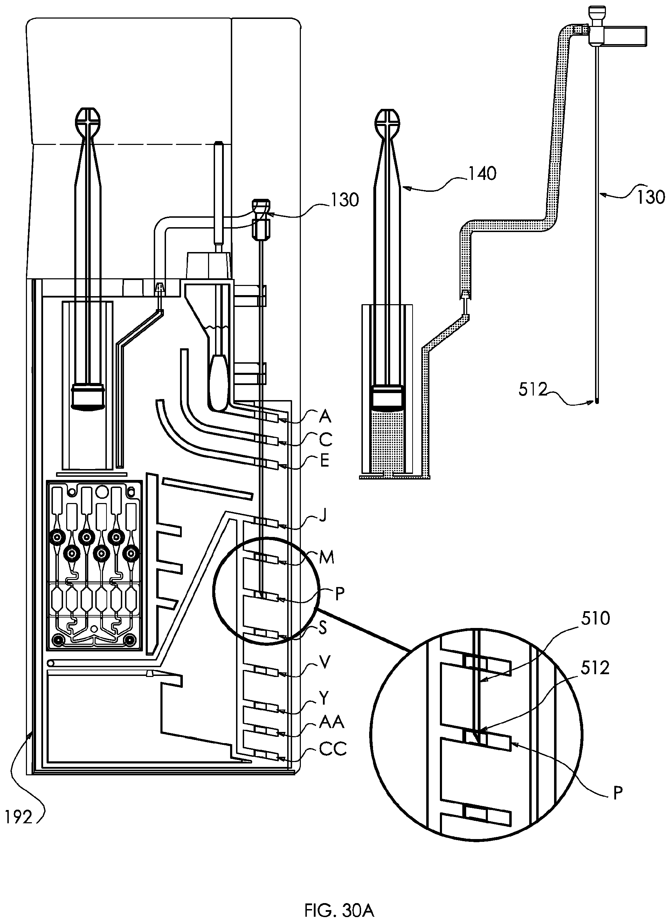

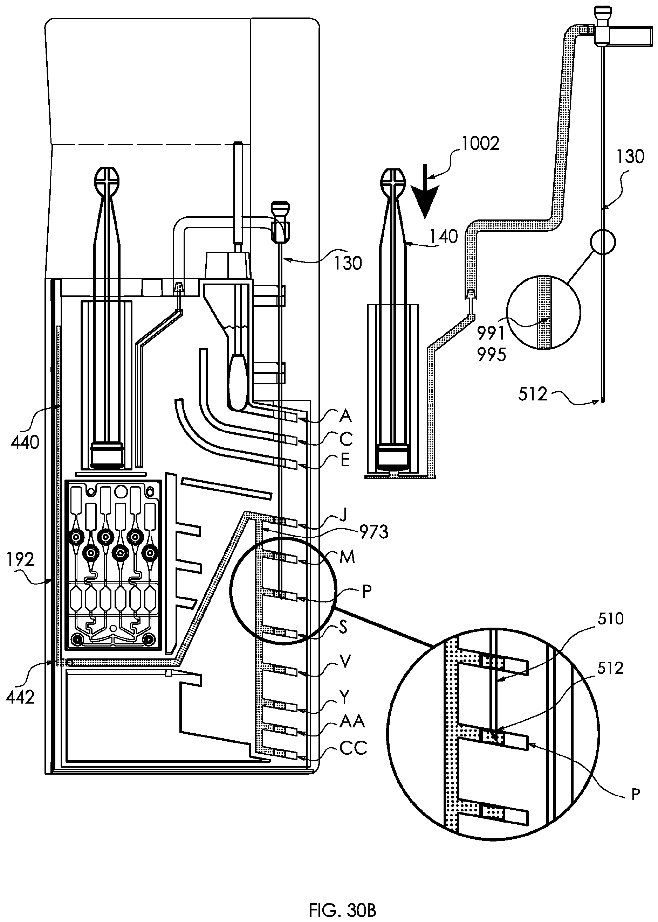

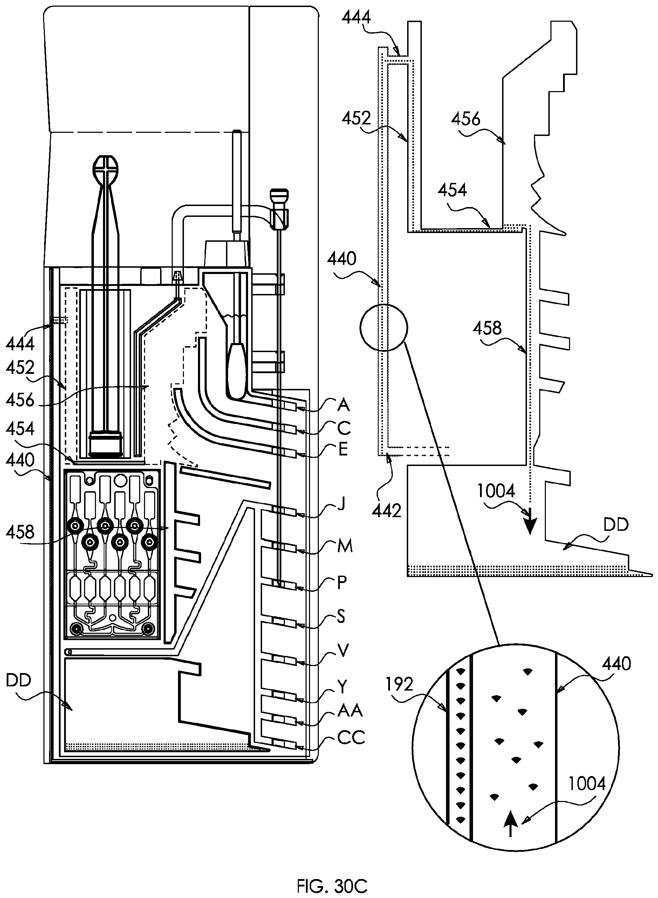

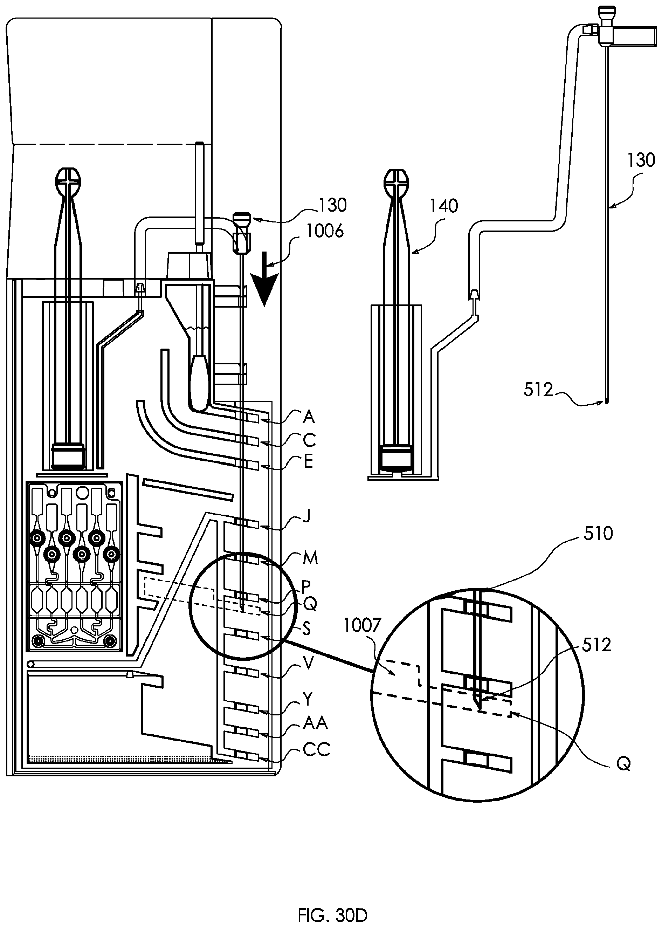

Figs. 30A, 30B, 30C and 30D are simplified illustrations of typical additional steps in the operation of the cartridge of Figs. 1A - 15F, wherein Fig. 30A shows an operational state identical to that of Fig. 29F, Figs. 30A - 30D showing the cartridge of Figs. 1A - 15F in a viewing direction opposite to that of Figs. 29A - 29F with the first outer housing portion thereof removed and showing operative engagement with operational volumes P and Q;

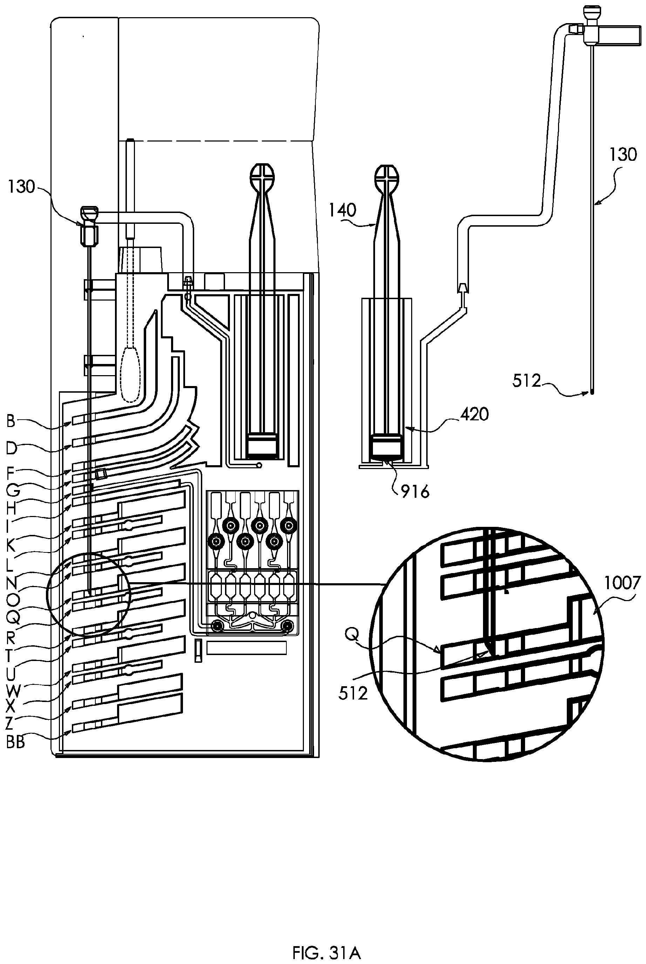

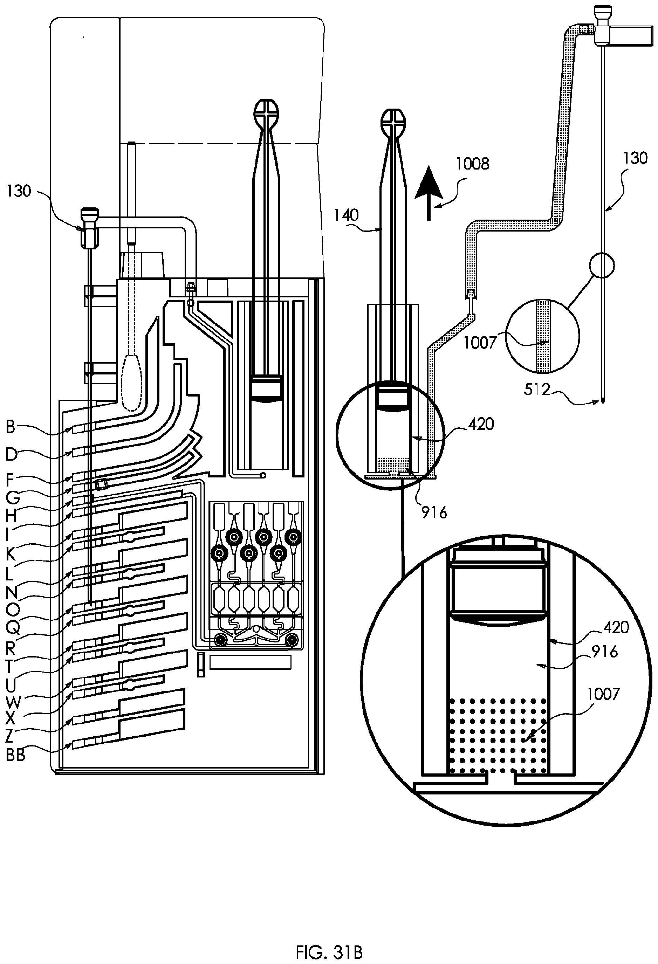

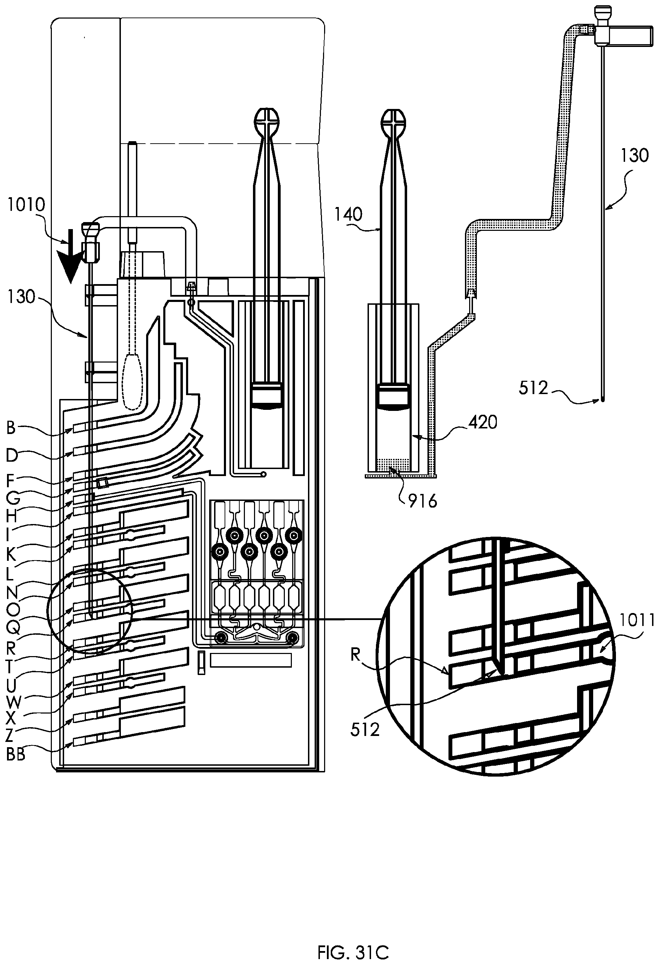

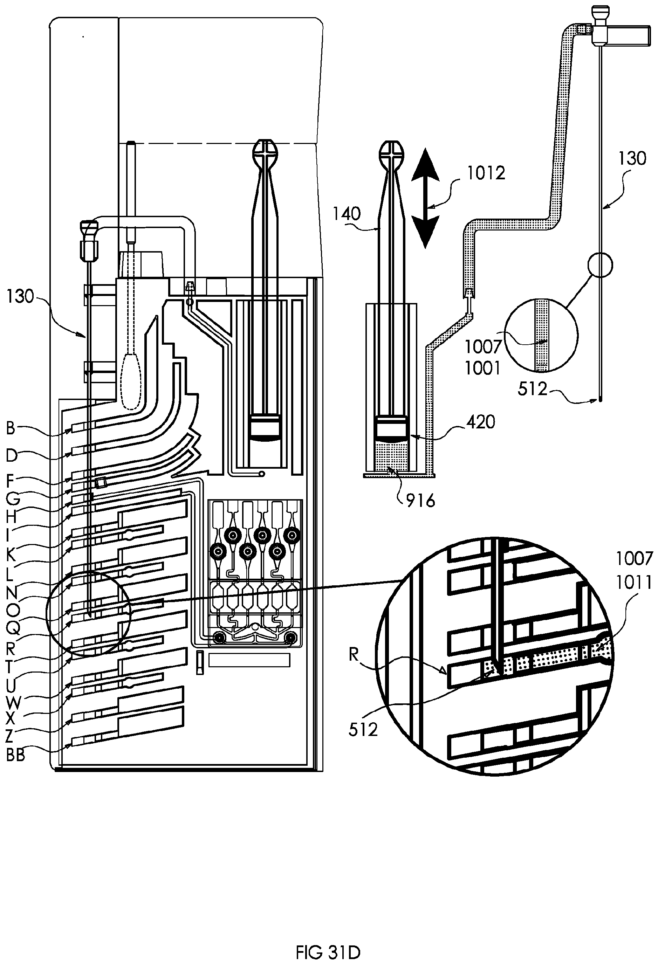

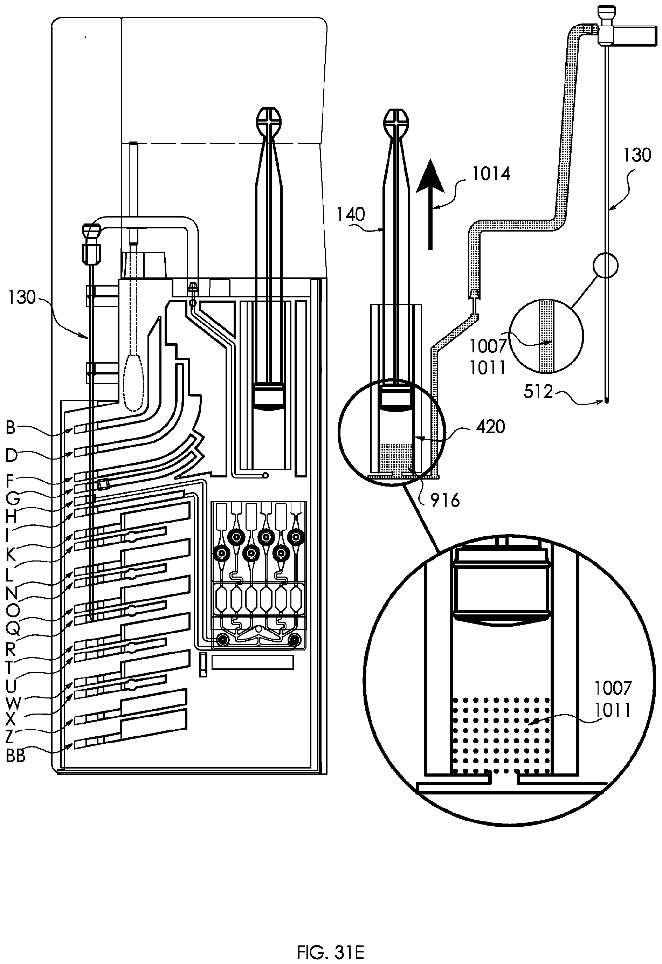

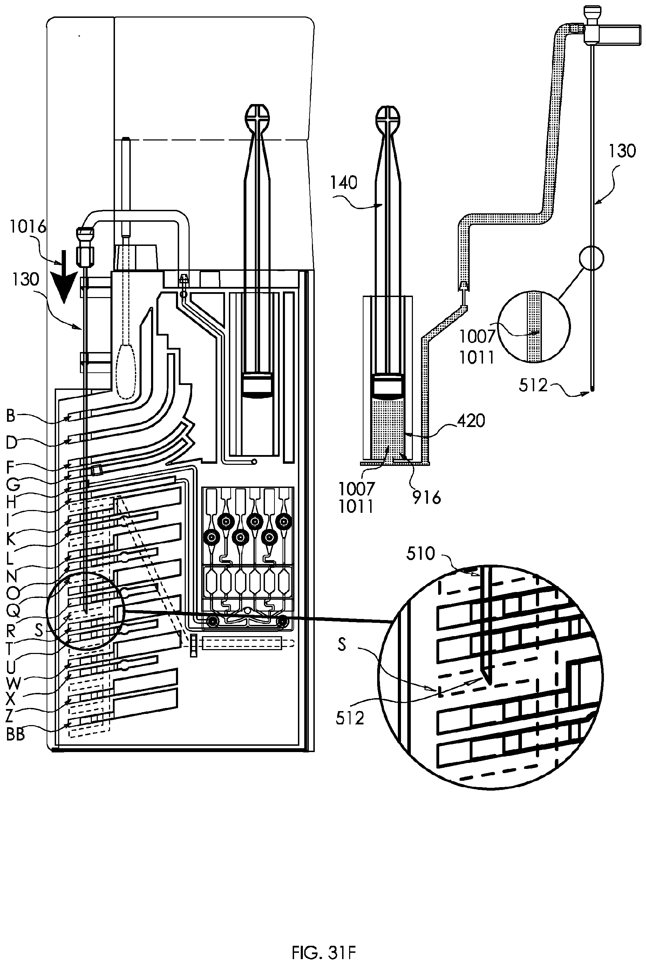

Figs. 31A, 31B, 31C, 31D, 31E and 31F are simplified illustrations of typical additional steps in the operation of the cartridge of Figs. 1A - 15F, wherein Fig. 31A shows an operational state identical to that of Fig. 30D, Figs. 31A - 31F showing the cartridge of Figs. 1A - 15F in a viewing direction opposite to that of Figs. 30A - 30D and with the second outer housing portion thereof removed and showing operative engagement with operational volumes Q, R and S;

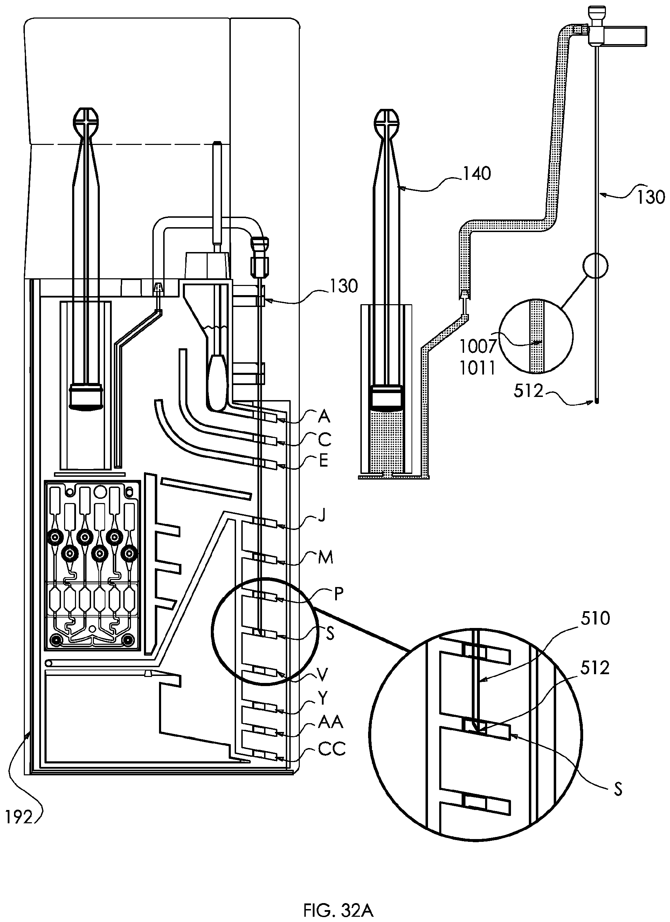

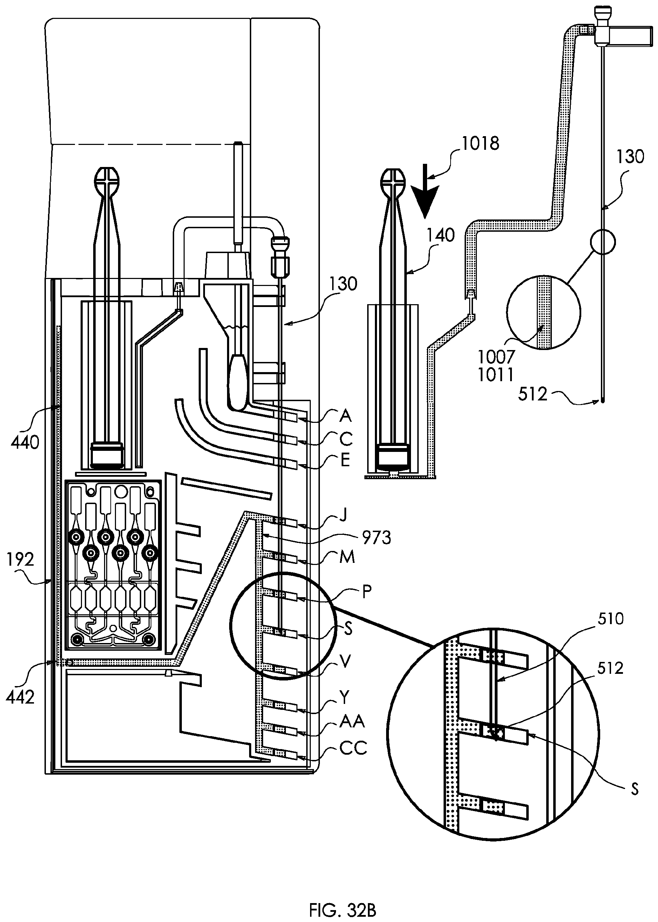

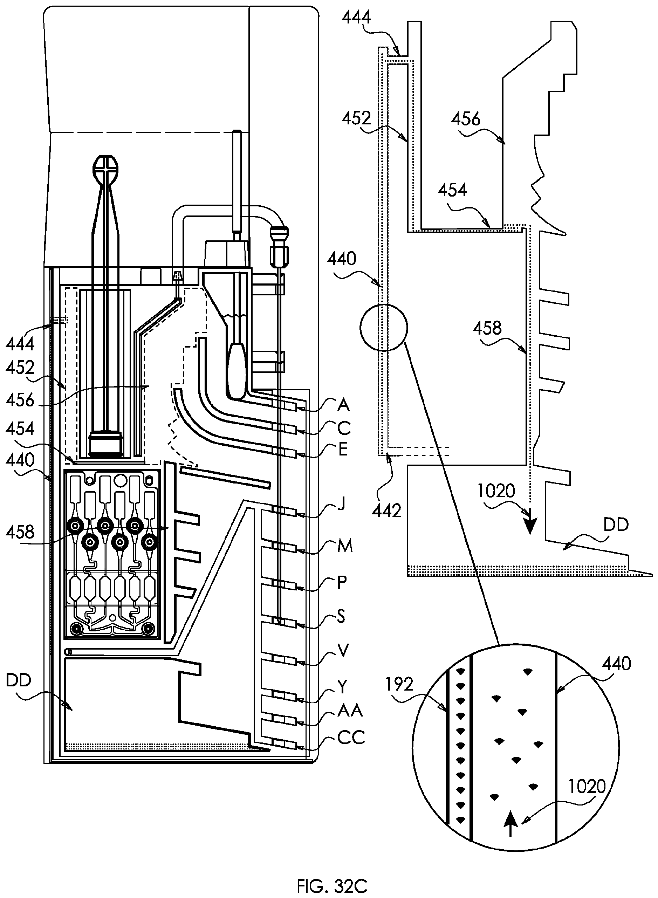

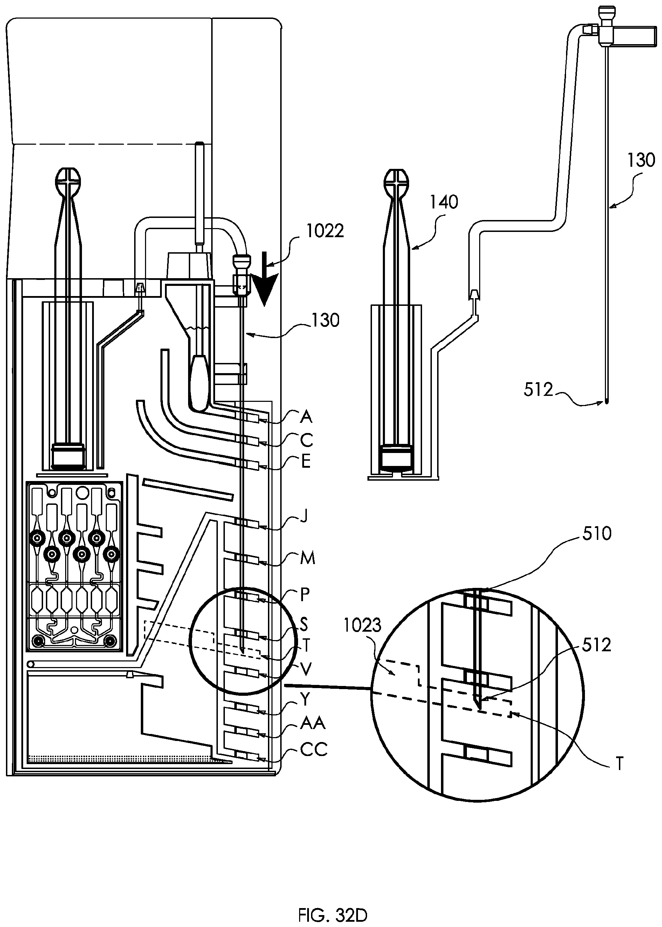

Figs. 32A, 32B, 32C and 32D are simplified illustrations of typical additional steps in the operation of the cartridge of Figs. 1A - 15F, wherein Fig. 32A shows an operational state identical to that of Fig. 31F, Figs. 32A - 32D showing the cartridge of Figs. 1A - 15F in a viewing direction opposite to that of Figs. 31A - 31F and with the first outer housing portion thereof removed and showing operative engagement with operational volumes S and T;

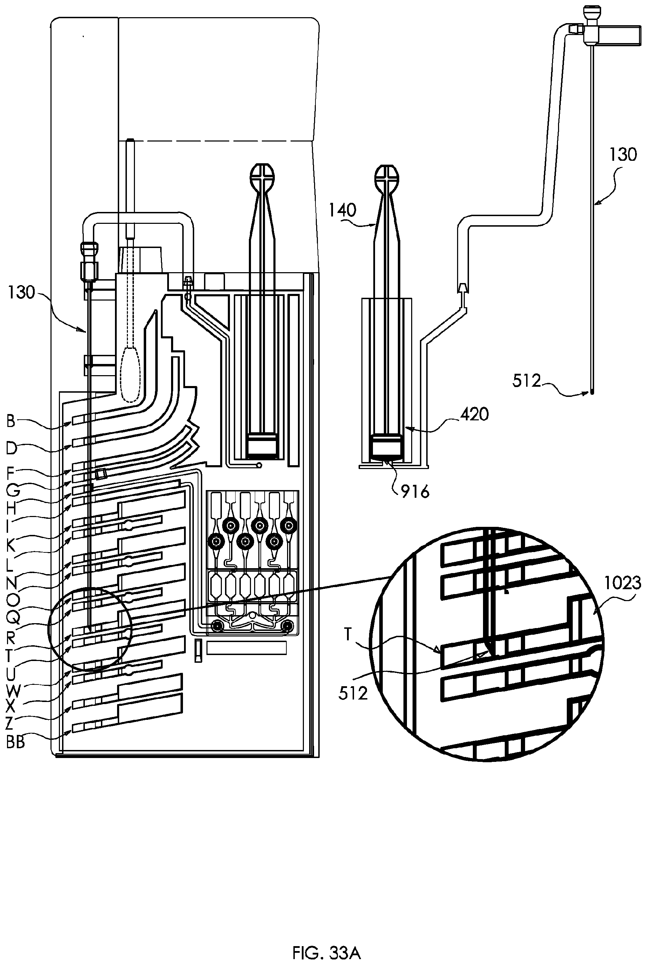

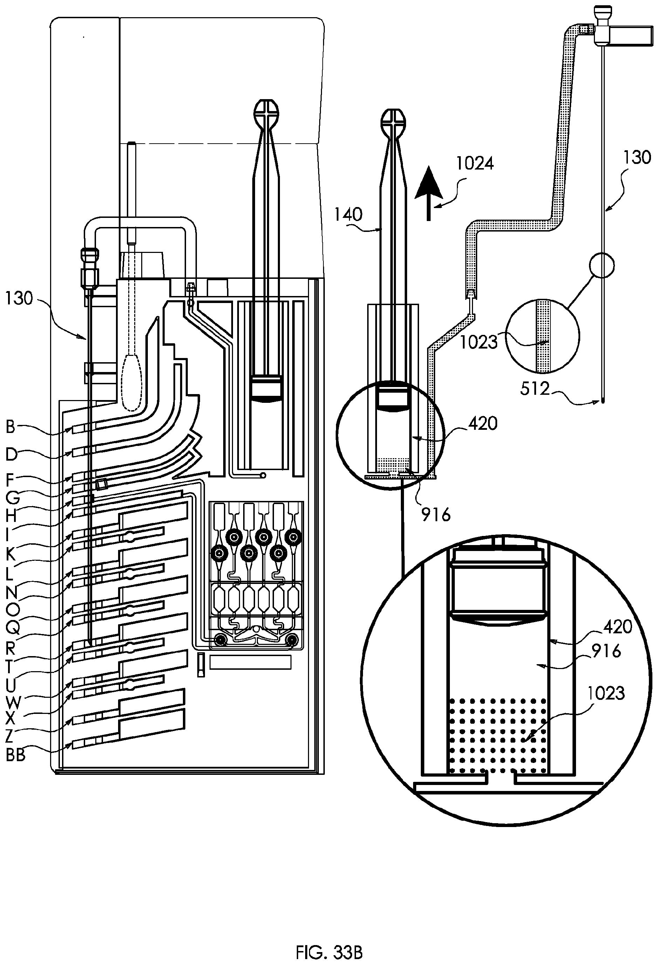

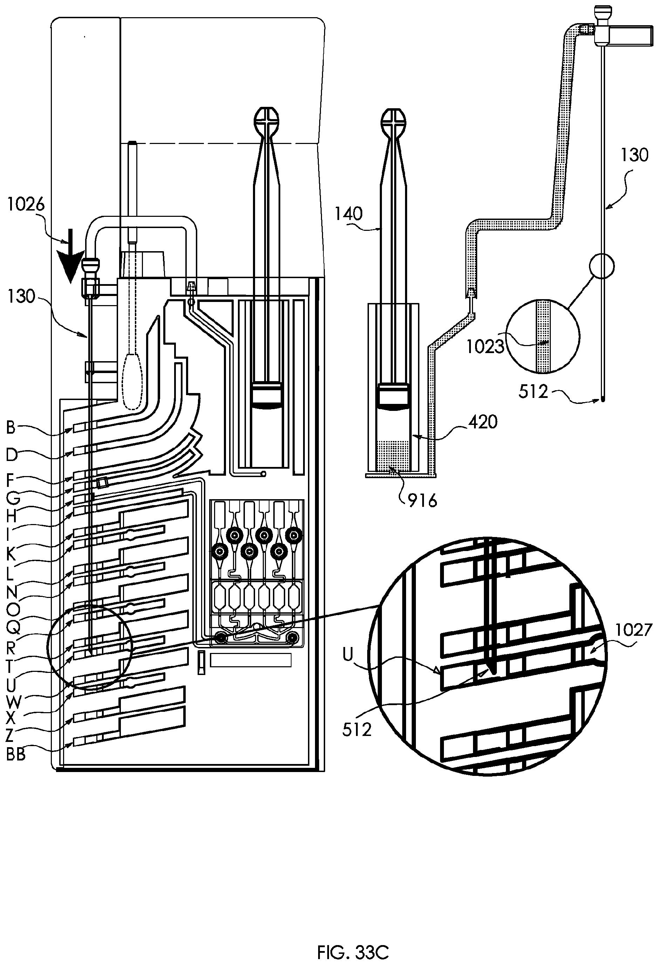

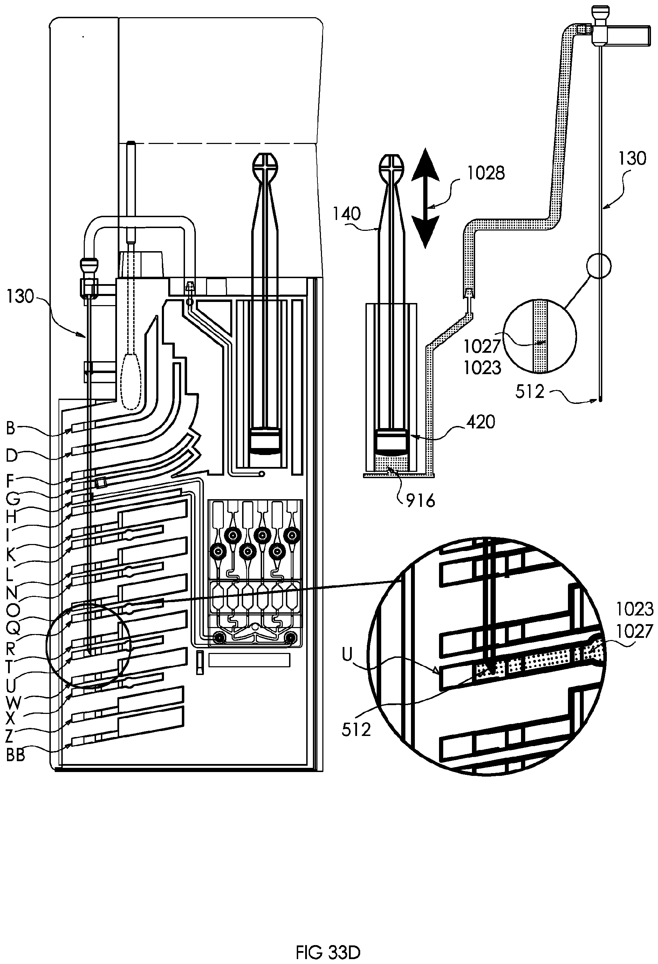

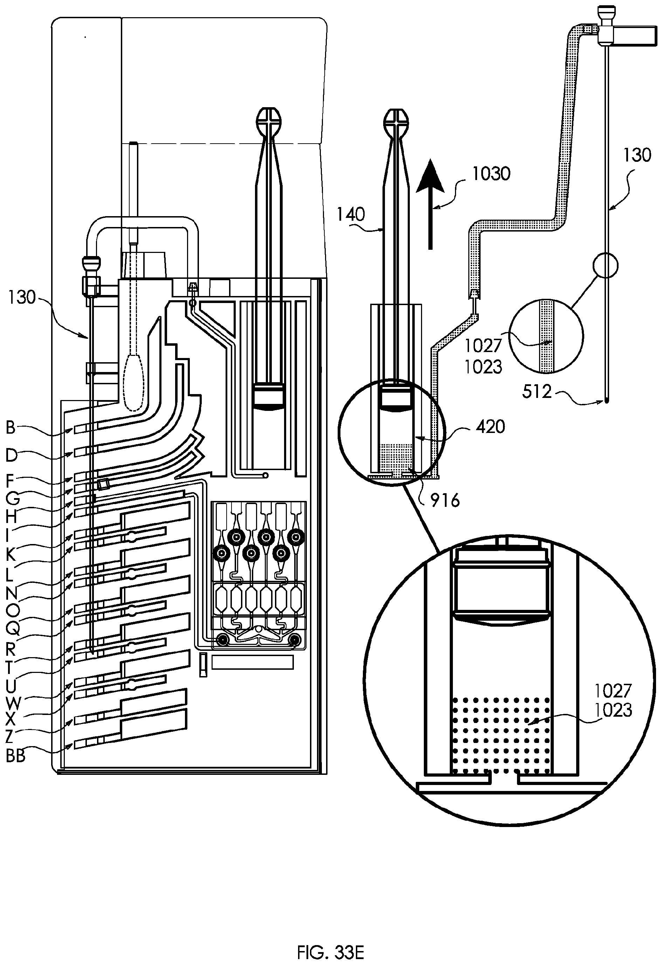

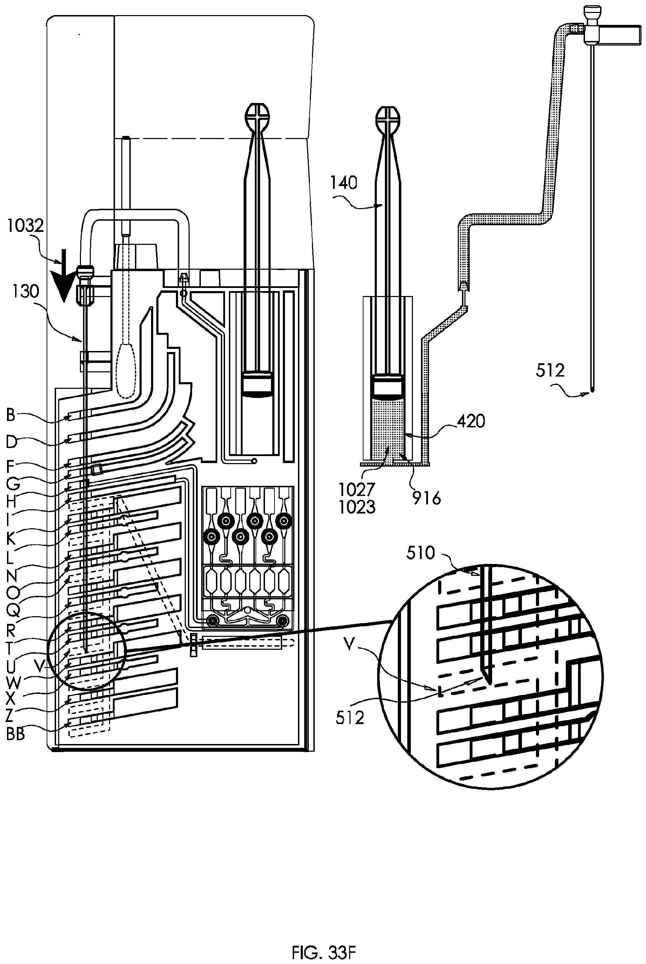

Figs. 33A, 33B, 33C, 33D, 33E and 33F are simplified illustrations of typical additional steps in the operation of the cartridge of Figs. 1A - 15F, wherein Fig. 33A shows an operational state identical to that of Fig. 32D, Figs. 33A - 33F showing the cartridge of Figs. 1A - 15F in a viewing direction opposite to that of Figs. 32A - 32D and with the second outer housing portion thereof removed and showing operative engagement with operational volumes T, U and V;

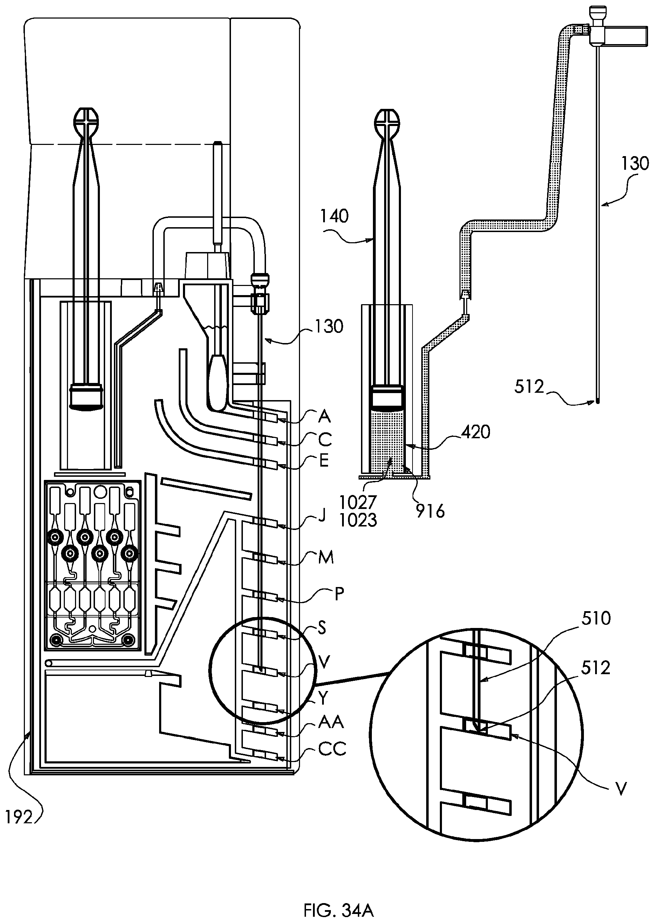

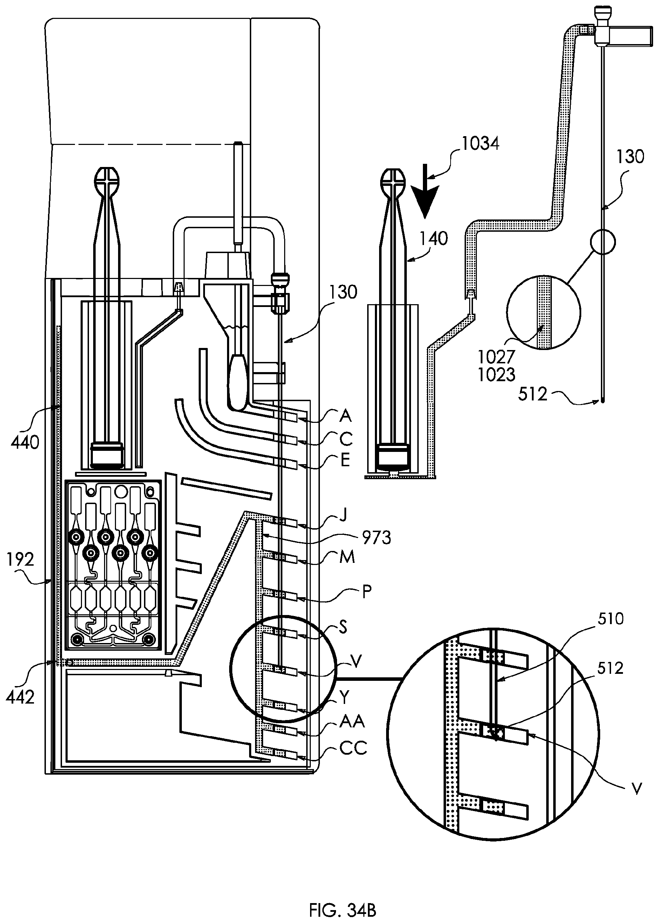

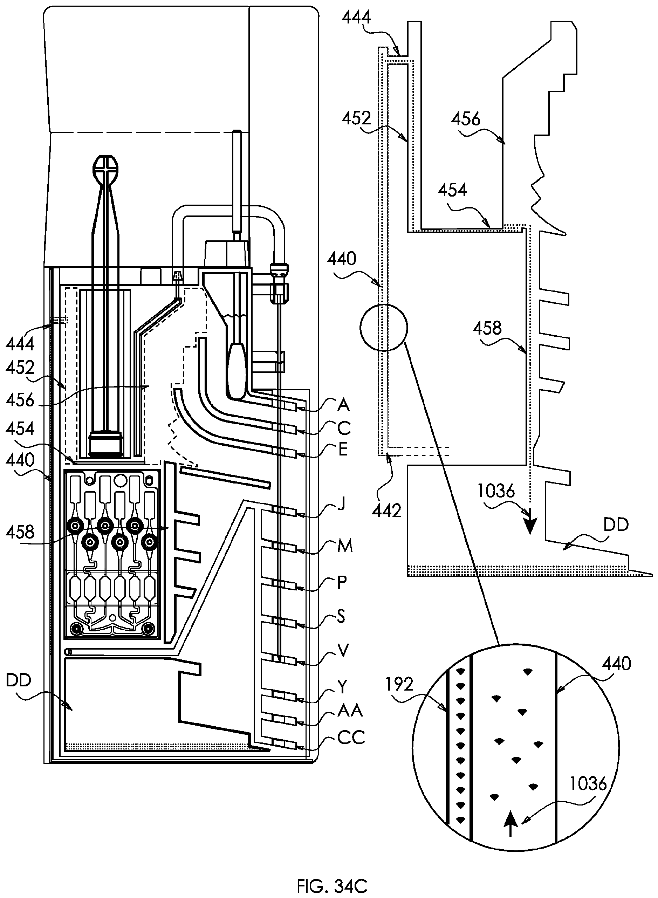

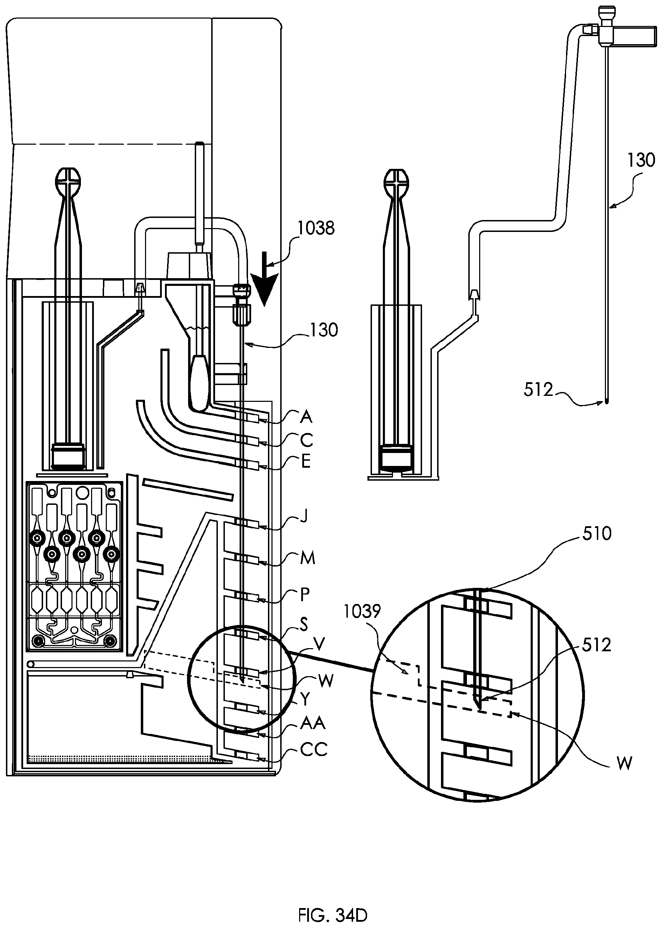

Figs. 34A, 34B, 34C and 34D are simplified illustrations of typical additional steps in the operation of the cartridge of Figs. 1A - 15F, wherein Fig. 34A shows an operational state identical to that of Fig. 33F, Figs. 34A - 34D showing the cartridge of Figs. 1A - 15F in a viewing direction opposite to that of Figs. 33A - 33F and with the first outer housing portion thereof removed and showing operative engagement with operational volumes V and W;

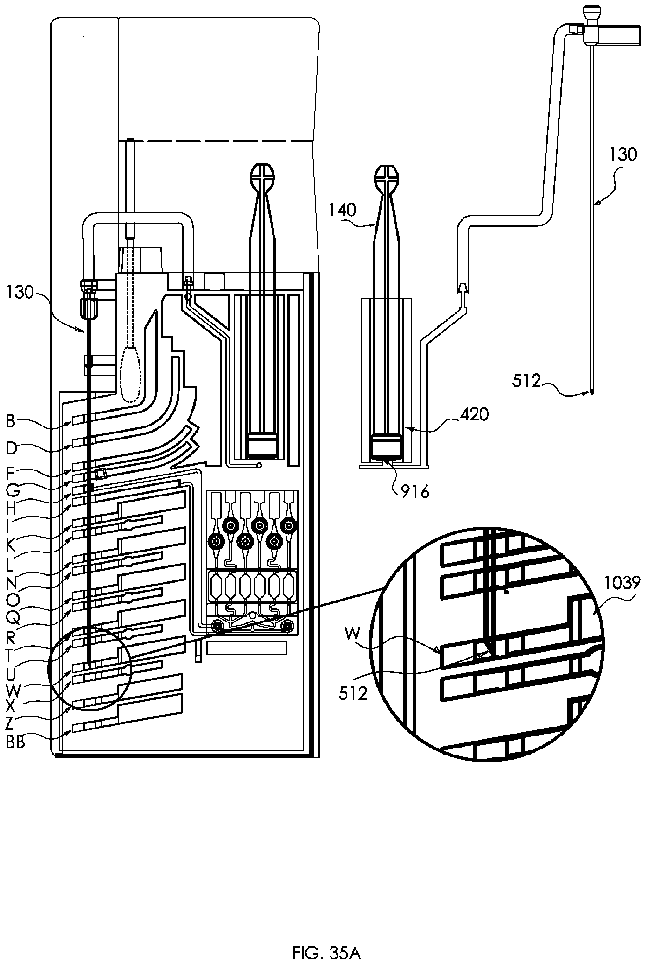

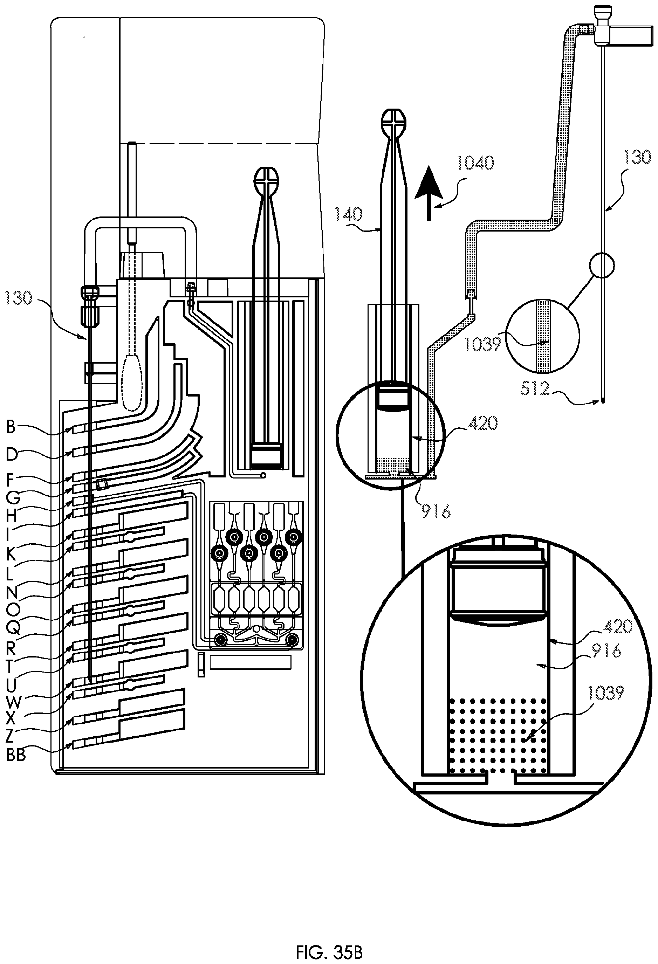

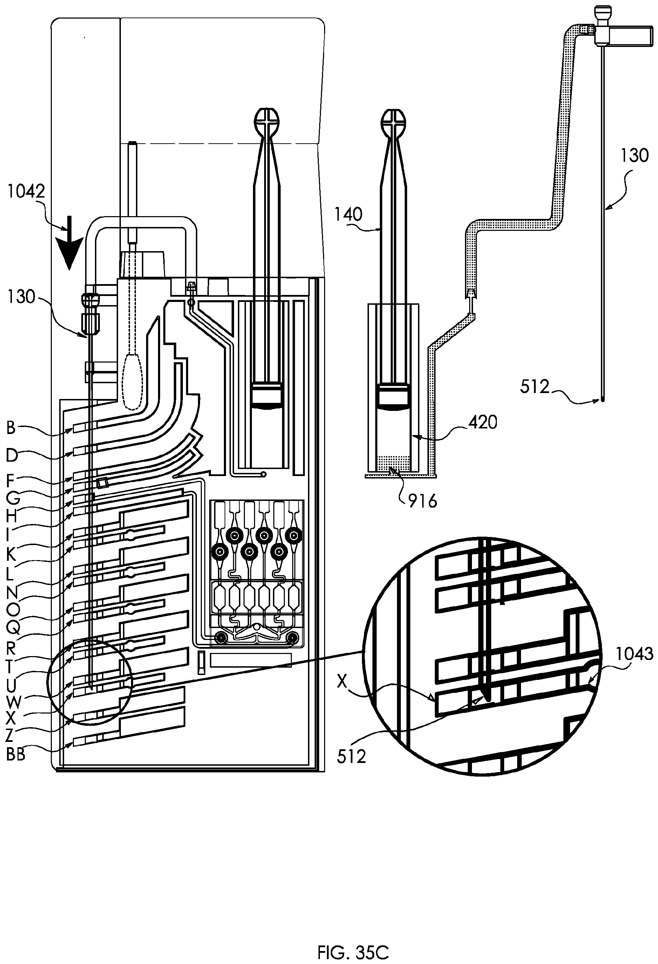

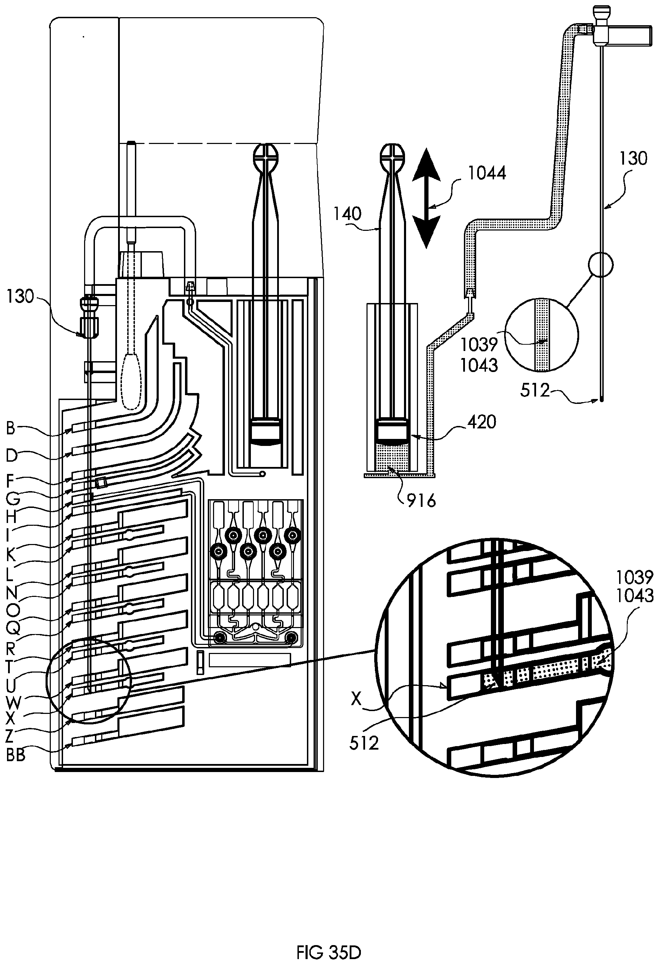

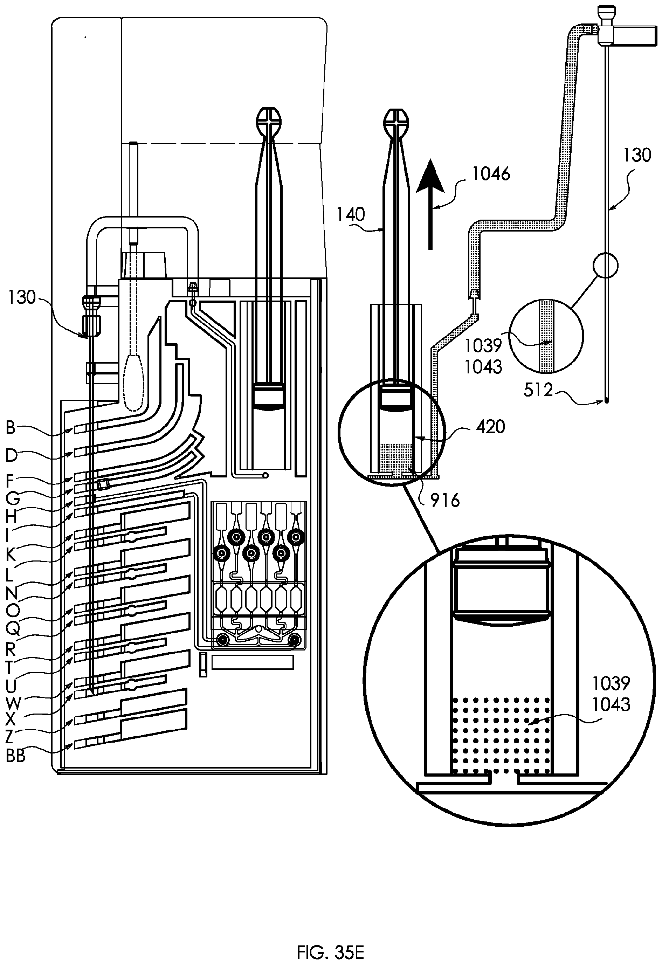

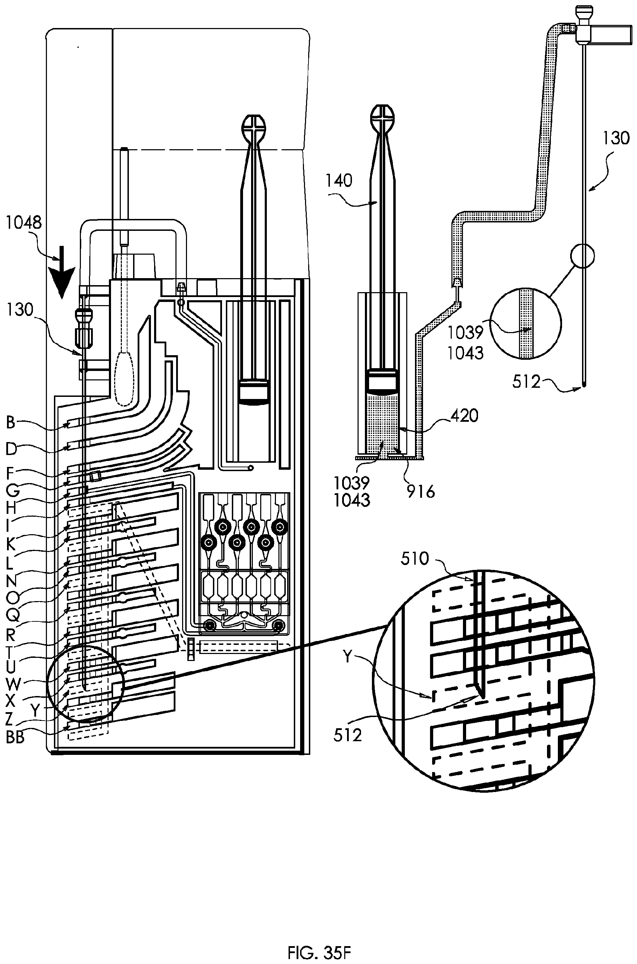

Figs. 35A, 35B, 35C, 35D, 35E and 35F are simplified illustrations of typical additional steps in the operation of the cartridge of Figs. 1A - 15F, wherein Fig. 35A shows an operational state identical to that of Fig. 34D, Figs. 35A - 35F showing the cartridge of Figs. 1A - 15F in a viewing direction opposite to that of Figs. 34A - 34D and with the second outer housing portion thereof removed and showing operative engagement with operational volumes W, X and Y;

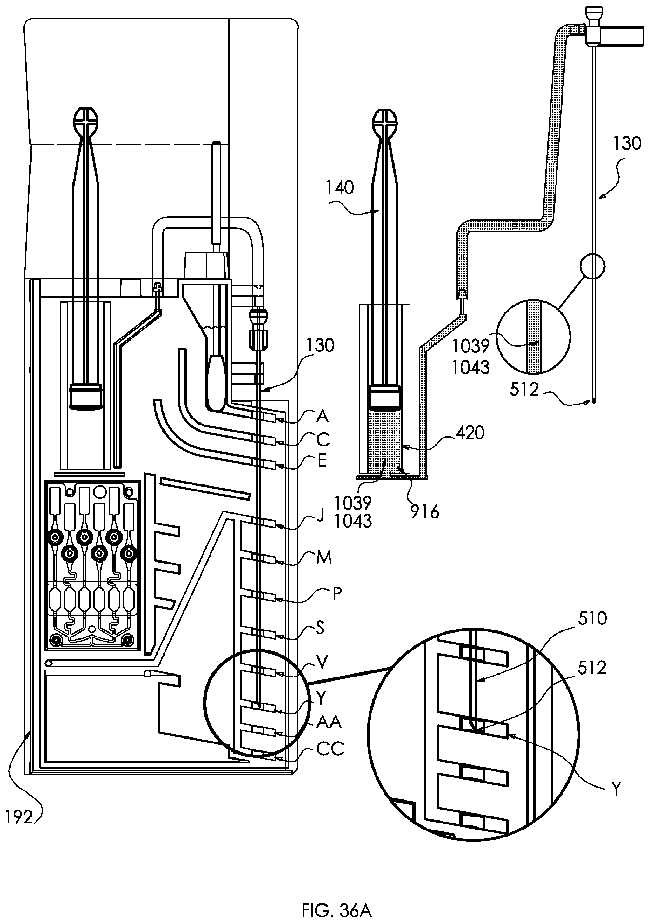

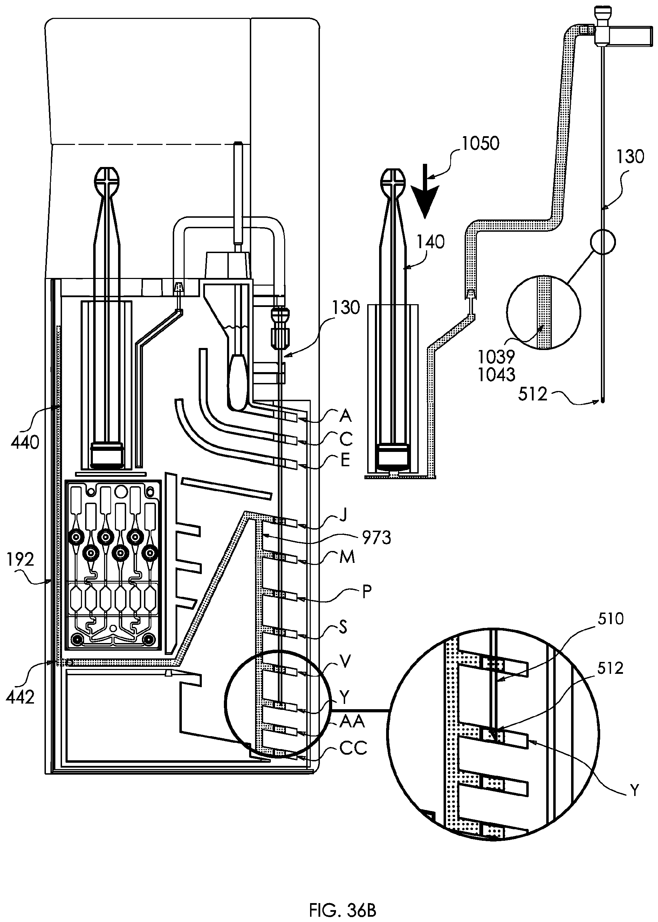

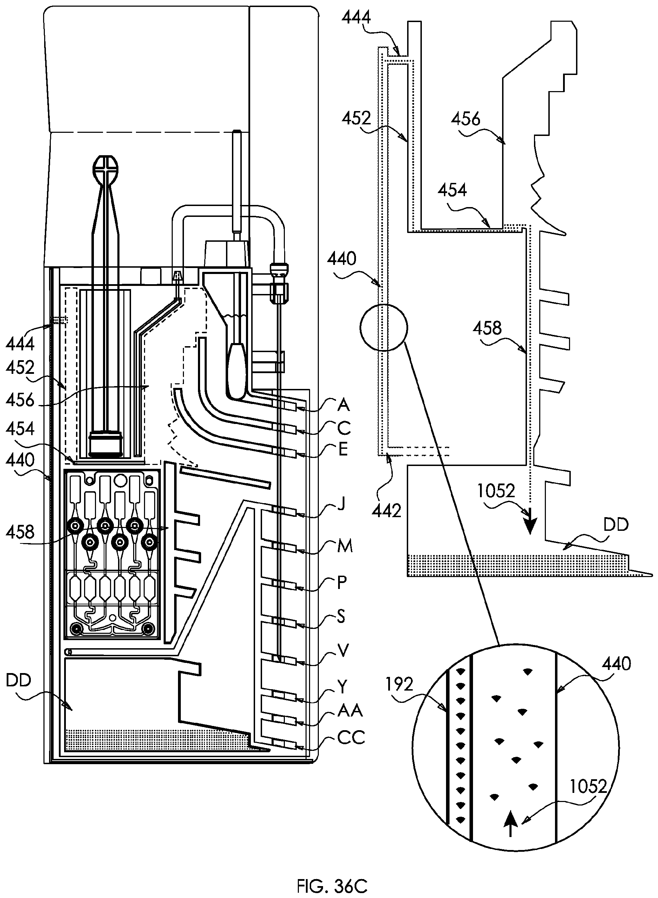

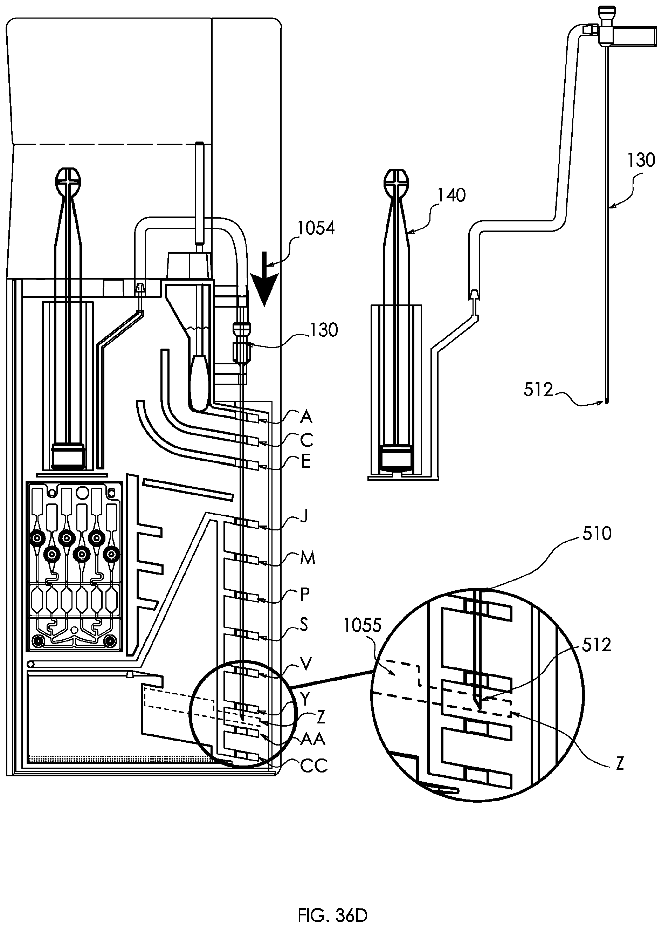

Figs. 36A, 36B, 36C and 36D are simplified illustrations of typical additional steps in the operation of the cartridge of Figs. 1A - 15F, wherein Fig. 36A shows an operational state identical to that of Fig. 35F, Figs. 36A - 36D showing the cartridge of Figs. 1A - 15F in a viewing direction opposite to that of Figs. 35A - 35F and with the first outer housing portion thereof removed and showing operative engagement with operational volumes Y and Z;

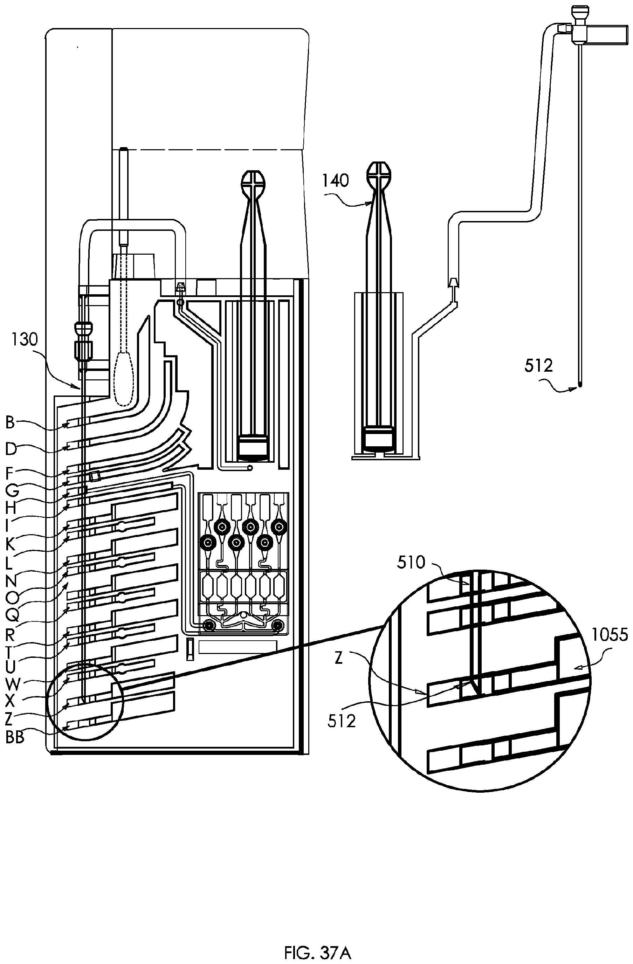

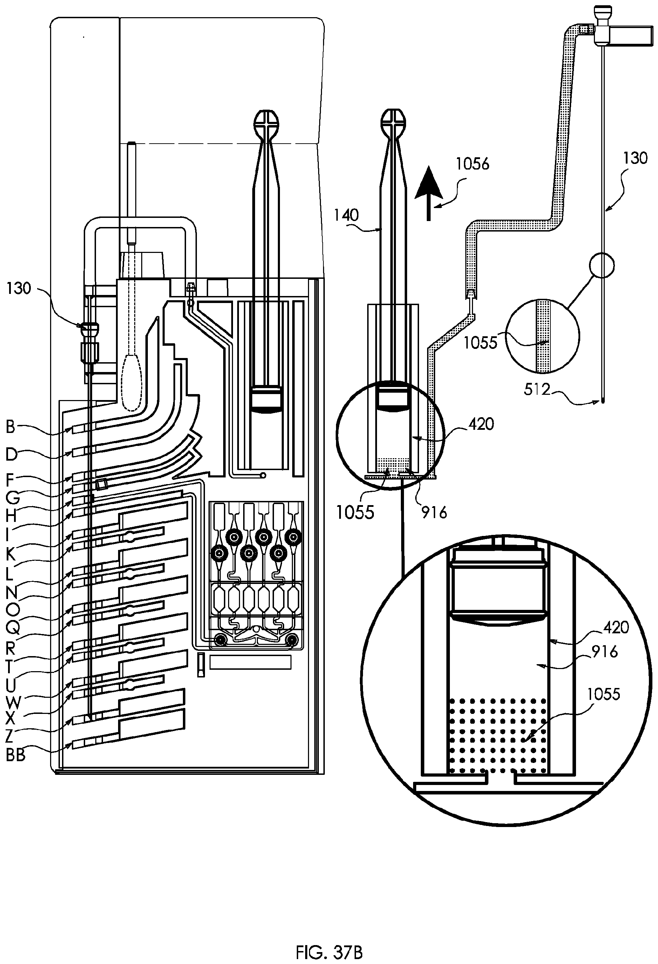

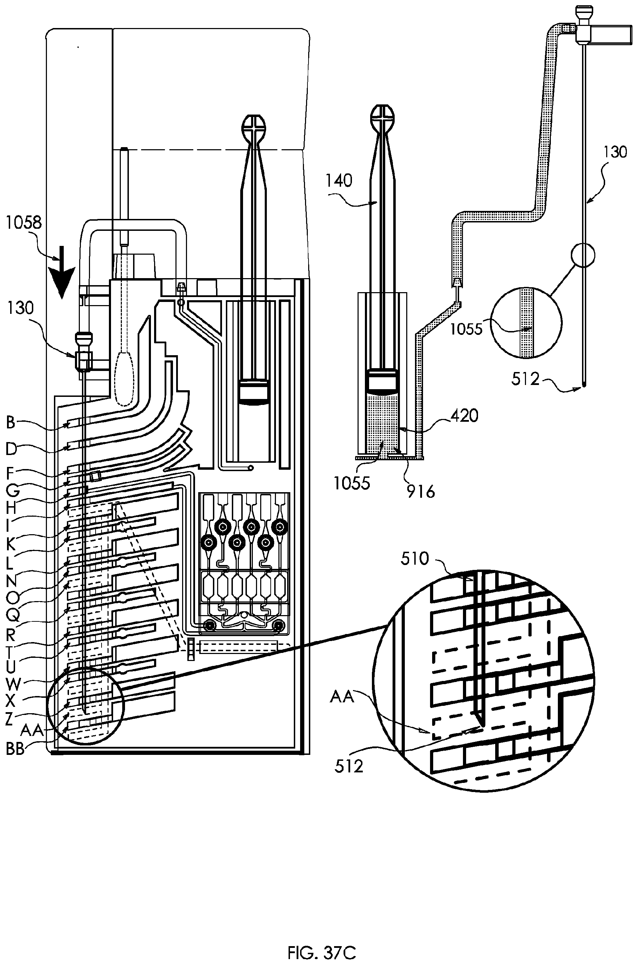

Figs. 37A, 37B and 37C are simplified illustrations of typical additional steps in the operation of the cartridge of Figs. 1A - 15F, wherein Fig. 37A shows an operational state identical to that of Fig. 36D, Figs. 37A - 37C showing the cartridge of Figs. 1A - 15F in a viewing direction opposite to that of Figs. 36A - 36D and with the second outer housing portion thereof removed and showing operative engagement with operational volumes Z and AA;

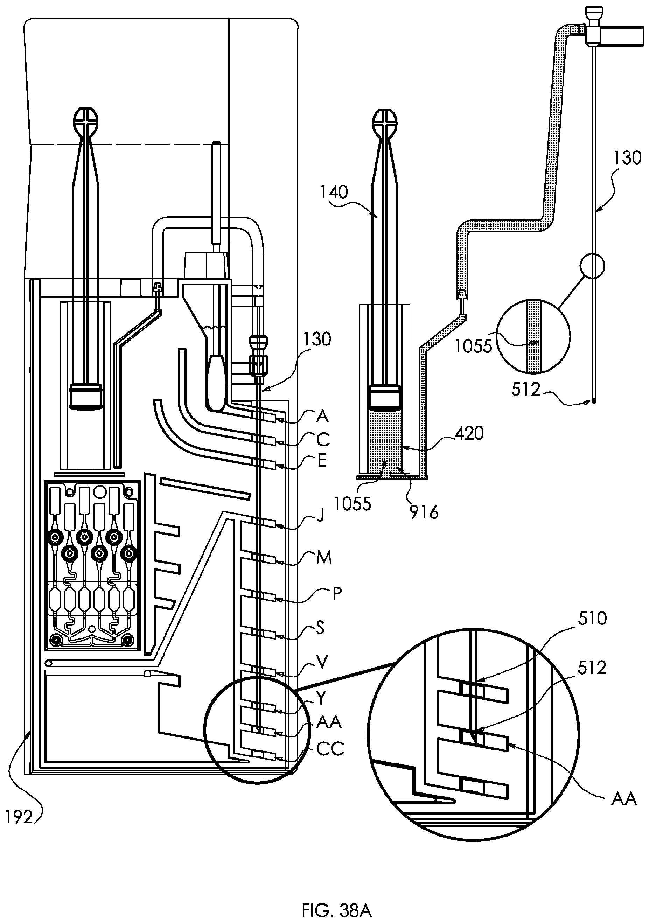

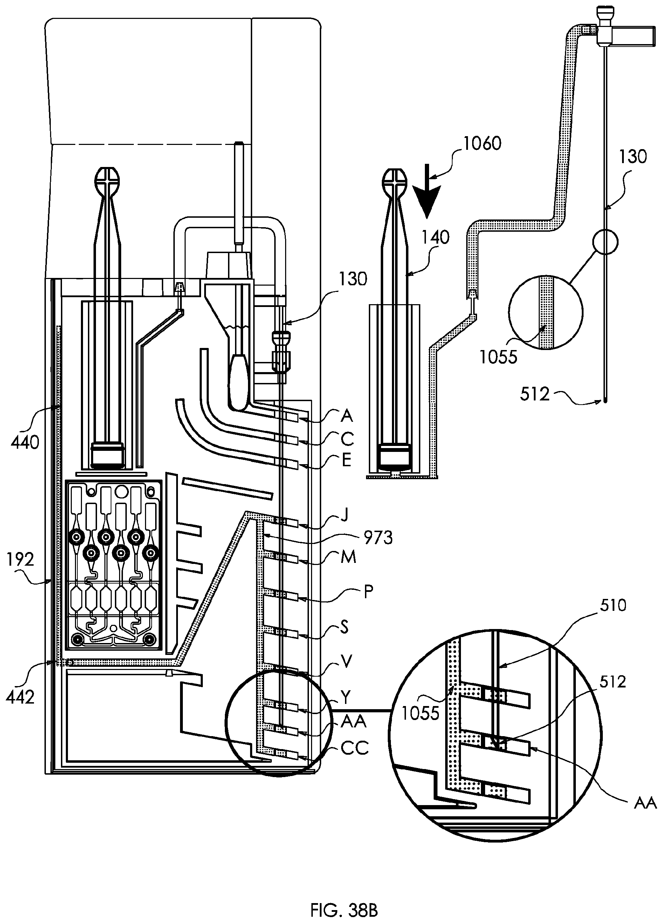

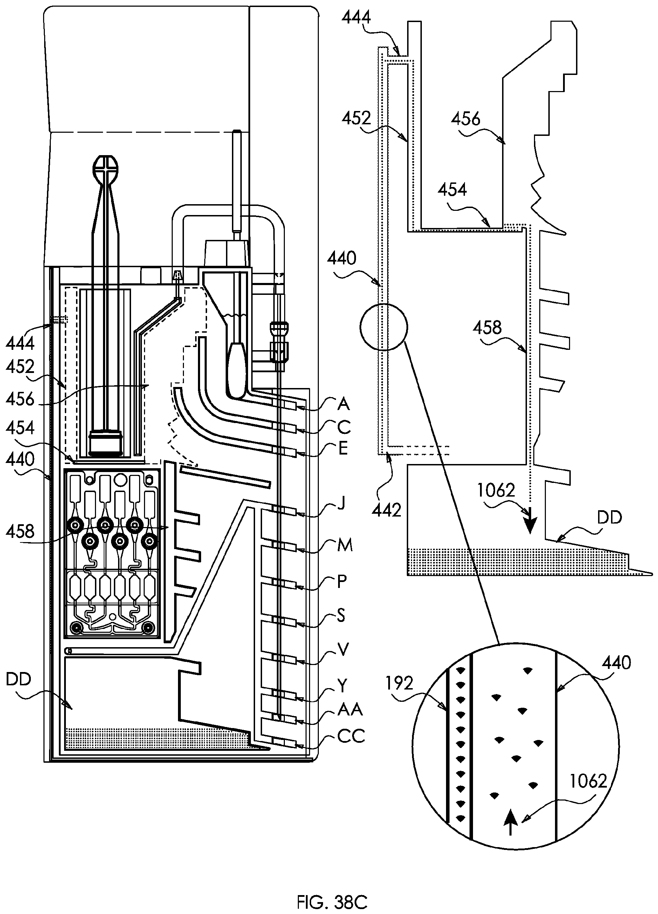

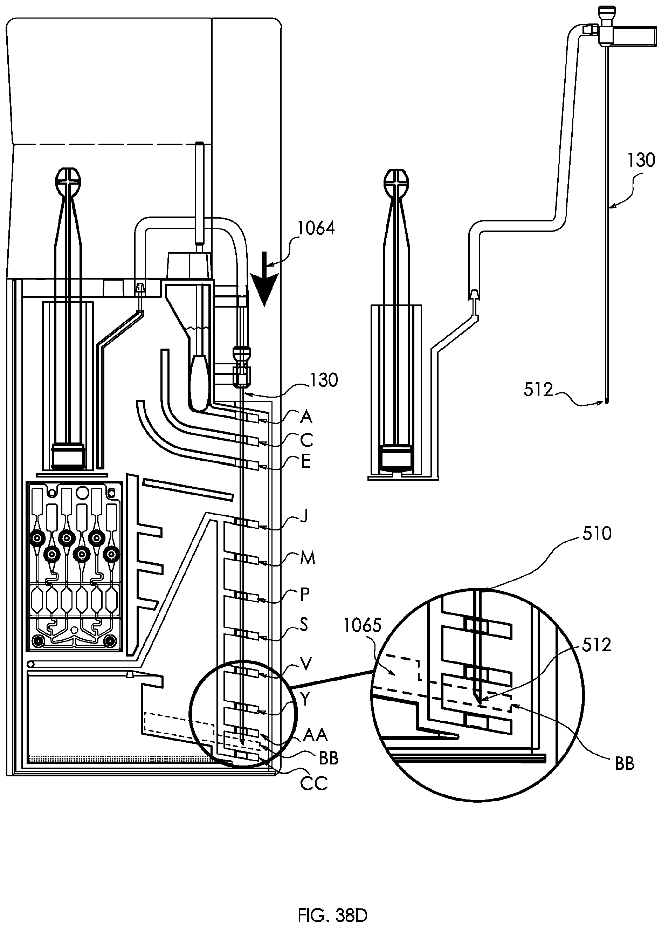

Figs. 38A, 38B, 38C and 38D are simplified illustrations of typical additional steps in the operation of the cartridge of Figs. 1A - 15F, wherein Fig. 38A shows an operational state identical to that of Fig. 37C, Figs. 38A - 38D showing the cartridge of Figs. 1A - 15F in a viewing direction opposite to that of Figs. 37A - 37C and with the first outer housing portion thereof removed and showing operative engagement with operational volumes AA and BB;

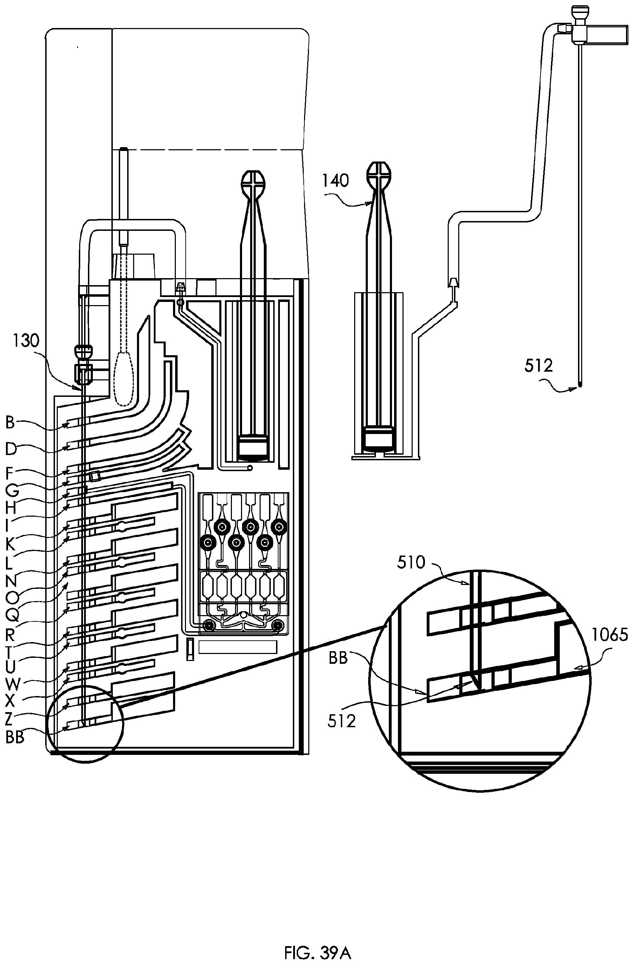

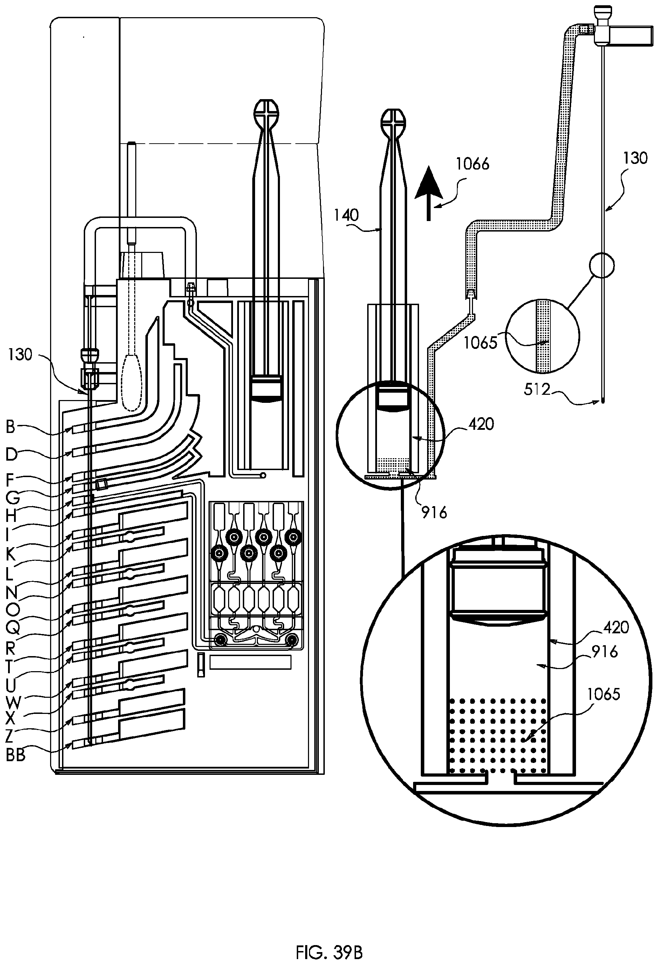

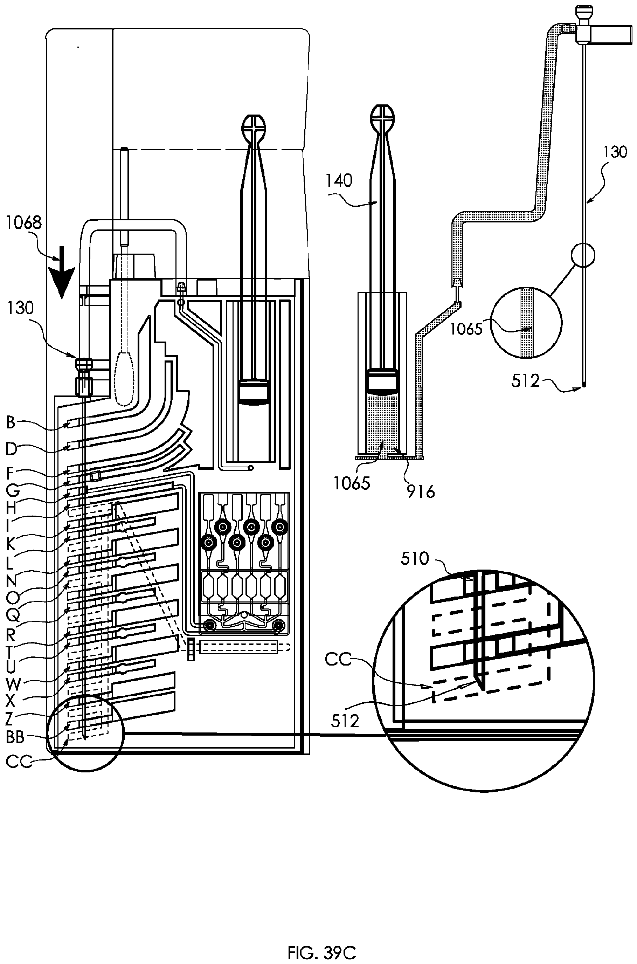

Figs. 39A, 39B and 39C are simplified illustrations of typical additional steps in the operation of the cartridge of Figs. 1A - 15F, wherein Fig. 39A shows an operational state identical to that of Fig. 38D, Figs. 39A - 39C showing the cartridge of Figs. 1A - 15F in a viewing direction opposite to that of Figs. 38A - 38D and with the second outer housing portion thereof removed and showing operative engagement with operational volumes BB and CC; and

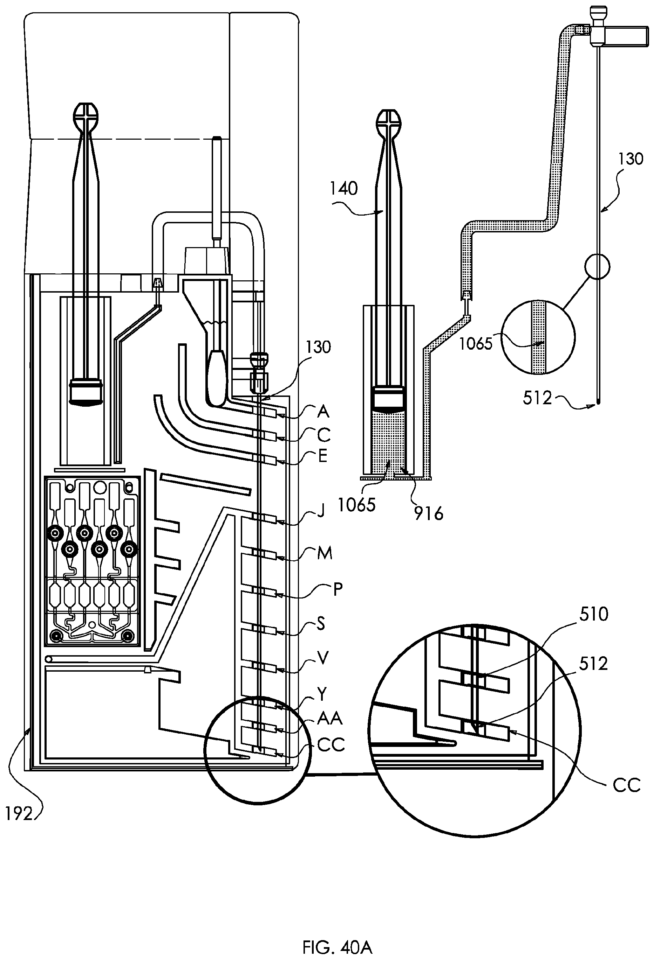

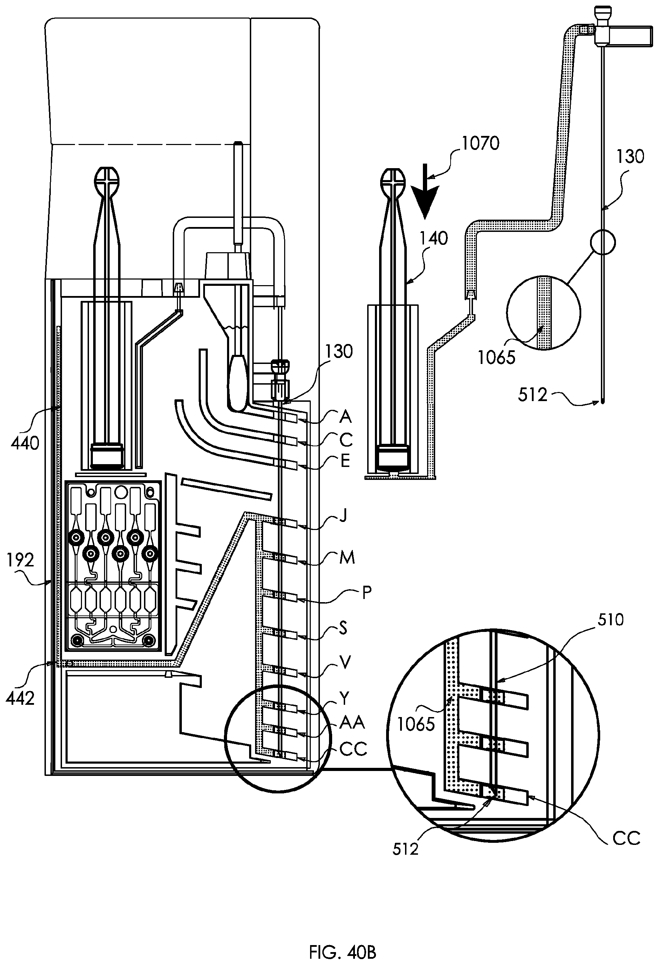

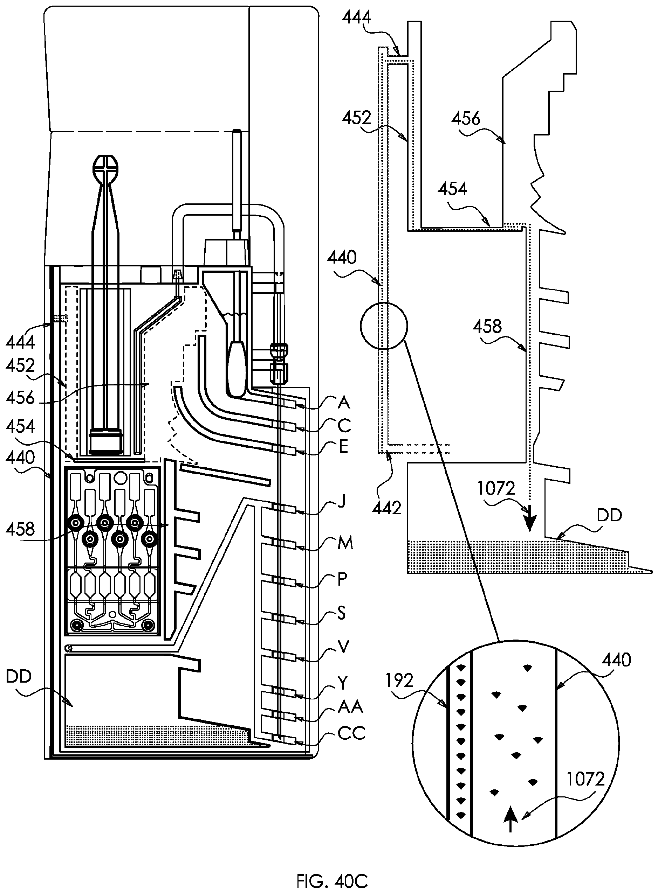

Figs. 40A, 40B and 40C are simplified illustrations of typical additional steps in the operation of the cartridge of Figs. 1A - 15F, wherein Fig. 40A shows an operational state identical to that of Fig. 39C, Figs. 40A - 40C showing the cartridge of Figs. 1A - 15F in a viewing direction opposite to that of Figs. 39A - 39C and with the first outer housing portion thereof removed and showing operative engagement with operational volume CC.

### DETAILED DESCRIPTION OF A PREFERRED EMBODIMENT

Reference is now made to Figs. 1A, 1B and 1C, which are simplified illustrations of a cartridge 100 for use in in-vitro diagnostics, constructed and operative in accordance with a preferred embodiment of the present invention.

As seen in Figs. 1A - 1C, cartridge 100 comprises first and second outer housing portions 110 and 112, respectively, which are described in detail hereinbelow with reference to Figs. 2A - 2D and 3A - 3D, respectively. Located between first and second outer housing portions 110 and 112 is a central portion 120, which is described hereinbelow in detail with respect to Figs. 4A - 4G.

A needle assembly 130 cooperates with central portion 120 and is described hereinbelow in detail with respect to Figs. 5A - 5D. A cover element 132 covers the needle assembly 130 and is described hereinbelow in detail with respect to Figs. 13A - 13E. A piston assembly 140 is located within part of central portion 120 and is described hereinbelow in detail with respect to Figs. 6A - 6D. A septum portion 150 is overmolded onto central portion 120 and is described hereinbelow in detail with respect to Figs. 7A - 7J.

A sample insertion subassembly 160 is mounted onto central portion 120 and is described hereinbelow in detail with respect to Figs. 9A - 12D. A cover element 170 for a fluid solution insertion wall 172 defined on septum portion 150 is removably mounted onto first and second outer housing portions 110 and 112 and is described hereinbelow in detail with respect to Figs. 14A - 14E.

A safety lock element 180 is mounted onto first outer housing portion 110 and engages central portion 120 and is described hereinbelow in detail with respect to Figs.15A - 15F. A microfluidic Polymerase Chain Reaction (PCR) array 190 is mounted within central portion 120 and is described in detail, inter alia, in U.S. Patent Publication Nos. 2012/0082599 and 2016/0167047, and is commercially available from Thinxxs Microtechnology AG of Zweibrucken, Germany. A sensor array 192 is mounted onto the central portion 120 and is described in detail, inter alia, in U.S. Patent Nos. 5,605,662; 6,238,624; 6,303,082; 6,403,367; 6,524,517; 6,960,298; 7,101,661; 7,601,493; 8,288,155, and is commercially available from Conductive Technologies, Inc. of York, Pennsylvania, USA. A flexible tube 194 interconnects needle assembly 130 with central portion 120.

Reference is now made to Figs. 2A - 2D, which are simplified illustrations of respective exterior and interior surfaces of first outer housing portion 110 of the cartridge 100 of Figs. 1A - 1C. As seen in Figs. 2A - 2D, the first outer housing portion 110 is a generally planar element, which is preferably injection molded of polypropylene. First outer housing portion 110 preferably includes a generally planar outer surface 202, which includes a main rectangular portion 204 and a narrow rectangular portion 206 extending upwardly from main rectangular portion 204. A preferably rectangular aperture 210 is formed in main rectangular portion 204 and includes a side protrusion 212. Aperture 210 is configured to receive and removably lock in place safety lock element 180 (Figs. 15A - 15F).

Outer surface 202 is generally surrounded by edge surfaces 220, which extend generally perpendicularly thereto. Edge surfaces 220 preferably define a first top edge gap 222 adjacent narrow rectangular portion 206 and a second top edge gap 224, narrower than and spaced from top edge gap 222.

First outer housing portion 110 preferably includes a generally planar inner surface 232, which includes a main rectangular portion 234 and a narrow rectangular portion 236 extending upwardly from main rectangular portion 234. A sealing element 238 is formed on main rectangular portion 234 and may be also visible on generally planar outer surface 202. Sealing element 238 is preferably a flexible element configured to be inwardly displaced as described further hereinbelow.

A pair of closely spaced longitudinal protrusions 240 extend outwardly from generally planar inner surface 232 and extend generally along the length of narrow rectangular portion 236 and into part of main rectangular portion 234. Longitudinal protrusions 240 are joined by an end portion 242.

A magnet insertion bore 244 extends from a magnet insertion aperture 246 formed in main rectangular portion 204.

As seen in Figs. 2B and 2D, contoured sealing elements 250 are preferably formed on main rectangular portion 234 of planar inner surface 232, providing for laser welding of first outer housing portion 110 to central portion 120. Contoured sealing elements 250 preferably conform to sides of operational volumes defined by central portion 120.

Reference is now made to Figs. 3A - 3D, which are simplified illustrations of respective exterior and interior surfaces of second outer housing portion 112 of cartridge 100 of Figs. 1A - 1C. As seen in Figs. 3A - 3D, the second outer housing portion 112 is a generally planar element, which is preferably injection molded of polypropylene. Second outer housing portion 112 preferably includes a generally planar outer surface 302, which includes a main rectangular portion 304 and a narrow rectangular portion 306 extending upwardly from main rectangular portion 304. A preferably rectangular aperture 310 is formed in main rectangular portion 304. Aperture 310 is configured to provide access to microfluidic PCR array 190.

Outer surface 302 is generally surrounded by edge surfaces 320, which extend generally perpendicularly thereto. Edge surfaces 320 preferably define a first top edge gap 322 adjacent narrow rectangular portion 306 and a second top edge gap 324, narrower than and spaced from top edge gap 322. Edge surfaces 320 also define a retaining slot 326, which is located on a side edge of narrow rectangular portion 306.

Second outer housing portion 112 preferably includes a generally planar inner surface 332, which includes a main rectangular portion 334 and a narrow rectangular portion 336 extending upwardly from main rectangular portion 334. A slot 340 is positioned in alignment with the pair of closely spaced longitudinal protrusions 240 of the first outer housing portion 110 and extends generally along the entire length of narrow rectangular portion 336 and into part of main rectangular portion 334. Slot 340 extends onto a top edge surface 342 and ends in an aperture 344 formed in top edge surface 342.

As seen in Figs. 3B and 3D, contoured sealing elements 350 are preferably formed on main rectangular portion 334 of planar inner surface 332, providing for laser welding of second outer housing portion 112 to central portion 120. Contoured sealing elements 350 preferably conform to sides of operational volumes defined by central portion 120.

Reference is now made to Figs. 4A - 4G, which are simplified illustrations of central portion 120 of cartridge 100 of Figs. 1A - 1C which is overmolded with septum portion 150 (Figs. 7A - 7F).

Turning to Figs. 4A - 4G, it is seen that central portion 120 includes multiple internal walls which partially define a multiplicity of operational volumes, and are designated by letters: A, B, C, D, E, F, G, H, I, J, K, L, M, N, O, P, Q, R, S, T, U, V, W, X, Y, Z, AA, BB, CC and DD. Twelve of the operational volumes are seen in Fig. 4C and eighteen of the operational volumes are seen in Fig. 4D. An opening of each of the operational volumes is seen in Fig. 4E and is labeled with the same letter as that of the operational volume. As will be described hereinbelow, each of the openings is sealed by septum portion 150 which is overmolded thereover and which allows injection of solutions, such as reagents, buffers, reconstitution fluids or dilution fluids, into appropriate operational volumes. It is appreciated that one or more of the multiplicity of operational volumes may contain a solution or a substance, such as a reagent or buffer to be reconstituted or diluted, prior to sealing of central portion 120 by septum portion 150.

A central slot 402 extends vertically in the sense of Figs. 4A - 4F and communicates with each of the operational volumes A - CC. Operational volumes A - CC are each sealed from each other by a septum defined by the septum portion 150, as will be described hereinbelow. The septa thus defined by the septum portion 150 are sealingly penetratable by needle assembly 130 (Figs. 5A - 5D) which is selectably positionable in central slot 402 to selectably communicate with each of the operational volumes A - CC. It is noted that central slot 402 is preferably located at or near the lowest point of each of the operational volumes A - CC. As noted above, solutions, such as reagents, buffers, reconstitution fluids or dilution fluids, are injected into appropriate operational volumes through septum portion 150, preferably by insertion of a needle into insertion wall 172 of septum portion 150 along an axis which is angled with respect to an axis along which needle assembly 130 is displaced.

Additionally or alternatively, a dried substance, such as a reagent or buffer, may be located in one or more of operational volumes A - CC by utilizing a carrier element inserted into a cavity therein, as described in detail, inter alia, in U.S. Patent Publication No. 2016/0167047. In this embodiment, the cavity is preferably formed in first outer housing portion 110 adjacent contoured sealing elements 250 or in second outer housing portion 112 adjacent contoured sealing elements 350.

A sample insertion aperture 410 communicates with sample insertion subassembly 160 and with operational volume A. A male connection portion 412 is connected to one end of flexible tube 194, the other end of which is connected to needle assembly 130. Male connection portion 412 communicates with an internal passageway 414 in central portion 120 which communicates with the interior volume of a cylinder 420, which sealingly accommodates piston assembly 140 via a piston aperture 422 formed in a top surface 423 and defines a operational volume. A connection boss 430 is provided for mounting of sample insertion subassembly 160 onto central portion 120. Needle guides 431 are mounted in alignment with central slot 402.

Operational volume G includes at least one miniature magnet 432 (Figs. 23A - 23D).

Operational volume H defines an internal passageway to an inlet of microfluidic PCR array 190 via a valve 433 (Figs. 24B - 24D) forming part of the microfluidic PCR array 190. PCR reaction products, preferably amplified DNA, are supplied via an outlet of microfluidic PCR array 190, via a valve 434 (Figs. 24B - 24D) forming part of microfluidic PCR array 190, to operational volume I.

Central portion 120 is formed at a side edge thereof with a recess 440 for fixedly receiving sensor array 192. PCR reaction products, preferably amplified DNA, are supplied to sensor array 192 from operational volume J via a supply aperture 442 and removed therefrom via a removal aperture 444 to operational volume DD, which is a waste collection operational volume.

Waste conduits 452, 454, 456 and 458 are provided in central portion 120 and communicate with operational volume DD.

Reference is now made to Figs. 5A, 5B, 5C and 5D, which are simplified illustrations of needle assembly 130, forming part of cartridge 100 of Figs. 1A & 1B. As seen in Figs. 5A - 5D, needle assembly 130 preferably comprises a hollow stainless steel tube 510, preferably having an outer diameter of 0.81 mm and an inner diameter of 0.41 mm and a pointed end 512. At an end 514 of tube 510, opposite to pointed end 512, there is provided a needle head connector 516, which may be overmolded onto end 514 or attached thereto by means of an adhesive.

Needle head connector 516 preferably includes a central portion 518 which surrounds end 514 of tube 510 and from which extends to a first track riding portion 520. A preferably barbed fluid connection portion 522 communicates with the interior of tube 510 via end 514 thereof and a channel 524 formed in central portion 518.

Reference is now made to Figs. 6A, 6B, 6C and 6D, which are simplified illustrations of piston assembly 140, forming part of cartridge 100 of Figs. 1A & 1B. As seen in Figs. 6A - 6D, piston assembly 140 comprises a generally elongate unitary plastic shaft 540 having a base portion 542, which is configured to retain thereon a sealing cap 544, an intermediate portion 546, having a generally uniform cruciform cross section, a tapered portion 548 and an engagement portion 550.

Intermediate portion 546, tapered portion 548 and engagement portion 550 preferably all include mutually parallel vanes 556, which are mutually separated from each other by 90 degrees. Engagement portion 550 is preferably defined as a generally elliptical protrusion and includes a transverse wall portion 558.

Sealing cap 544 preferably includes a pair of sealing ring portions 560 and 562 which extend along respective circumferential edges of a main cylindrical portion 564 and extend radially outwardly therefrom.

Reference is now made to Figs. 7A - 7J, which are simplified illustrations of septum portion 150, forming part of cartridge 100 of Figs. 1A - 1C, and to Figs. 8A - 8E, which show septum portion 150 overmolded over central portion 120. As seen in Figs. 1C, 7A - 7J and 8A - 8E, septum portion 150 includes fluid solution insertion wall 172, which may be penetrated by one or more hypodermic needles at one or more locations thereon which communicate with operational volumes A - CC.

It is appreciated that septum portion 150 also defines a multiplicity of operational volume wall portions 570 which each extend in a plane nearly perpendicular to that of fluid solution insertion wall 172 and tilted preferably by 10 degrees from the perpendicular. When the septum portion 150 is overmolded over the central portion 120, as seen particularly in Figs. 8A - 8E, the operational volume wall portions 570 sealingly engage the walls of the central portion 120, which each define the top and bottom walls of each one of operational volumes A - CC and are correspondingly also labeled A - CC.

The septum portion 150 also defines a multiplicity of needle penetrable septum seal portions 580, all aligned along central slot 402, which are selectably penetrable by suitable positioning of needle assembly 130 along central slot 402. Each of needle penetrable septum seal portions 580 preferably is arranged to extend partially into central slot 402 generally in the plane of each of the internal walls of the central portion 120 which define operational volumes A - CC and defines a needle penetrable seal between a different pair of operational volumes.

Each of needle penetrable septum seal portions 580 is labeled by letters which indicate the operational volumes between which it is sealingly disposed. Accordingly, needle penetrable septum seal portion A-B is sealingly disposed between operational volumes A and B and is needle penetrable along central slot 402 by needle assembly 130.

Reference is now made to Figs. 9A and 9B, which are simplified assembled and exploded view illustrations of sample insertion subassembly 160, forming part of cartridge 100 of Figs. 1A - 1C. As seen in Figs. 9A and 9B, the sample insertion subassembly 160 preferably comprises a base portion 610, an intermediate portion 612, a cover portion 614 and a mounting bracket 616 for slidable mounting of intermediate portion 612 onto base portion 610.

Reference is now made to Figs. 10A, 10B, 10C, 10D, 10E, 10F and 10G, which are simplified respective first and second pictorial, top and bottom plan view, front plan view and first and second side plan view illustrations of base portion 610 of the sample insertion subassembly 160 of Figs. 9A & 9B.

As seen in Figs. 10A - 10G, base portion 610 is preferably a unitary element, injection molded of plastic, and defines a first side surface 630, a second side surface 632, a first end surface 634, a second end surface 636 and a top surface 638. It is noted that a slot 639 is defined in first end surface 634, which slot 639 communicates with the interior of base portion 610.

A sample insertion bore 640 extends through base portion 610 from an inlet aperture 642 at top surface 638 to an outlet port 644, which is seated in sample insertion aperture 410 of central portion 120.

A pair of mounting bosses 650 extend upwardly from top surface 638. A recessed counterbore 652 extends downwardly from top surface 638 and accommodates a screw (not shown) which fixedly connects base portion 610 to connection boss 430 of central portion 120. An aligning tab 654 extends upwardly from top surface 638 for aligning base portion 610 and intermediate portion 612, as described further hereinbelow.

A piston displacement bore 660 having associated therewith a slot 662 leading therefrom to first side surface 630, extends from an aperture 664 on top surface 638 to a piston displacement port 666, having a bottom edge 668 which, when base portion 610 is assembled onto central portion 120, engages top surface 423 of central portion 120 about piston aperture 422 (Fig. 4A), such that the interior of piston displacement bore 660 is in communication with the operational volume below piston aperture 422. It is appreciated that slot 662 extends only partially alongside piston displacement bore 660.

Reference is now made to Figs. 11A, 11B, 11C, 11D, 11E, 11F and 11G, which are simplified respective first and second pictorial, top and bottom plan view, front plan view and first and second side plan view illustrations of intermediate portion 612, forming part of the sample insertion subassembly of Figs. 9A & 9B.

As seen in Figs. 11A - 11G, intermediate portion 612 is preferably a unitary element, injection molded of plastic, and defines a first side surface 670, a second side surface 672, a first end surface 674, a second end surface 676 and a bottom surface 678. Bottom surface 678 is preferably formed with a pair of generally parallel elongate slots 680, which slidably accommodate mounting bosses 650 of base portion 610. Bottom surface 678 also preferably includes alignment slot 681 for aligning base portion 610 and intermediate portion 612.

A sample insertion bore sealing plug 682 extends downwardly from bottom surface 678 for selectably sealing inlet aperture 642 of sample insertion bore 640 of base portion 610, when intermediate portion 612 is suitably slidably aligned with base portion 610.

A piston displacement bore 686 and an associated slot 688, leading therefrom to first side surface 670, both extend from a top edge 689 through intermediate portion 612 to bottom surface 678 at an aperture 690, which is aligned with aperture 664 on top surface 638 of base portion 610 when intermediate portion 612 is suitably slidably aligned with base portion 610.

A button 691 is preferably formed on first end surface 674 for selectable engagement with retaining slot 326 (Fig. 3A) of housing portion 112 for alignment of intermediate portion 612 with housing portion 112.

Intermediate portion 612 is retained in slidable engagement with base portion 610 by means of mounting bracket 616, which is preferably screwed onto mounting bosses 650.

Reference is now made to Figs. 12A, 12B, 12C and 12D, which are simplified respective pictorial and planar top, bottom and edge views of cover portion 614 of the sample insertion subassembly of Figs. 9A & 9B. As seen in Figs. 12A - 12D, cover portion 614 preferably defines a top surface 692 and a bottom surface 694 and an aperture 696 having a slot 698, which are aligned with piston displacement bore 686 and an associated slot 688 of intermediate portion 612.

Reference is now made to Figs. 13A, 13B, 13C and 13D, which are simplified respective first and second pictorial and planar side, edge and top views of cover element 132. As seen in Figs. 13A - 13D, cover element 132 is a generally elongate planar element having a generally planar outer surface 720 and a generally planar inner surface 722 having a pair of parallel raised edge portions 724. Edge portions 724 define first and second vertical edges 726 and 728, which are configured to tightly fit to corresponding portions of edges 220 and 320 of first and second outer housing portions 110 and 112, respectively.

Reference is now made to Figs. 14A, 14B, 14C, 14D and 14E, which are simplified respective pictorial and planar top, outer side, inner side and edge views of cover element 170 for a fluid solution insertion area of cartridge 100 of Figs. 1A - 1C.

As seen in Figs. 14A - 14E, cover element 170 is a generally planar element including a generally planar outer surface 740 and a generally planar inner surface 742 and having a plurality of attachment tabs 744 arranged along side edges 746 and 748 thereof. An additional attachment tab 750 is located on a top edge 752 of cover element 170 and an inwardly-extending portion 754 is arranged at a bottom edge 756 of cover element 170.

Reference is now made to Figs. 15A, 15B, 15C, 15D, 15E and 15F, which are simplified illustrations of safety lock element 180, forming part of cartridge 100 of Figs. 1A - 1C.

As seen in Figs. 15A - 15F, safety lock element 180 is an integrally formed element which preferably includes an outer protective panel portion 810 which defines one wall of a generally U-shaped engagement portion 812 and is arranged to cover and protect sensor array 192 (Fig. 1C). The U-shaped engagement portion 812 also includes an apertured connection portion 814, extending generally perpendicular to panel portion 810 and having a protrusion engagement aperture 815 for removable snap-fit retaining engagement with protrusion 212 of aperture 210 of first housing element 110.

Extending perpendicularly with respect to aperture connection portion 814 and generally in mutually spaced parallel relationship to panel portion 810 are first and second insertion wall panels 820 and 822, which are joined by an intermediate panel 824, all of which are removably inserted into aperture 210 for preventing access to microfluidic PCR array 190. An outer edge of panel 822 is preferably formed with a manually manipulatable engagement edge portion 826, which may be engaged by a user for manual removal of the safety lock element 180 prior to use of cartridge 100.

First and second valve protection knobs 833 and 834 are preferably formed on an outer surface of intermediate panel 824. Knobs 833 and 834 seal respective valves 433 and 434 (Figs. 24B - 24D) of PCR array 190 when safety lock element 180 engages cartridge 100, preventing contaminants and humidity from entering PCR array 190 and thereby extending the shelf life of cartridge 100.

It will be appreciated by persons skilled in the art that cartridge 100 of Figs. 1A - 15F described above is preferably employed for carrying out a biological process. A preferred arrangement of this process is summarized in the tables below. Table I sets forth preferred contents/functions of each of the operational volumes A - CC and waste receptacle DD:

**TABLE I**

| **Operational Volume No.** | **Operational Volume** | **Contents/Function** | **Comments** |
|---|---|---|---|
| 1 | A | Proteinase K and Sample 906 | Sample heating at 56°C, 10 min |
| 2 | B | Lysis solution (Guanidinium thiocyanate, ionic detergent in TRIS-HCL) and magnetic beads, preferably modified Sera-Mag^{™} SpeedBeads magnetic particles | |
| 3 | C | Wash Buffer I | |
| 4 | D | Wash Buffer II | |
| 5 | E | Wash Buffer III | |
| 6 | F | Elution Buffer | |
| 7 | G | Bead removal operational volume contains at least one miniature magnet | |
| 8 | H | Tube to PCR | |
| 9 | I | Sample Buffer A (histidine + alpha-thioglycerol)-dilution of PCR products | |
| 10 | J | Tube to Array | |
| 11 | K | Buffer for Discriminator 1 reconstitution [Either DDW (Ultra-Pure DNase and RNase free water produced by Biological Industries Beit Haemek, Israel) or High Salt Buffer]. | |
| 12 | L | Discriminator 1 (discriminators are either dried or in TE solution) | |
| 13 | M | Tube to Array | |
| 14 | N | Buffer for Discriminator 2 reconstitution | |
| 15 | O | Discriminator 2 | |
| 16 | P | Tube to Array | |
| 17 | Q | Buffer for Discriminator 3 reconstitution | |
| 18 | R | Discriminator 3 | |
| 19 | S | Tube to Array | |
| 20 | T | Buffer for Discriminator 4 reconstitution | |
| 21 | U | Discriminator 4 | |
| 22 | V | Tube to Array | |
| 23 | W | Buffer for Reporter reconstitution [Either DDW if Reporter is dried in High Salt Buffer (HSB) or vice versa, i.e. dried in water and reconstituted in HSB] | |
| 24 | X | RED Reporter (dried) | |
| 25 | Y | Tube to Array | |
| 26 | Z | Array Wash Buffer - Low Salt Buffer (washes the electronic sensor array before image acquisition) | |
| 27 | AA | Tube to Array | |
| 28 | BB | Array Wash Buffer - Low Salt Buffer (washes the electronic sensor array prior to image acquisition) | |
| 29 | CC | Tube to Array | |
| 30 | DD | Waste Receptacle | |

Table II sets forth a simplified description of a typical biological process that takes place in each operational volume in accordance with the present disclosure.

**TABLE II**

| **Operational volume** | **The Biological Process** |
|---|---|
| A | Sample pretreatment at 56°C for 10 minutes in the presence of the enzyme Proteinase K that dissolves cell membranes and eliminates nucleases, which are enzymes that catalyze the hydrolytic cleavage of phosphodiester linkages in the nucleic acid (NA) backbone, thus breaking down DNA and/or RNA. Once the pretreatment is completed, the contents of operational volume A are pumped by the action of piston assembly 140 in cylinder 420 and transferred to operational volume B. |
| | The corresponding mechanical operations of the relevant elements of cartridge 100 of Figs. 1A - 15F are described hereinbelow with reference to Figs. 17A - 17D. |
| B | Lysis: In operational volume B, the sample is mixed with the lysis solution and magnetic beads, followed by continuous mixing as the material passes through the needle. The piston is raised and lowered multiple times and the contents of operational volume B are pumped in and out of operational volume B via the fluid connections between the interior of the needle assembly 130 and the interior of cylinder 420 underlying piston assembly 140. The physical transport through the narrow needle of needle assembly 130, the pressure and shear forces of the transport lyses cells. Guanimidinium Thiocyanate is used to lyse cells and as a general protein denaturant which denatures proteins (including nucleases) which may otherwise inhibit nucleic acids from binding to the magnetic beads. Denaturation is through the disruption of hydrogen bonding and weakening of hydrophobic interactions. Ionic detergent and isopropanol act as detergents and help solubilize membrane proteins and lipids, causing the cell to lyse and to release its contents. Nucleic Acids (NAs) bind to the magnetic beads 923 via a coating thereof. |
| | At the end of the lysis stage, the Nucleic Acids (NAs) are bound to the magnetic beads 923 and the beads-NAs are then captured by a magnet to the wall of syringe cylinder (420). The remaining material (containing cell debris) is forced back to the Lysis operational volume (operational volume B). The needle now moves to wash 1 operational volume (operational volume C). |
| | The corresponding mechanical operations of the relevant elements of cartridge 100 of Figs. 1A - 15F are described hereinbelow with reference to Figs. 18A - 18F. |
| C | The magnet is removed and the beads and the nucleic acids bound thereto are washed. Wash Buffer I is pumped into syringe cylinder 420, and the wash buffer, beads and the nucleic acids bound thereto are pumped in and out of operational volume C numerous times as before via the fluid connections between the interior of the needle assembly 130 and the interior volume 916 of cylinder 420 underlying piston assembly 140. At the end of the wash, the magnet is returned to propinquity with the cylinder 420 and recaptures the beads onto the wall of syringe cylinder 420. The remaining material is forced back to operational volume C. |
| | The corresponding mechanical operations of the relevant elements of cartridge 100 of Figs. 1A - 15F are described hereinbelow with reference to Figs. 19A - 19F. |
| D, E | The needle is moved to the wash II operational volume (operational volume D) and then to wash III operational volume (operational volume E) with the biological processing of the sample done as previously described with reference to operational volume C. At the end of third wash cycle the beads-NAs are attached via magnet to the wall of syringe cylinder 420, the piston is lowered and the remaining material pushed back into operational volume E. The needle now moves to the next operational volume, operational volume F. |
| | The corresponding mechanical operations of the relevant elements of cartridge 100 of Figs. 1A - 15F are described hereinbelow with reference to Figs. 20A - 20F and Figs. 21A - 21F. |
| F | The piston is raised, pumping elution buffer into syringe cylinder 420 and the magnet is removed to allow the release of beads-NAs and washing thereof. The Elution Buffer releases the NAs from the beads due to the change in salt concentration. At the end of the elution the piston is raised and the needle moves to the next operational volume so as to move the contents of operational volume F to operational volume G. |
| | The corresponding mechanical operations of the relevant elements of cartridge 100 of Figs. 1A - 15F are described hereinbelow with reference to Figs. 22A - 22D. |
| G, H | The piston is lowered transferring the eluted NAs and the beads into operational volume G. The operational volume comprises at least one small magnet for binding and removal of the beads (i.e. binds the beads to one end of the operational volume). Next the NAs are moved to the PCR (microfluidic PCR array 190) via operational volume H through sequential raising and lowering of the piston and needle. |
| | The corresponding mechanical operations of the relevant elements of cartridge 100 of Figs. 1A - 15F are described hereinbelow with reference to Figs. 23A - 23D and Figs. 24A - 24D. |
| I, J | Once PCR amplification (NA amplification) is completed, PCR products (amplicons) are moved through the exit valve 434 on the PCR chip to an exit tunnel leading directly into operational volume I for dilution with SBA (Sample Buffer A- histidine and alpha-thioglycerol). |
| | The PCR chip preferably comprises air springs - kind of pressure volumes that push the amplicons through the exit valve. Diluted amplicons are transported via operational volume J to the sensor array 192. |
| | The corresponding mechanical operations of the relevant elements of cartridge 100 of Figs. 1A - 15F are described hereinbelow with reference to Figs. 25A - 25C and Figs. 26A - 26C. |
| K | Through sequential movements of the piston and needle, a Reconstitution buffer, comprising either DDW or high salt buffer, is taken from operational volume K into the next operational volume, operational volume L. |
| | The corresponding mechanical operations of the relevant elements of cartridge 100 of Figs. 1A - 15F are described hereinbelow with reference to Figs. 27A - 27C. |
| L, M | Heat dried Discriminator 1 undergoes reconstitution with Reconstitution buffer comprising DDW taken from operational volume K. |
| | Alternatively, Discriminator 1 in TE buffer solution is diluted with Reconstitution buffer (comprising high salt buffer (HSB)) from operational volume K. The needle and piston are sequentially moved up and down so as to mix the solution. Next follows the transport of reconstituted discriminator 1 to Sensor array 192 via operational volume M. Hybridization of reconstituted discriminator 1 with the amplicons takes place in the sensor array. |
| | The corresponding mechanical operations of the relevant elements of cartridge 100 of Figs. 1A - 15F are described hereinbelow with reference to Figs. 27A - 27F and Figs. 28A - 28D. |
| N to V | The loading of discriminators 2-4 is performed as previously described for discriminator 1 (operational volumes K, L and M). Hybridization of reconstituted discriminator 2-4 with the amplicons takes place in the sensor array. |
| | The corresponding mechanical operations of the relevant elements of cartridge 100 of Figs. 1A - 15F are described hereinbelow with reference to Figs. 29A - 34D. |
| W | Needle moves to operational volume W and the piston is raised so as to draw Reporter Reconstitution Buffer (see above table) into the interior volume 916 of cylinder 420 underlying piston assembly 140. Needle moves to operational volume X. |
| | The corresponding mechanical operations of the relevant elements of cartridge 100 of Figs. 1A - 15F are described hereinbelow with reference to Figs. 35A - 35B. |
| X, Y | Dried RED Reporter is reconstituted in Reporter Reconstitution Buffer. Piston is moved up and down so as to insure full reconstitution. Reconstituted Reporter is transported via operational volume Y to Sensor Array. |
| | The corresponding mechanical operations of the relevant elements of cartridge 100 of Figs. 1A - 15F are described hereinbelow with reference to Figs. 35C - 35F and Figs. 36A - 36D. |
| Z, AA | Needle moves to operational volume Z and the piston is raised to draw Array Wash buffer (Low Salt Buffer) from operational volume Z into the interior volume 916 of cylinder 420 underlying piston assembly 140. Needle moves to the next operational volume (operational volume AA) and the buffer washes the Sensor Array 192 after transport via operational volume AA. |
| | The corresponding mechanical operations of the relevant elements of cartridge 100 of Figs. 1A - 15F are described hereinbelow with reference to Figs. 37A - 37C and Figs. 38A - 38D. |
| BB, CC | Second wash of sensor array performed as previously described for operational volumes Z and AA |
| | The corresponding mechanical operations of the relevant elements of cartridge 100 of Figs. 1A - 15F are described hereinbelow with reference to Figs. 39A - 39C and Figs. 40A - 40C. |

Table III is a list of preferred buffers referenced in Tables I and II

**TABLE III**

| **Buffers** | **Composition (concentrations prior to addition of sample)** |
|---|---|
| Proteinase K | Proteinase K in buffer, e.g., TRIS-HCl (ph 7.4) |
| Lysis Solution with beads | Guanidinium Thiocyanate, ionic detergent, buffer (pH 7.4), Isopropanol, Carrier RNA; modified Sera-Mag^{™} SpeedBeads magnetic particles |
| Wash Buffer I | Guanidinium Thiocyanate, ionic detergent, TRIS-HCl (pH 7.4), isopropanol, (DEPC)-Water |
| Wash Buffer II | KCl/Tris (pH 7), Ethanol, in Rnase-free water |
| Wash Buffer III | KCl/Tris (pH 7), in Rnase-free water |
| Elution Buffer | Tris based buffer / DDW (Ultra-Pure DNase and RNase free water produced by Biological Industries Beit Haemek, Israel) |
| Sample Buffer A (histidine + alpha-thioglycerol) | L-Histidine, 1-Thioglycerol in DDW |
| Discriminators | Either dried in High Salt Buffer (HSB) (NaPO4, NaCl, Triton, pH 7.4), or in solution in TE buffer |
| Reconstitution Buffer (Discriminators) | Either DDW (Ultra-Pure DNase and RNase free water produced by Biological Industries Beit Haemek, Israel), if discriminators are dried in HSB, or HSB, if discriminators are in TE buffer solution. |
| Reconstitution Buffer (Reporter) | Either HSB if reporter is dried in DDW or DDW if reporter is dried in HSB. |
| Array Wash buffer | Low Salt Buffer (LSB) (NaPO4, Triton, pH 7.4) |

It is appreciated that the composition, concentration and functioning of the various solutions, such as reagents or buffers, described hereinabove are typically known in the art and are provided as examples. The role that each chemical plays in the overall process may change from one sample to another. Similarly, different samples may require different chemicals, thus changing the exact contents of each operational volume.

Reference is now made to Figs. 16A, 16B, 16C, 16D, 16E, 16F, 16G, 16H, 16I and 16J, which are simplified illustrations of typical initial steps in the operation of cartridge 100 of Figs. 1A - 15F.

As seen in Fig. 16A, preferably initially the safety lock element 180 is removed from engagement with first outer housing portion 110 as indicated by an arrow 880.

As seen in Fig. 16B, preferably intermediate portion 612 is raised relative to base portion 610 (Figs. 9A & 9B), as indicated by an arrow 882, thereby unsealing sample insertion bore 640 at inlet aperture 642 at top surface 638 of base portion 610.

As seen in Fig. 16C, preferably intermediate portion 612 is slid sideways relative to base portion 610, as indicated by an arrow 884, in order to enable aperture 642 of bore 640 to be accessed for sample insertion thereinto.

Fig. 16D shows a sample carrying swab 900 about to be inserted into bore 640, via aperture 642 thereof. It is appreciated that alternatively, a sample may be inserted in another suitable way, such as by use of a pipette.

Fig. 16E shows partial insertion of swab 900 into bore 640, via aperture 642, as indicated by an arrow 902.

Fig. 16F shows full insertion of swab 900 and the sample carried thereby via bore 640 into operational volume A (Fig. 4C). Preferably the swab is rotated, as indicated by an arrow 904, or displaced to dislodge the sample, indicated schematically by dots 906, therefrom.

Preferably, as shown in Fig. 16G, the top part of the swab 900 is broken off and discarded.

As seen in Fig. 16H, preferably intermediate portion 612 is slid sideways relative to base portion 610, as indicated by an arrow 908, in an opposite direction to that shown in Fig. 16C.

As seen in Fig. 16I, preferably intermediate portion 612 is lowered relative to base portion 610, as indicated by an arrow 910, thereby sealing sample insertion bore 640 at inlet aperture 642 at top surface 638 of base portion 610. Preferably, intermediate portion 612 is aligned relative to base portion 610 by engagement of alignment slot 681 of intermediate portion 612 with aligning tab 654 of base portion 610.

As seen in Fig. 16J, the cartridge 100 is shown ready for biological processing of the sample, which is described hereinabove with reference to Tables I - III and hereinbelow with reference to Figs. 17A - 40C.

Reference is now made to Figs. 17A, 17B, 17C and 17D, which are simplified illustrations of typical further steps in the operation of cartridge 100 of Figs. 1A - 15F, wherein Fig. 17A shows an operational state identical to that of Fig. 16J, Figs. 17A - 17D showing cartridge 100 of Figs. 1A - 15F with the first outer housing portion 110 thereof removed.

Fig. 17A shows the sample 906 located in operational volume A together with a reaction liquid 912, preferably a buffer containing Proteinase K, and piston assembly 140 in its fully lowered position in cylinder 420.

As described hereinabove in Table I and Table II, the sample 906 is preferably pretreated by heating at 56°C for 10 minutes in the presence of reaction liquid 912, prior to being transferred, via needle assembly 130 and piston assembly 140, to operational volume B as described hereinbelow. It is appreciated that the heating of the sample 906 is achieved utilizing a heating element which is not part of cartridge 100 of Figs. 1A - 15F.

Fig. 17B shows lowering of needle assembly 130, as indicated by an arrow 914, such that pointed end 512 thereof communicates with operational volume A. Fig. 17B also includes a simplified illustration of the fluid connections between the interior of the needle assembly 130 and the interior of cylinder 420 underlying piston assembly 140. As shown in Fig. 17B and partially in greater detail in Figs. 5A - 5D, the hollow pointed end 512 of needle tube 510 communicates via the interior of needle tube 510, channel 524, fluid connection portion 522 and flexible tube 194 with male connection portion 412. Male connection portion 412 in turn communicates via internal passageway 414 in central portion 120, which communicates with the interior volume of cylinder 420 below piston assembly 140, which interior volume is hereinafter indicated by reference numeral 916.

Fig. 17C shows raising of the piston assembly 140, as indicated by an arrow 918, thereby drawing the sample 906 and the reaction liquid 912 at least partially into the interior volume 916 of cylinder 420 below piston assembly 140. Fig. 17C also includes the simplified illustration of the fluid connections between the interior of the needle assembly 130 and the interior volume of cylinder 420 underlying piston assembly 140 that appears in Fig. 17B and shows the displacement of the sample 906 and the reaction liquid 912, as indicated by an arrow 920.

Fig. 17D shows subsequent lowering of needle assembly 130, as indicated by an arrow 921, such that pointed end 512 thereof communicates with operational volume B.

Reference is now made to Figs. 18A, 18B, 18C, 18D, 18E and 18F which are simplified illustrations of typical still further steps in the operation of cartridge 100 of Figs. 1A - 15F, wherein Fig. 18A shows an operational state identical to that of Fig. 17D, Figs. 18A - 18D showing cartridge 100 of Figs. 1A - 15F in a viewing direction opposite to that of Figs. 17A - 17D and with the second outer housing portion 112 thereof removed.

Fig. 18A shows the pointed end 512 of needle assembly 130 in communication with operational volume B, which preferably contains an additional reaction liquid 922, such as a Lysis solution containing Guanidinium Thiocyanate, ionic detergent in TRIS-HCL, as well as magnetic beads 923, preferably modified Sera-Mag^{™} SpeedBeads Magnetic particles, commercially available from GE Life Sciences.

Fig. 18B shows lowering of the piston assembly 140, as indicated by an arrow 924, thus forcing the sample 906 and reaction liquid 912 via needle assembly 130 into operational volume B. Fig. 18B also includes the simplified illustration of the fluid connections between the interior of the needle assembly 130 and the interior of cylinder 420 underlying piston assembly 140 that appears in Figs. 17B and 17C and shows the displacement of the sample 906 and the reaction liquid 912 as indicated by an arrow 925.

Fig. 18C shows raising and lowering of the piston assembly 140, as indicated by an arrow 926, thereby repeatedly drawing the sample 906, the reaction liquids 912 and 922 and beads 923 at least partially into the interior volume 916 of cylinder 420 below piston assembly 140. Fig. 18C also includes the simplified illustration of the fluid connections between the interior of the needle assembly 130 and the interior volume 916 of cylinder 420 underlying piston assembly 140 that appears in Fig. 18B and shows the displacement of the sample 906, the reaction liquids 912 and 922 and beads 923. It is appreciated that the raising and lowering of piston assembly 140 is preferably carried out multiple times to produce lysis of cells forming part of the sample 906, which is indicated symbolically by breaking up of dots which represent the cells of sample 906.

Fig. 18D shows a magnet 927, which is not part of cartridge 100 of Figs. 1A - 15F, being brought into propinquity with cylinder 420 such that it attracts the magnetic beads 923 to which are bound nucleic acids from sample 906.

Fig. 18E shows partial lowering of the piston assembly 140, in a direction indicated by an arrow 928, such as to separate most of the reaction liquids 912 and 922 and the remainder of sample 906, including cell debris, from the magnetic beads 923 and the nucleic acids bound thereto. Most of the reaction liquids 912 and 922 and the remainder of sample 906, including cell debris, is returned to operational volume B, where it remains, while the magnetic beads 923 and the nucleic acids bound thereto are retained in volume 916 by magnet 927.

It is appreciated that virtually all of reaction liquids 912 and 922 are returned to operational volume B and that only traces thereof remain in volume 916 together with magnetic beads 923 and the nucleic acids bound thereto.

Fig. 18F shows subsequent lowering of needle assembly 130, as indicated by an arrow 929, such that pointed end 512 thereof communicates with operational volume C.

Reference is now made to Figs. 19A, 19B, 19C and 19D, which are simplified illustrations of typical yet further steps in the operation of cartridge 100 of Figs. 1A - 15F, wherein Fig. 19A shows an operational state identical to that of Fig. 18D, Figs. 19A - 19D showing cartridge 100 of Figs. 1A - 15F in a viewing direction opposite to that of Figs. 18A - 18D and with the first outer housing portion 110 thereof removed.

Fig. 19A shows the pointed end 512 of needle assembly 130 in communication with operational volume C, which preferably contains an additional reaction liquid 930, preferably a wash buffer I, such as Guanidinium Thiocyanate, ionic detergent in Tris-HCl (pH 7.4), isopropanol, (DEPC)-Water.

Fig. 19B shows removal of magnet 927 out of propinquity with cylinder 420 and raising of the piston assembly 140, as indicated by an arrow 931, thus drawing reaction liquid 930 from operational volume C into the volume 916 underlying the piston assembly, together with the beads 923 and the nucleic acids bound thereto from sample 906 and any remaining traces of reaction liquids 912 and 922 and residual sample material, via needle assembly 130.

Fig. 19B also includes the simplified illustration of the fluid connections between the interior of the needle assembly 130 and the interior volume 916 of cylinder 420 underlying piston assembly 140 that appears in Figs. 18B and 18C and shows the displacement of reaction liquid 930 into engagement with the beads 923 and the nucleic acids bound thereto from sample 906 and any remaining traces of reaction liquids 912 and 922 and residual sample material.

Fig. 19C shows repeated lowering and raising of the piston assembly 140, as indicated by an arrow 932, thereby forcing the reaction liquid 930 together with beads 923 and the nucleic acids bound thereto from sample 906 and any remaining traces of reaction liquids 912 and 922 and sample material repeatedly into and out of the interior volume 916 of cylinder 420 below piston assembly 140 via the interior of needle assembly 130.

Fig. 19C also includes the simplified illustration of the fluid connections between the interior of the needle assembly 130 and the interior volume 916 of cylinder 420 underlying piston assembly 140 that appears in Fig. 19B and shows the displacement of the reaction liquid 930 together with beads 923 and the nucleic acids bound thereto from sample 906 and any remaining traces of reaction liquids 912 and 922 and sample material. It is appreciated that the raising and lowering of piston assembly 140 is preferably carried out multiple times to produce washing of beads 923 and the nucleic acids bound thereto from sample 906.

Fig. 19D shows magnet 927 being brought into propinquity with cylinder 420 such that it attracts the magnetic beads 923 to which are bound nucleic acids from sample 906.

Fig. 19E shows partial lowering of the piston assembly 140, in a direction indicated by an arrow 933, such as to separate most of the reaction liquid 930 and any remaining traces of reaction liquids 912 and 922 and any remainder of sample 906, including cell debris, from the magnetic beads 923 and the nucleic acids bound thereto. Most of the material other than the beads 923 and nucleic acids bound thereto is returned to operational volume C, where it remains, while the magnetic beads 923 and the nucleic acids bound thereto are retained in volume 916 by magnet 927.

It is appreciated that virtually all of reaction liquid 930 is returned to operational volume C and that only traces thereof remain in volume 916 together with magnetic beads 923 and the nucleic acids bound thereto.

Fig. 19F shows subsequent lowering of needle assembly 130, as indicated by an arrow 934, such that pointed end 512 thereof communicates with operational volume D.

Reference is now made to Figs. 20A, 20B, 20C, 20D, 20E and 20F, which are simplified illustrations of typical additional steps in the operation of cartridge 100 of Figs. 1A - 15F, wherein Fig. 20A shows an operational state identical to that of Fig. 19F, Figs. 20A - 20F showing cartridge 100 of Figs. 1A - 15F in a viewing direction opposite to that of Figs. 19A - 19F and with the second outer housing portion 112 thereof removed and showing operative engagement with operational volumes D and E.

Fig. 20A shows the pointed end 512 of needle assembly 130 in communication with operational volume D, which preferably contains an additional reaction liquid 935, such as a Wash buffer II, preferably comprising KCI, Tris (pH 7), Ethanol, in Rnase-free water.

Fig. 20B shows removal of magnet 927 out of propinquity with cylinder 420 and raising of the piston assembly 140, as indicated by an arrow 936, thus drawing reaction liquid 935 from operational volume D into engagement with magnetic beads 923 and the nucleic acids from sample 906 bound thereto including any remaining traces of reaction liquids 930, 912 and 922 and any remainder of sample 906, including cell debris. Fig. 20B also includes the simplified illustration of the fluid connections between the interior of the needle assembly 130 and the interior volume 916 of cylinder 420 underlying piston assembly 140 that appears in Fig. 19B and 19C and shows the displacement of reaction liquid 935 and any remaining traces of reaction liquids 930, 912 and 922 and any remainder of sample 906, including cell debris, as well as magnetic beads 923 and the nucleic acids bound thereto.

Fig. 20C shows repeated lowering and raising of the piston assembly 140, as indicated by an arrow 937, thereby forcing reaction liquid 935 and any remainder of sample 906, including cell debris, as well as magnetic beads 923 and the nucleic acids bound thereto, at least partially out of and into the interior volume 916 of cylinder 420 below piston assembly 140. Fig. 20C also includes the simplified illustration of the fluid connections between the interior of the needle assembly 130 and the interior volume 916 of cylinder 420 underlying piston assembly 140 that appears in Fig. 20B and shows the displacement of the reaction liquid 935, magnetic beads 923 and the nucleic acids from sample 906 bound thereto and any remaining traces of reaction liquids 930, 912 and 922 and any remainder of sample 906, including cell debris.

Fig. 20D shows magnet 927 being brought into propinquity with cylinder 420 such that it attracts the magnetic beads 923 to which are bound nucleic acids from sample 906.

Fig. 20E shows partial lowering of the piston assembly 140, in a direction indicated by an arrow 938, such as to separate most of the reaction liquid 935 and any remaining traces of reaction liquids 930, 912 and 922 and any remainder of sample 906, including cell debris, from the magnetic beads 923 and the nucleic acids bound thereto. Most of the material other than the beads 923 and nucleic acids from sample 906 bound thereto is returned to operational volume D, where it remains, while the magnetic beads 923 on the nucleic acids from sample 906 bound thereto are retained in volume 916.

It is appreciated that virtually all of reaction liquid 935 is returned to operational volume D and that only traces thereof remain in volume 916 together with magnetic beads 923 and the nucleic acids bound thereto.

Fig. 20F shows subsequent lowering of needle assembly 130 in a direction 939 such that pointed end 512 thereof communicates with operational volume E.

Reference is now made to Figs. 21A, 21B, 21C, 21D, 21E and 21F, which are simplified illustrations of typical additional steps in the operation of cartridge 100 of Figs. 1A - 15F, wherein Fig. 21A shows an operational state identical to that of Fig. 20F, Figs. 21A - 21F showing cartridge 100 of Figs. 1A - 15F in a viewing direction opposite to that of Figs. 20A - 20F and with the first outer housing portion 112 thereof removed and showing operative engagement with operational volumes E and F.

Fig. 21A shows the pointed end 512 of needle assembly 130 in communication with operational volume E, which preferably contains an additional reaction liquid 940, preferably a Wash Buffer III, such as KCI, Tris (pH 7), in Rnase-free water.

Fig. 21B shows removal of magnet 927 out of propinquity with cylinder 420 and raising of the piston assembly 140, as indicated by an arrow 941, thus drawing the reaction liquid 940 from operational volume E into the volume 916 underlying the piston assembly 140 in engagement with magnetic beads 923 and the nucleic acids from sample 906 bound thereto and any remaining traces of reaction liquids 935, 930, 922 and 912 and any remainder of sample 906, including cell debris, via needle assembly 130.

Fig. 21B also includes the simplified illustration of the fluid connections between the interior of the needle assembly 130 and the interior volume 916 of cylinder 420 underlying piston assembly 140 that appears in Figs. 20B and 20C and shows the displacement of the reaction liquid 940 into engagement with magnetic beads 923 and the nucleic acids from sample 906 bound thereto and any remaining traces of reaction liquids 935, 930, 922 and 912 and any remainder of sample 906, including cell debris.

Fig. 21C shows repeated lowering and raising of the piston assembly 140, as indicated by an arrow 942, thereby forcing the reaction liquid 940 and any remaining traces of reaction liquids 935, 930, 922 and 912 and any remainder of sample 906, including cell debris, as well as magnetic beads 923 and the nucleic acids bound thereto out of and into the interior volume 916 of cylinder 420 below piston assembly 140. Fig. 21C also includes the simplified illustration of the fluid connections between the interior of the needle assembly 130 and the interior volume 916 of cylinder 420 underlying piston assembly 140 that appears in Fig. 21B and shows the displacement of reaction liquid 940 and any remaining traces of reaction liquids 935, 930, 922 and 912 and any remainder of sample 906, including cell debris, as well as magnetic beads 923 and the nucleic acids bound thereto.

It is appreciated that the raising and lowering of piston assembly 140 is preferably carried out multiple times to wash away cell debris and unbound nucleic acids. This is achieved through serial washings with reaction liquid 940.

Fig. 21D shows magnet 927 being brought into propinquity with cylinder 420 such that it attracts the magnetic beads 923 to which are bound nucleic acids from sample 906.

Fig. 21E shows partial lowering of the piston assembly 140, in a direction indicated by an arrow 943, such as to separate most of the wash buffer 940 and any remaining traces of reaction liquids 935, 930, 922 and 912 and any remainder of sample 906, including cell debris, from the magnetic beads 923 and the nucleic acids bound thereto. Most of the material other than the beads 923 and nucleic acids bound thereto is returned to operational volume E, where it remains, while the magnetic beads 923 and the nucleic acids from sample 906 bound thereto are retained in volume 916.

It is appreciated that virtually all of reaction liquid 940 is returned to operational volume E and that only traces thereof remain in volume 916 together with magnetic beads 923 and the nucleic acids bound thereto.

Fig. 21F shows subsequent lowering of needle assembly 130, as indicated by an arrow 944, such that pointed end 512 thereof communicates with operational volume F.

Reference is now made to Figs. 22A, 22B, 22C and 22D, which are simplified illustrations of typical further steps in the operation of cartridge 100 of Figs. 1A - 15F, Figs. 22A - 22D showing cartridge 100 of Figs. 1A - 15F with the second outer housing portion 112 thereof removed and showing operative engagement with operational volume F.

Fig. 22A shows the pointed end 512 of needle assembly 130 in communication with operational volume F, which preferably contains an additional reaction liquid 945, preferably an Elution Buffer, such as Tris based buffer or Ultra-Pure DNase and RNase free water (DDW), commercially available from Biological Industries, Beit Haemek, Israel.

Fig. 22B shows removal of magnet 927 out of propinquity with cylinder 420 and subsequent raising of the piston assembly 140, as indicated by an arrow 946, thus drawing reaction liquid 945 from operational volume F into volume 916 underlying piston assembly 140 in engagement with magnetic beads 923 and the nucleic acids from sample 906 and any remaining traces of reaction liquids 940, 935, 930, 922 and 912 via needle assembly 130.

Fig. 22B also includes the simplified illustration of the fluid connections between the interior of the needle assembly 130 and the interior volume 916 of cylinder 420 underlying piston assembly 140 that appears in Figs. 21B and 21C and shows the displacement of reaction liquid 945 into engagement with magnetic beads 923 and the nucleic acids from sample 906 bound thereto and any remaining traces of reaction liquids 940, 935, 930, 922 and 912.

Fig. 22C shows subsequent repeated lowering and raising of the piston assembly 140, as indicated by an arrow 947, thereby forcing reaction liquid 945 and any remainder of sample 906, including cell debris, as well as magnetic beads 923 and the nucleic acids bound thereto, at least partially out of and into the interior volume 916 of cylinder 420 below piston assembly 140. As a result, the nucleic acids from sample 906 are disengaged from magnetic beads 923.

Fig. 22D shows the nucleic acids from sample 906 separated from the magnetic beads 923 in the solution containing the reaction liquid 945 located within volume 916, underlying piston assembly 140. The separated nucleic acids are symbolically shown and designated by reference numeral 950.

Reference is now made to Figs. 23A, 23B, 23C and 23D, which are simplified illustrations of typical further steps in the operation of cartridge 100 of Figs. 1A - 15F, Figs. 23A - 23D showing cartridge 100 of Figs. 1A - 15F with the second outer housing portion 112 thereof removed and showing operative engagement with operational volumes G and H.

Fig. 23A shows lowering of needle assembly 130, as indicated by an arrow 952, such that pointed end 512 thereof communicates with operational volume G, which contains at least one fixed miniature magnet 432.

Fig. 23B shows lowering of the piston assembly 140, as indicated by an arrow 956, thus forcing the reaction liquid 945, including separated nucleic acids 950 and beads 923, into operational volume G.

Fig. 23B also includes the simplified illustration of the fluid connections between the interior of the needle assembly 130 and the interior volume 916 of cylinder 420 underlying piston assembly 140 that appears in Figs. 22B and 22C and shows the displacement of reaction liquid 945, including separated nucleic acids 950 and beads 923.

Fig. 23C shows magnetic attraction of beads 923 by at least one miniature magnet 432 such that only the unbound nucleic acids 950 from sample 906 remain free in the reaction liquid 945 located in operational volume G.

Fig. 23D shows raising of the piston assembly 140, as indicated by an arrow 958, thereby drawing the unbound nucleic acids 950 from sample 906 and the reaction liquid 945 at least partially into the interior volume 916 of cylinder 420 below piston assembly 140. Fig. 23D also includes the simplified illustration of the fluid connections between the interior of the needle assembly 130 and the interior volume 916 of cylinder 420 underlying piston assembly 140 that appears in Fig. 23B and shows the displacement of the unbound nucleic acids 950 from sample 906 and the reaction liquid 945.

Reference is now made to Figs. 24A, 24B, 24C and 24D, which are simplified illustrations of typical further steps in the operation of cartridge 100 of Figs. 1A - 15F, Figs. 24A - 24D showing cartridge 100 of Figs. 1A - 15F with the second outer housing portion 112 thereof removed and showing operative engagement with operational volumes H and I.

Fig. 24A shows lowering of needle assembly 130, as indicated by an arrow 960, such that pointed end 512 thereof communicates with operational volume H, which communicates with microfluidic PCR array 190 which produces amplification of the nucleic acids 950.

Generally, microfluidic PCR array 190 operates as follows: nucleic acids (NAs) 950 enter the microfluidic PCR array 190 via operational volume H. A pressure source, preferably incorporated in the microfluidic PCR array 190, provides even distribution of the NAs 950 among the various PCR operational volumes located at the bottom part of the microfluidic PCR array 190. Operation of the piston assembly 140 pushes a solution comprising the NAs 950 up a channel leg of the microfluidic PCR array 190 until it reaches a valve element comprising a projecting carrier element having a carrier surface carrying dry PCR reaction mixture and is located in a cavity within the microfluidic flow cell along the channel leg. This arrangement allows for reconstitution of the dry PCR reaction mixture and subsequently NAs amplification within the PCR operational volume. Once PCR amplification is completed, the resulting PCR products, or amplicons, are moved through the action of air springs, pressure volumes that push the amplicons to exit valve 434 and directly into operational volume I for dilution with a Sample Buffer A- histidine and alpha-thioglycerol (SBA).

Fig. 24B shows lowering of the piston assembly 140, as indicated by an arrow 962, thus forcing the unbound nucleic acids 950 of sample 906 into operative engagement with microfluidic PCR array 190 via PCR valve 433.

Fig. 24C symbolically shows part of the PCR process in which the unbound nucleic acids 950 of sample 906 are amplified.

Fig. 24D shows the amplified unbound nucleic acids 950 of sample 906 in solution being released from microfluidic PCR array 190 to operational volume I under pressure by virtue of opening of valve 434 in the microfluidic PCR array 190, as indicated by an arrow 964. As noted above, operational volume I also contains a Sample Buffer A 965, preferably histidine and alpha-thioglycerol, such as L-Histidine and 1-Thioglycerol, in DDW, which is useful for diluting PCR products.

Reference is now made to Figs. 25A, 25B and 25C, which are simplified illustrations of typical additional steps in the operation of cartridge 100 of Figs. 1A - 15F, Figs. 25A - 25C showing cartridge 100 of Figs. 1A - 15F with the second outer housing portion 112 thereof removed and showing operative engagement with operational volumes I and J.

Fig. 25A shows lowering of needle assembly 130, as indicated by an arrow 966, such that pointed end 512 thereof communicates with operational volume I, which also contains a Sample Buffer A 965, preferably histidine and alpha-thioglycerol, such as L-Histidine and 1-Thioglycerol, in DDW, which is useful for diluting PCR products.

Fig. 25B shows raising of the piston assembly 140, as indicated by an arrow 968, thus drawing diluted amplified nucleic acids 950 from sample 906 at least partially into the interior volume 916 of cylinder 420 below piston assembly 140.

Fig. 25C shows lowering of needle assembly 130, as indicated by an arrow 970, such that pointed end 512 thereof communicates with operational volume J, which, in turn communicates with sensor array 192 (Fig. 26A) via supply aperture 442 (Fig. 26A).

Reference is now made to Figs. 26A, 26B and 26C, which are simplified illustrations of typical additional steps in the operation of cartridge 100 of Figs. 1A - 15F, wherein Fig. 26A shows an operational state identical to that of Fig. 25C, Figs. 26A - 26D showing cartridge 100 of Figs. 1A - 15F in a viewing direction opposite to that of Figs. 25A - 25C and with the first outer housing portion 110 thereof removed and showing operative engagement with operational volumes J and K.

Fig. 26A shows the needle assembly 130 in communication with operational volume J, which, in turn, communicates with the sensor array 192 via supply aperture 442.

Fig. 26B shows lowering of the piston assembly 140, as indicated by an arrow 972, thus forcing the diluted amplified nucleic acids 950 of sample 906 at least partially into operative engagement with sensor array 192. The diluted amplified nucleic acids 950, also known as amplicons, become attached to predetermined locations on the sensor array 192, as described in detail, inter alia, in the following U.S. Patents: 5,605,662; 6,238,624; 6,303,082; 6,403,367; 6,524,517; 6,960,298; 7,101,661; 7,601,493 and 8,288,155.

Prior to the lowering of piston assembly 140 seen in Fig. 26B, sealing element 238 (Figs. 2A - 2D) formed on first outer housing portion 110 has been inwardly displaced into sealing engagement with a passage 973 connecting operational volume J and operational volume M so that the diluted nucleic acids 950 flow only towards sensor array 192 and not in the direction of operational volume M. It is appreciated that sealing element 238, which seals passage 973, is not seen in Fig. 26B, since, as noted above, Fig. 26B shows cartridge 100 with the first outer housing portion 110 thereof removed and sealing element 238 is formed on first outer housing portion 110.

Fig. 26C shows lowering of needle assembly 130, as indicated by an arrow 974, such that pointed end 512 thereof communicates with operational volume K, which contains a Buffer for Discriminator 1 reconstitution 975 [Either DDW (Ultra-Pure DNase and RNase free water produced by Biological Industries Beit Haemek, Israel) or High Salt Buffer]. The Discriminator may be either dried in High Salt Buffer (HSB) (NaPO4 NaCl, Triton, pH 7.4), or in solution in TE buffer (TRIS, EDTA).

Reference is now made to Figs. 27A, 27B, 27C, 27D, 27E and 27F, which are simplified illustrations of typical additional steps in the operation of cartridge 100 of Figs. 1A - 15F, Figs. 27A - 27F showing cartridge 100 of Figs. 1A - 15F in a viewing direction opposite to that of Figs. 26A - 26C with the second outer housing portion 112 thereof removed and showing operative engagement with operational volumes K, L and M, wherein Fig. 27A shows an operational state identical to that of Fig. 26C.

Fig. 27B shows raising of the piston assembly 140, as indicated by an arrow 976, thus drawing the contents of operational volume K at least partially into the interior volume 916 of cylinder 420 below piston assembly 140.

Fig. 27C shows lowering of needle assembly 130, as indicated by an arrow 978, such that pointed end 512 thereof communicates with operational volume L, which preferably contains a dried discriminator 1, designated by reference numeral 979, which contains nucleic acids which are complementary to specific ones of the diluted amplified nucleic acids 950 of sample 906.

Fig. 27D shows multiple raising and lowering movements of piston assembly 140, as indicated by arrows 980, for mixing the content of operational volumes K and L.

Fig. 27E shows raising of the piston assembly 140, as indicated by an arrow 982, thus drawing the mixed contents of operational volumes K and L, namely the reconstituted discriminator 1, at least partially into the interior volume 916 of cylinder 420 below piston assembly 140.

Fig. 27F shows lowering of needle assembly 130, as indicated by an arrow 984, such that pointed end 512 thereof communicates with operational volume M, which, in turn, communicates with the interior of sensor array 192 via supply aperture 442.

Reference is now made to Figs. 28A, 28B, 28C and 28D, which are simplified illustrations of typical additional steps in the operation of cartridge 100 of Figs. 1A - 15F, Figs. 28A - 28D showing cartridge 100 of Figs. 1A - 15F in a viewing direction opposite to that of Figs. 27A - 27F with the first outer housing portion 112 thereof removed, wherein Fig. 28A shows an operational state identical to that of Fig. 27F, Figs. 28A - 28D showing operative engagement with operational volumes M and N.

Fig. 28B shows lowering of the piston assembly 140, as indicated by an arrow 986, thus forcing the reconstituted discriminator 1 into operative engagement with the interior of sensor array 192 via operational volume M and supply aperture 442 and specifically with the diluted amplified nucleic acids 950, also known as amplicons, which are attached to predetermined locations on the sensor array 192. To the extent that the nucleic acids in the reconstituted discriminator 1 are complementary to the diluted amplified nucleic acids 950, hybridization occurs.

As seen by comparing the fluid flows shown in Fig. 26B with those shown in Fig. 28B, prior to lowering of piston assembly 140, sealing element 238 has been disengaged from sealing engagement with passageway 973 connecting operational volume J and operational volume M so that the reconstituted discriminator 1 is enabled to flow towards sensor array 192 after filling a passageway connecting operational volume M and operational volumes P, S, V, Y, AA and CC.

Fig. 28C shows displacement and removal of the diluted amplified nucleic acids 950, also known as amplicons, which are not attached to predetermined locations on the sensor array 192, from sensor array 192 into waste receptacle operational volume DD via removal aperture 444 and waste conduits 452, 454, 456 and 458, as indicated by arrows 988.

Fig. 28D shows lowering of needle assembly 130, as indicated by an arrow 990, such that pointed end 512 thereof communicates with operational volume N, which contains a Buffer for Discriminator 2 reconstitution 991, which buffer may be identical to buffer 975.

Reference is now made to Figs. 29A, 29B, 29C, 29D, 29E and 29F, which are simplified illustrations of typical additional steps in the operation of cartridge 100 of Figs. 1A - 15F, Figs. 29A - 29F showing cartridge 100 of Figs. 1A - 15F in a viewing direction opposite to that of Figs. 28A - 28D with the second outer housing portion 112 thereof removed and showing operative engagement with operational volumes N, O and P, wherein Fig. 29A shows an operational state identical to that of Fig. 28D.

Fig. 29B shows raising of the piston assembly 140, as indicated by an arrow 992, thus drawing the contents of operational volume N at least partially into the interior volume 916 of cylinder 420 below piston assembly 140.

Fig. 29C shows lowering of needle assembly 130, as indicated by an arrow 994, such that pointed end 512 thereof communicates with operational volume O, which preferably contains a dried discriminator 2, designated by reference numeral 995, which contains nucleic acids which are complementary to other specific ones of the diluted amplified nucleic acids 950 of sample 906.

Fig. 29D shows multiple raising and lowering movements of piston assembly 140, as indicated by arrows 996, for mixing the content of operational volumes N and O.

Fig. 29E shows raising of the piston assembly 140, as indicated by an arrow 998, thus drawing the mixed contents of operational volumes N and O, namely the reconstituted discriminator 2, at least partially into the interior volume 916 of cylinder 420 below piston assembly 140.

Fig. 29F shows lowering of needle assembly 130, as indicated by an arrow 1000, such that pointed end 512 thereof communicates with operational volume P, which, in turn, communicates with the interior of sensor array 192 via supply aperture 442.

Reference is now made to Figs. 30A, 30B, 30C and 30D, which are simplified illustrations of typical additional steps in the operation of cartridge 100 of Figs. 1A - 15F, Figs. 30A - 30D showing cartridge 100 of Figs. 1A - 15F in a viewing direction opposite to that of Figs. 29A - 29F with the first outer housing portion 110 thereof removed, wherein Fig. 30A shows an operational state identical to that of Fig. 29F, Figs. 30A - 30D showing operative engagement with operational volumes P and Q.

Fig. 30B shows lowering of the piston assembly 140, as indicated by an arrow 1002, thus forcing the reconstituted discriminator 2 into operative engagement with the interior of sensor array 192, via supply aperture 442, and specifically with the diluted amplified nucleic acids 950, also known as amplicons, which are attached to predetermined locations on the sensor array 192. To the extent that the nucleic acids in the reconstituted discriminator 2 are complementary to the diluted amplified nucleic acids 950, hybridization occurs.

Sealing element 238 remains disengaged from sealing engagement with passageway 973 connecting operational volume J and operational volume M, as described above with reference to Fig. 28B, so that the reconstituted discriminator 2 is enabled to flow towards sensor array 192 after filling the passageway connecting operational volume M and operational volumes P, S, V, Y, AA and CC.

Fig. 30C shows displacement and removal of the reconstituted discriminator 1 from sensor array 192 into waste receptacle operational volume DD via removal aperture 444 and waste conduits 452, 454, 456 and 458, as indicated by arrows 1004.

Fig. 30D shows lowering of needle assembly 130, as indicated by an arrow 1006, such that pointed end 512 thereof communicates with operational volume Q, which contains a Buffer for Discriminator 3 reconstitution 1007, which buffer may be identical to buffer 975.

Reference is now made to Figs. 31A, 31B, 31C, 31D, 31E and 31F, which are simplified illustrations of typical additional steps in the operation of cartridge 100 of Figs. 1A - 15F, Figs. 31A - 31F showing cartridge 100 of Figs. 1A - 15F in a viewing direction opposite to that of Figs. 30A - 30D with the second outer housing portion 112 thereof removed and showing operative engagement with operational volumes Q, R and S, wherein Fig. 31A shows an operational state identical to that of Fig. 30D.

Fig. 31B shows raising of the piston assembly 140, as indicated by an arrow 1008, thus drawing the contents of operational volume Q at least partially into the interior volume 916 of cylinder 420 below piston assembly 140.

Fig. 31C shows lowering of needle assembly 130, as indicated by an arrow 1010, such that pointed end 512 thereof communicates with operational volume R, which preferably contains a dried discriminator 3, designated by reference numeral 1011, which contains nucleic acids which are complementary to further specific ones of the diluted amplified nucleic acids 950 of sample 906.

Fig. 31D shows multiple raising and lowering movements of piston assembly 140, as indicated by arrows 1012, for mixing the content of operational volumes Q and R.

Fig. 31E shows raising of the piston assembly 140, as indicated by an arrow 1014, thus drawing the mixed contents of operational volumes Q and R, namely the reconstituted discriminator 3, at least partially into the interior volume 916 of cylinder 420 below piston assembly 140.

Fig. 31F shows lowering of needle assembly 130, as indicated by an arrow 1016, such that pointed end 512 thereof communicates with operational volume S, which, in turn, communicates with the interior of sensor array 192 via supply aperture 442.

Reference is now made to Figs. 32A, 32B, 32C and 32D, which are simplified illustrations of typical additional steps in the operation of cartridge 100 of Figs. 1A - 15F, Figs. 32A - 32D showing cartridge 100 of Figs. 1A - 15F in a viewing direction opposite to that of Figs. 31A - 31F with the first outer housing portion 110 thereof removed, wherein Fig. 32A shows an operational state identical to that of Fig. 31F, Figs. 32A - 32D showing operative engagement with operational volumes S and T.

Fig. 32B shows lowering of the piston assembly 140, as indicated by an arrow 1018, thus forcing the reconstituted discriminator 3 into operative engagement with the interior of sensor array 192, via supply aperture 442, and specifically with the diluted amplified nucleic acids 950, also known as amplicons, which are attached to predetermined locations on the sensor array 192. To the extent that the nucleic acids in the reconstituted discriminator 3 are complementary to the diluted amplified nucleic acids 950, hybridization occurs.

Sealing element 238 remains disengaged from sealing engagement with passageway 973 connecting operational volume J and operational volume M, as described above with reference to Fig. 28B, so that the reconstituted discriminator 3 is enabled to flow towards sensor array 192 after filling the passageway connecting operational volume M and operational volumes P, S, V, Y, AA and CC.

Fig. 32C shows displacement and removal of the reconstituted discriminator 2 from sensor array 192 into waste receptacle operational volume DD via removal aperture 444 and waste conduits 452, 454, 456 and 458, as indicated by arrows 1020.

Fig. 32D shows lowering of needle assembly 130, as indicated by an arrow 1022, such that pointed end 512 thereof communicates with operational volume T, which contains a Buffer for Discriminator 4 reconstitution 1023, which buffer may be identical to buffer 975.

Reference is now made to Figs. 33A, 33B, 33C, 33D, 33E and 33F, which are simplified illustrations of typical additional steps in the operation of cartridge 100 of Figs. 1A - 15F, Figs. 33A - 33F showing cartridge 100 of Figs. 1A - 15F in a viewing direction opposite to that of Figs. 32A - 32D with the second outer housing portion 112 thereof removed and showing operative engagement with operational volumes T, U and V, wherein Fig. 33A shows an operational state identical to that of Fig. 32D.

Fig. 33B shows raising of the piston assembly 140, as indicated by an arrow 1024, thus drawing the contents of operational volume T at least partially into the interior volume 916 of cylinder 420 below piston assembly 140.

Fig. 33C shows lowering of needle assembly 130, as indicated by an arrow 1026, such that pointed end 512 thereof communicates with operational volume U, which preferably contains a dried discriminator 4, designated by reference numeral 1027, which contains nucleic acids which are complementary to yet further specific ones of the diluted amplified nucleic acids 950 of sample 906.

Fig. 33D shows multiple raising and lowering movements of piston assembly 140, as indicated by arrows 1028, for mixing the content of operational volumes T and U.

Fig. 33E shows raising of the piston assembly 140, as indicated by an arrow 1030, thus drawing the mixed contents of operational volumes T and U, namely the reconstituted discriminator 4, at least partially into the interior volume 916 of cylinder 420 below piston assembly 140.

Fig. 33F shows lowering of needle assembly 130, as indicated by an arrow 1032, such that pointed end 512 thereof communicates with operational volume V, which, in turn, communicates with the interior of sensor array 192 via supply aperture 442.

Reference is now made to Figs. 34A, 34B, 34C and 34D, which are simplified illustrations of typical additional steps in the operation of cartridge 100 of Figs. 1A - 15F, Figs. 34A - 34D showing cartridge 100 of Figs. 1A - 15F in a viewing direction opposite to that of Figs. 33A - 33F with the first outer housing portion 110 thereof removed, wherein Fig. 34A shows an operational state identical to that of Fig. 33F, Figs. 34A - 34D showing operative engagement with operational volumes V and W.

Fig. 34B shows lowering of the piston assembly 140, as indicated by an arrow 1034, thus forcing the reconstituted discriminator 4 into operative engagement with the interior of sensor array 192, via supply aperture 442, and specifically with the diluted amplified nucleic acids 950, also known as amplicons, which are attached to predetermined locations on the sensor array 192. To the extent that the nucleic acids in the reconstituted discriminator 4 are complementary to the diluted amplified nucleic acids 950, hybridization occurs.

Sealing element 238 remains disengaged from sealing engagement with passageway 973 connecting operational volume J and operational volume M, as described above with reference to Fig. 28B, so that the reconstituted discriminator 4 is enabled to flow towards sensor array 192 after filling the passageway connecting operational volume M and operational volumes P, S, V, Y, AA and CC.

Fig. 34C shows displacement and removal of the reconstituted discriminator 3 from sensor array 192 into waste receptacle operational volume DD via removal aperture 444 and waste conduits 452, 454, 456 and 458, as indicated by arrows 1036.

Fig. 34D shows lowering of needle assembly 130, as indicated by an arrow 1038, such that pointed end 512 thereof communicates with operational volume W, which contains a Buffer for Reporter reconstitution 1039, which buffer may be identical to buffer 975.

Reference is now made to Figs. 35A, 35B, 35C, 35D, 35E and 35F, which are simplified illustrations of typical additional steps in the operation of cartridge 100 of Figs. 1A - 15F, Figs. 35A - 35F showing cartridge 100 of Figs. 1A - 15F in a viewing direction opposite to that of Figs. 34A - 34D with the second outer housing portion 112 thereof removed and showing operative engagement with operational volumes W, X and Y, wherein Fig. 35A shows an operational state identical to that of Fig. 34D.

Fig. 35B shows raising of the piston assembly 140, as indicated by an arrow 1040, thus drawing the contents of operational volume W at least partially into the interior volume 916 of cylinder 420 below piston assembly 140.

Fig. 35C shows lowering of needle assembly 130, as indicated by an arrow 1042, such that pointed end 512 thereof communicates with operational volume X, which preferably contains a dried reporter, designated by reference numeral 1043, which contains nucleic acids to which are bound fluorescent dyes which hybridize to any one or more of the complementary nucleic acids which are contained in discriminators 1-4. Detection of the fluorescent dyes at predetermined locations in the sensor array indicates which one or more of the discriminators is complementary to a predetermined nucleic acid target site and thus provides an indication of presence of a given nucleic acid in the sample 906.

Fig. 35D shows multiple raising and lowering movements of piston assembly 140, as indicated by arrows 1044, for mixing the content of operational volumes W and X.

Fig. 35E shows raising of the piston assembly 140, as indicated by an arrow 1046, thus drawing the mixed contents of operational volumes W and X, namely the reconstituted reporter, at least partially into the interior volume 916 of cylinder 420 below piston assembly 140.

Fig. 35F shows lowering of needle assembly 130, as indicated by an arrow 1048, such that pointed end 512 thereof communicates with operational volume Y, which, in turn, communicates with the interior of sensor array 192 via supply aperture 442.

Reference is now made to Figs. 36A, 36B, 36C and 36D which are simplified illustrations of typical additional steps in the operation of cartridge 100 of Figs. 1A - 15F, Figs. 36A - 36D showing cartridge 100 of Figs. 1A - 15F in a viewing direction opposite to that of Figs. 35A - 35F with the first outer housing portion 110 thereof removed, wherein Fig. 36A shows an operational state identical to that of Fig. 35F, Figs. 36A - 36D showing operative engagement with operational volumes Y and Z.

Fig. 36B shows lowering of the piston assembly 140, as indicated by an arrow 1050, thus forcing the reconstituted reporter into operative engagement with the interior of sensor array 192, via supply aperture 442, and specifically with the nucleic acids contained in discriminators 1 - 4, which are attached to predetermined locations on the sensor array 192. To the extent that the nucleic acids in the reporter are complementary to the nucleic acids in any one or more of discriminators 1-4, hybridization occurs at the location of the relevant one or more of discriminators 1 - 4.

Sealing element 238 remains disengaged from sealing engagement with passageway 973 connecting operational volume J and operational volume M, as described above with reference to Fig. 28B, so that the reconstituted reporter is enabled to flow towards sensor array 192 after filling the passageway connecting operational volume M and operational volumes P, S, V, Y, AA and CC.

Fig. 36C shows displacement and removal of the reconstituted discriminator 4 from sensor array 192 into waste receptacle operational volume DD via removal aperture 444 and waste conduits 452, 454, 456 and 458, as indicated by arrows 1052.

Fig. 36D shows lowering of needle assembly 130, as indicated by an arrow 1054, such that pointed end 512 thereof communicates with operational volume Z, which contains an array wash buffer 1055, preferably a Low Salt Buffer (LSB) (NaPO4, Triton, pH 7.4).

Reference is now made to Figs. 37A, 37B and 37C, which are simplified illustrations of typical additional steps in the operation of cartridge 100 of Figs. 1A - 15F, wherein Fig. 37A shows an operational state identical to that of Fig. 36D, Figs. 37A - 37C showing cartridge 100 of Figs. 1A - 15F in a viewing direction opposite to that of Figs. 36A - 36C and with the second outer housing portion 112 thereof removed and showing operative engagement with operational volumes Z and AA.

Fig. 37B shows raising of the piston assembly 140, as indicated by an arrow 1056, thus drawing the contents of operational volume Z at least partially into the interior volume 916 of cylinder 420 below piston assembly 140.

Fig. 37C shows lowering of needle assembly 130, as indicated by an arrow 1058, such that pointed end 512 thereof communicates with operational volume AA, which communicates with the sensor array 192.

Reference is now made to Figs. 38A, 38B, 38C and 38D, which are simplified illustrations of typical additional steps in the operation of cartridge 100 of Figs. 1A - 15F, wherein Fig. 38A shows an operational state identical to that of Fig. 37C, Figs. 38A - 38D showing cartridge 100 of Figs. 1A - 15F in a viewing direction opposite to that of Figs. 37A - 37C and with the first outer housing portion 110 thereof removed and showing operative engagement with operational volumes AA and BB.

Fig. 38B shows lowering of the piston assembly 140, as indicated by an arrow 1060, thus forcing the contents of operational volume Z, via aperture 442, at least partially into operative engagement with the interior of the sensor array 192 washing and flushing thereof.

Sealing element 238 remains disengaged from sealing engagement with passageway 973 connecting operational volume J and operational volume M, as described above with reference to Fig. 28B, so that the array wash buffer 1055 is enabled to flow towards sensor array 192 after filling the passageway connecting operational volume M and operational volumes P, S, V, Y, AA and CC.

Fig. 38C shows displacement and removal of the residual reporter from the sensor array 192 into waste receptacle operational volume DD, via removal aperture 444 and waste conduits 452, 454, 456 and 458, as indicated by arrows 1062.

Fig. 38D shows lowering of needle assembly 130, as indicated by an arrow 1064, such that pointed end 512 thereof communicates with operational volume BB, which contains an array wash buffer 1065, preferably a Low Salt Buffer (LSB) (NaPO4, Triton, pH 7.4).

Reference is now made to Figs. 39A, 39B and 39C, which are simplified illustrations of typical additional steps in the operation of cartridge 100 of Figs. 1A - 15F, wherein Fig. 39A shows an operational state identical to that of Fig. 38D, Figs. 39A - 39C showing cartridge 100 of Figs. 1A - 15F in a viewing direction opposite to that of Figs. 38A - 38D and with the second outer housing portion 112 thereof removed and showing operative engagement with operational volumes BB and CC.

Fig. 39B shows raising of the piston assembly 140, as indicated by an arrow 1066, thus drawing the contents of operational volume BB at least partially into the interior volume 916 of cylinder 420 below piston assembly 140.

Fig. 39C shows lowering of needle assembly 130, as indicated by an arrow 1068, such that pointed end 512 thereof communicates with operational volume CC, which communicates with the sensor array 192.

Reference is now made to Figs. 40A, 40B and 40C, which are simplified illustrations of typical additional steps in the operation of cartridge 100 of Figs. 1A - 15F, wherein Fig. 40A shows an operational state identical to that of Fig. 39C, Figs. 40A & 40B showing cartridge 100 of Figs. 1A - 15F in a viewing direction opposite to that of Figs. 39A - 39C and with the first outer housing portion 110 thereof removed and showing operative engagement with operational volume CC.

Fig. 40B shows lowering of the piston assembly 140, as indicated by an arrow 1070, thus forcing the contents of operational volume BB at least partially into operative engagement with the sensor array 192 via aperture 442.

Sealing element 238 remains disengaged from sealing engagement with passageway 973 connecting operational volume J and operational volume M, as described above with reference to Fig. 28B, so that the array wash buffer 1065 is enabled to flow towards sensor array 192 after filling the passageway connecting operational volume M and operational volumes P, S, V, Y, AA and CC.

Fig. 40C shows displacement and removal of the array wash buffer 1055 from sensor array 192 into waste receptacle operational volume DD via removal aperture 444 and waste conduits 452, 454, 456 and 458, as indicated by arrows 1072.

It is appreciated that the heating of the sample 906, described hereinabove with reference to Fig. 17A, as well as movement of magnet 927, needle assembly 130 and piston assembly 140 and the engagement and disengagement of sealing element 238, described hereinabove, are preferably provided by a computerized controller and mechanism which is not part of cartridge 100.

It is also appreciated that the contents of operational volumes described with reference to Tables I-III and Figs 17A-40C which are described as being reconstituted, typically from a powder or a solid, may alternatively be supplied as a concentrated fluid and be diluted, typically using a high salt buffer (HSB).

It is further appreciated that the volume of fluid shown in the needle assembly and piston assembly at the various stages throughout the process described hereinabove with reference to Figs. 17A - 40C is not shown to scale and may illustrate more or less than the actual volume of fluid present.

It will be appreciated by persons skilled in the art that the present invention is defined by the appended claims, and is not necessarily limited by what has been specifically described hereinabove and may include both combinations and subcombinations of the features described hereinabove as well as equivalents and modifications thereof which would occur to persons skilled in the art upon reading the foregoing and which are not in the prior art.

## Claims

1. A cartridge (100) for use in in-vitro diagnostics, the cartridge comprising:
a cartridge housing (110, 112,120,150) defining a plurality of operational volumes (A to Z, AA, BB, CC, DD), at least some of said plurality of operational volumes (A to Z, AA, BB, CC, DD) being mutually linearly aligned; and
a fluid solution transporter (130, 140, 510) operative to transfer fluid solutions from at least one of said plurality of linearly aligned operational volumes (A to Z, AA, BB, CC, DD) to at least another of said plurality of linearly aligned operational volumes (A to Z, AA, BB, CC, DD),
**characterized in that**:
said cartridge housing defines at least one septum (150, 580) which mutually sealingly separates said plurality of linearly aligned operational volumes; and
said fluid solution transporter (130, 140, 510) includes:
a linearly displaceable transport element (130) operative to sequentially communicate with interiors of said at least some of said plurality of operational volumes (A to Z, AA, BB, CC, DD), with said linearly displaceable transport element (130) comprising a hollow needle (510); and
a piston assembly (140) communicating with said linearly displaceable transport element (130).

2. A cartridge (100) for use in in-vitro diagnostics according to claim 1 and also comprising a sample insertion subassembly (160).

3. A cartridge (100) for use in in-vitro diagnostics according to any of the preceding claims and wherein said plurality of operational volumes include a multiplicity of operational volumes, at least some of which are configured to allow injection of fluid solutions thereinto.

4. A cartridge (100) for use in in-vitro diagnostics according to any of the preceding claims and also comprising a microfluidic Polymerase Chain Reaction (PCR) array (190) mounted within said cartridge housing.

5. A cartridge (100) for use in in-vitro diagnostics according to claim 4 and wherein at least one of said operational volumes defines an internal passageway to a port of said microfluidic PCR array (190).

6. A cartridge (100) for use in in-vitro diagnostics according to any of the preceding claims and also comprising a sensor array (192) mounted onto said cartridge housing.

7. A cartridge (100) for use in in-vitro diagnostics according to claim 7 and wherein at least one of said operational volumes defines an internal passageway to a port of said sensor array.

8. A cartridge (100) for use in in-vitro diagnostics according to claim 1 and wherein said cartridge housing includes at least one waste conduit communicating with at least one operational volume operating as a waste collection volume.

9. A cartridge (100) for use in in-vitro diagnostics according to claim 1 and wherein said cartridge housing comprises first and second outer housing portions (110,112) and a central portion (120) and a septum portion (150), together defining at least some of said plurality of operational volumes.

10. A cartridge (100) for use in in-vitro diagnostics according to any of claims 1-9 and also comprising a flexible tube (194) interconnecting said piston assembly (140) with said linearly displaceable transport element (130).

## Patentansprüche

1. Kartusche (100) zur Verwendung in der In-vitro-Diagnostik, wobei die Kartusche Folgendes umfasst:
ein Kartuschengehäuse (110,112,120,150), das eine Vielzahl von Betriebsvolumen (A bis Z, AA, BB, CC, DD) definiert, wobei mindestens einige der Vielzahl von Betriebsvolumen (A bis Z, AA, BB, CC, DD) linear aufeinander ausgerichtet sind; und
einen Fluidlösungstransporter (130, 140, 510), der dazu betriebsfähig ist, Fluidlösungen von mindestens einem der Vielzahl von linear ausgerichteten Betriebsvolumen (A bis Z, AA, BB, CC, DD) zu mindestens einem anderen der Vielzahl von linear ausgerichteten Betriebsvolumen (A bis Z, AA, BB, CC, DD) zu übertragen, **dadurch gekennzeichnet, dass**:
das Kartuschengehäuse mindestens eine Trennwand (150, 580) definiert, die die Vielzahl von linear ausgerichteten Betriebsvolumen gegeneinander abdichtend trennt; und
der Fluidlösungstransporter (130, 140, 510) Folgendes enthält:
ein linear verschiebbares Transportelement (130), das dazu betriebsfähig ist, sequentiell mit Inneren von mindestens einigen der Vielzahl von Betriebsvolumen (A bis Z, AA, BB, CC, DD) zu kommunizieren, wobei das linear verschiebbare Transportelement (130)
eine Hohlnadel (510) umfasst; und
eine Kolbenbaugruppe (140), die mit dem linear verschiebbaren Transportelement (130) kommuniziert.

2. Kartusche (100) zur Verwendung in der In-vitro-Diagnostik nach Anspruch 1 und außerdem umfassend eine Probeneinführungs-Unterbaugruppe (160).

3. Kartusche (100) zur Verwendung in der In-vitro-Diagnostik nach einem der vorhergehenden Ansprüche und wobei die Vielzahl von Betriebsvolumen eine Vielheit von Betriebsvolumen enthält, von denen mindestens einige dazu konfiguriert sind, eine Injektion von Fluidlösungen dahinein zu ermöglichen.

4. Kartusche (100) zur Verwendung in der In-vitro-Diagnostik nach einem der vorhergehenden Ansprüche und außerdem eine mikrofluidische Polymerase-Kettenreaktions(PCR)-Anordnung (190) umfassend, die in dem Kartuschengehäuse montiert ist.

5. Kartusche (100) zur Verwendung in der In-vitro-Diagnostik nach Anspruch 4 und wobei mindestens eines der Betriebsvolumen einen internen Durchgang zu einem Anschluss der mikrofluidischen PCR-Anordnung (190) definiert.

6. Kartusche (100) zur Verwendung in der In-vitro-Diagnostik nach einem der vorhergehenden Ansprüche und außerdem eine Sensoranordnung (192) umfassend, die an dem Kartuschengehäuse montiert ist.

7. Kartusche (100) zur Verwendung in der In-vitro-Diagnostik nach Anspruch 7 und wobei mindestens eines der Betriebsvolumen einen internen Durchgang zu einem Anschluss der Sensoranordnung definiert.

8. Kartusche (100) zur Verwendung in der In-vitro-Diagnostik nach Anspruch 1 und wobei das Kartuschengehäuse mindestens eine Abfallstoffleitung enthält, die mit mindestens einem Betriebsvolumen kommuniziert, das als Abfallstoffsammelvolumen dient.

9. Kartusche (100) zur Verwendung in der In-vitro-Diagnostik nach Anspruch 1 und wobei das Kartuschengehäuse einen ersten und einen zweiten Außengehäuseabschnitt (110,112) und einen Mittelabschnitt (120) und einen Trennwandabschnitt (150) umfasst, die zusammen mindestens einige der Vielzahl von Betriebsvolumen definieren.

10. Kartusche (100) zur Verwendung in der In-vitro-Diagnostik nach einem der Ansprüche 1 - 9 und außerdem einen flexiblen Schlauch (194) umfassend, der die Kolbenbaugruppe (140) mit dem linear verschiebbaren Transportelement (130) verbindet.

## Revendications

1. Cartouche (100) devant être utilisée dans des diagnostics in vitro, la cartouche comprenant :
un boîtier de cartouche (110, 112, 120, 150) définissant une pluralité de volumes opérationnels (A à Z, AA, BB, CC, DD), au moins certains de ladite pluralité de volumes opérationnels (A à Z, AA, BB, CC, DD) étant alignés mutuellement linéairement ; et
un transporteur de solution fluide (130, 140, 510) opérationnel pour transférer des solutions fluides depuis au moins l'un de ladite pluralité de volumes opérationnels alignés linéairement (A à Z, AA, BB, CC, DD) jusqu'à au moins un autre de ladite pluralité de volumes opérationnels alignés linéairement (A à Z, AA, BB, CC, DD),
**caractérisé en ce que** :
ledit boîtier de cartouche définit au moins un septum (150, 580) qui sépare mutuellement de manière étanche ladite pluralité de volumes opérationnels alignés linéairement ; et
ledit transporteur de solution fluide (130, 140, 510) comprend :
un élément de transport à déplacement linéaire (130) opérationnel pour communiquer séquentiellement avec les intérieurs desdits au moins certains de ladite pluralité de volumes opérationnels (A à Z, AA, BB, CC, DD),
ledit élément de transport à déplacement linéaire (130) comprenant :
une aiguille creuse (510) ; et
un ensemble piston (140) communiquant avec ledit élément de transport à déplacement linéaire (130).

2. Cartouche (100) devant être utilisée dans des diagnostics in vitro selon la revendication 1 et comprenant également un sous-ensemble d'insertion d'échantillon (160).

3. Cartouche (100) devant être utilisée dans des diagnostics in vitro selon l'une quelconque des revendications précédentes et dans laquelle ladite pluralité de volumes opérationnels comprend une multiplicité de volumes opérationnels, dont au moins certains sont configurés pour permettre l'injection de solutions fluides dans ceux-ci.

4. Cartouche (100) devant être utilisée dans des diagnostics in vitro selon l'une quelconque des revendications précédentes et comprenant également un réseau de réaction en chaîne par polymérase (PCR) microfluidique (190) monté à l'intérieur dudit boîtier de cartouche.

5. Cartouche (100) devant être utilisée dans des diagnostics in vitro selon la revendication 4 et dans laquelle au moins l'un desdits volumes opérationnels définit un passage interne vers un port dudit réseau PCR microfluidique (190).

6. Cartouche (100) devant être utilisée dans des diagnostics in vitro selon l'une quelconque des revendications précédentes et comprenant également un réseau de capteurs (192) monté sur ledit boîtier de cartouche.

7. Cartouche (100) devant être utilisée dans des diagnostics in vitro selon la revendication 7 et dans laquelle au moins l'un desdits volumes opérationnels définit un passage interne vers un port dudit réseau de capteurs.

8. Cartouche (100) devant être utilisée dans des diagnostics in vitro selon la revendication 1 et dans laquelle ledit boîtier de cartouche comprend au moins un conduit de déchets communiquant avec au moins un volume opérationnel fonctionnant comme un volume de collecte de déchets.

9. Cartouche (100) devant être utilisée dans des diagnostics in vitro selon la revendication 1 et dans laquelle ledit boîtier de cartouche comprend des première et seconde parties de boîtier externe (110, 112) et une partie centrale (120) et une partie de septum (150), définissant ensemble au moins certains de ladite pluralité de volumes opérationnels.

10. Cartouche (100) devant être utilisée dans des diagnostics in vitro selon l'une quelconque des revendications 1 à 9 et comprenant également un tube flexible (194) interconnectant ledit ensemble piston (140) avec ledit élément de transport à déplacement linéaire (130).
